(19) Europäisches Patentamt / European Patent Office / Office européen des brevets

(11) EP 2 242 749 B1

(12) EUROPEAN PATENT SPECIFICATION

(45) Date of publication and mention of the grant of the patent: **10.04.2013 Bulletin 2013/15**

(21) Application number: **09709135.9**

(22) Date of filing: **27.01.2009**

(51) Int Cl.: *C07D 401/10* (2006.01) *C07D 401/14* (2006.01) *A61K 31/4725* (2006.01) *A61P 19/02* (2006.01)

(86) International application number: **PCT/EP2009/050875**

(87) International publication number: **WO 2009/098144 (13.08.2009 Gazette 2009/33)**

# (54) NOVEL PYRIDINONES AND PYRIDAZINONES

Neue Pyridinone und Pyridazinone

NOUVELLES PYRIDINONES ET PYRIDAZINONES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **05.02.2008 US 26204**
**15.12.2008 US 122510**

(43) Date of publication of application: **27.10.2010 Bulletin 2010/43**

(73) Proprietor: **F. Hoffmann-La Roche AG**
**4070 Basel (CH)**

(72) Inventors:
- **DEWDNEY, Nolan James** Saratoga California 95070 (US)
- **KENNEDY-SMITH, Joshua** San Francisco California 94103 (US)
- **KONDRU, Rama K.** Morris Plains, NJ 07950 (US)
- **LOE, Bradley E.** Santa Cruz California 95060 (US)
- **LOU, Yan** San Jose California 95103 (US)
- **MCINTOSH, Joel** Pacifica California 94044 (US)
- **OWENS, Timothy D.** Mountain View California 94040 (US)
- **SOTH, Michael** Milpitas California 95035 (US)
- **SWEENEY, Zachary Kevin** Redwood City California 94062 (US)
- **TAYGERLY, Joshua Paul Gergely** San Francisco California 94131 (US)

(74) Representative: **Sauer, Frank et al**
**F. Hoffmann-La Roche AG**
**Patent Department**
**Grenzacherstrasse 124**
**4070 Basel (CH)**

(56) References cited:
**WO-A-2006/124731** **WO-A-2007/021795**
**WO-A-2007/027594** **WO-A-2007/136465**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 242 749 B1

## Description

The present invention relates to the use of novel derivatives which inhibit Btk and are useful for the treatment of auto-immune and inflammatory diseases caused by aberrant B-cell activation. The novel 5-phenyl-1H-pyridin-2-one and 6-phenyl-2H-pyridazin-3-one derivatives described herein are useful for the treatment of arthritis.

Protein kinases constitute one of the largest families of human enzymes and regulate many different signaling processes by adding phosphate groups to proteins (Hunter, Cell 1987 50:823-829). Specifically, tyrosine kinases phosphorylate proteins on the phenolic moiety of tyrosine residues. The tyrosine kinase family includes members that control cell growth, migration, and differentiation. Abnormal kinase activity has been implicated in a variety of human diseases including cancers, autoimmune and inflammatory diseases. Since protein kinases are among the key regulators of cell signaling they provide a target to modulate cellular function with small molecular kinase inhibitors and thus make good drug design targets. In addition to treatment of kinase-mediated disease processes, selective and efficacious inhibitors of kinase activity are also useful for investigation of cell signaling processes and identification of other cellular targets of therapeutic interest.

There is good evidence that B-cells play a key role in the pathogenesis of autoimmune and/or inflammatory disease. Protein-based therapeutics that deplete B cells such as Rituxan are effect-tive against autoantibody-driven inflammatory diseases such as rheumatoid arthritis (Rastetter et al. Annu Rev Med 2004 55:477). Therefore inhibitors of the protein kinases that play a role in B-cell activation should be useful therapeutics for B-cell mediated disease pathology such as autoantibody production.

Signaling through the B-cell receptor (BCR) controls a range of B-cell responses including proliferation and differentiation into mature antibody producing cells. The BCR is a key regulatory point for B-cell activity and aberrant signaling can cause deregulated B-cell proliferation and formation of pathogenic autoantibodies that lead to multiple autoimmune and/or inflammatory diseases. Bruton's Tyrosine Kinase (Btk) is a non-BCR associated kinase that is membrane proximal and immediately downstream from BCR. Lack of Btk has been shown to block BCR signaling and therefore inhibition of Btk could be a useful therapeutic approach to block B-cell mediated disease processes.

Btk is a member of the Tec family of tyrosine kinases, and has been shown to be a critical regulator of early B-cell development and mature B-cell activation and survival (Khan et al. Immunity 1995 3:283; Ellmeier et al. J. Exp. Med. 2000 192:1611). Mutation of Btk in humans leads to the condition X-linked agammaglobulinemia (XLA) (reviewed in Rosen et al. New Eng. J. Med. 1995 333:431 and Lindvall et al. Immunol. Rev. 2005 203:200). These patients are immunocom-promised and show impaired maturation of B-cells, decreased immunoglobulin and peripheral B-cell levels, diminished T-cell independent immune responses as well as attenuated calcium mobilization following BCR stimulation.

Evidence for a role for Btk in autoimmune and inflammatory diseases has also been provided by Btk-deficient mouse models. In preclinical murine models of systemic lupus erythematosus (SLE), Btk-deficient mice show marked amelioration of disease progression. In addition, Btk-deficient mice are resistant to collagen-induced arthritis (Jansson and Holmdahl Clin. Exp. Immunol. 1993 94:459). A selective Btk inhibitor has been demonstrated dose-dependent efficacy in a mouse arthritis model (Pan et al., Chem. Med Chem. 2007 2:58-61).

Btk is also expressed by cells other than B-cells that may be involved in disease processes. For example, Btk is expressed by mast cells and Btk-deficient bone marrow derived mast cells demonstrate impaired antigen induced degranulation (Iwaki et al. J. Biol. Chem. 2005 280:40261). This shows Btk could be useful to treat pathological mast cells responses such as allergy and asthma. Also monocytes from XLA patients, in which Btk activity is absent, show decreased TNF alpha production following stimulation (Horwood et al. J Exp Med 197:1603, 2003). Therefore TNF alpha mediated inflammation could be modulated by small molecular Btk inhibitors. Also, Btk has been reported to play a role in apoptosis (Islam and Smith Immunol. Rev. 2000 178:49,) and thus Btk inhibitors would be useful for the treatment of certain B-cell lymphomas and leukemias (Feldhahn et al. J. Exp. Med. 2005 201:1837,).

The present application provides the Btk inhibitor compounds of Formulae I-III, methods of use thereof, as described herein below:

The application provides a compound of Formula I,

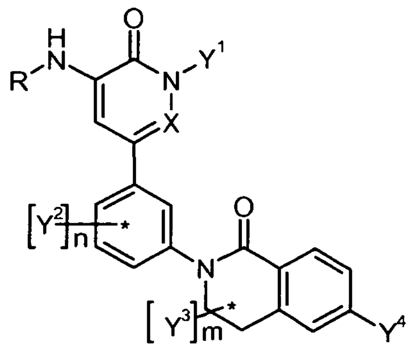

(I)

wherein:

| | |
|---|---|
| R | is H, $-R^1$, $-R^1-R^2-R^3$, or $-R^2$-R3; |
| $R^1$ | is aryl, heteroaryl, cycloalkyl, or heterocycloalkyl, and is optionally substituted with lower alkyl, hydroxy, lower alkoxy, halo, nitro, amino, cyano, or halo-lower alkyl; |
| $R^2$ | is -C(=O), -C(=O)O, -C(=O)NH, or $-S(=O)_2$; |
| $R^3$ | is H or $R^4$; wherein $R^4$ is lower alkyl, aryl, arylalkyl, akylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with lower alkyl, hydroxy, lower alkoxy, halo, nitro, amino, cyano, or halo-lower alkyl; |
| X | is CH or N; |
| $Y^1$ | is H or lower alkyl; |
| each $Y^2$ | is independently halogen or lower alkyl, wherein lower alkyl is optionally substituted with one or more substituents selected from the group consisting of hydroxy, lower alkoxy, and halogen; |
| n | is 0, 1, 2, or 3. |
| $Y^3$ | is H, halogen, or lower alkyl, wherein lower alkyl is optionally substituted with one or more substituents selected from the group consisting of hydroxy, lower alkoxy, amino, and halogen; |
| M | is 0 or 1; |
| $Y^4$ | is $Y^{4a}$, $Y^{4b}$, $Y^{4c}$ or $Y^{4d}$; wherein $Y^{4a}$ is H or halogen; $Y^{4b}$ is lower alkyl, optionally substituted with one or more substituents selected from the group consisting of lower haloalkyl, halogen, hydroxy, amino, and lower alkoxy; $Y^{4c}$ is lower cycloalkyl, optionally substituted with one or more substituents selected from the group consisting of lower alkyl, lower haloalkyl, halogen, hydroxy, amino, and lower alkoxy; and $Y^{4d}$ is amino, optionally substituted with one or more lower alkyl; |

or a pharmaceutically acceptable salt thereof

The application also provides a compound of Formula I,

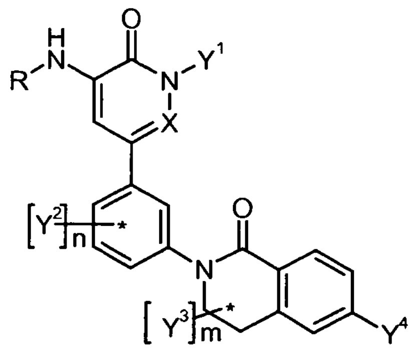

(I)

wherein:

| | |
|---|---|
| R | is H, $-R^1$, $-R^1-R^2-R^3$, $-R^1-R^3$, or $-R^2-R^3$; |
| $R^1$ | is aryl, heteroaryl, cycloalkyl, or heterocycloalkyl, and is optionally substituted with one or more lower alkyl, hydroxy, hydroxy lower alkyl, lower alkoxy, halo, nitro, amino, amido, cyano, oxo, or halo-lower alkyl; |

$R^2$ is -C(=O), -C(=O)O, -C(=O)NR$^2$, -NHC(=O)O, , -C(=NH)NR$^2$, or -S(=O)$_2$; wherein $R^2$ is H or lower alkyl;

$R^3$ is H or $R^4$; wherein $R^4$ is lower alkyl, amino, aryl, arylalkyl, alkylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with one or more lower alkyl, hydroxy, lower alkoxy, hydroxy lower alkyl, hydroxy lower alkoxy, lower alkyl sulfonyl, lower alkyl sulfonamido, carbamate, carboxy, ester, amido, acyl, halo, nitro, amino, cyano, oxo, or halo-lower alkyl;

X is CH or N;

$Y^1$ is H, lower alkyl, or lower haloalkyl;

each $Y^2$ is independently halogen, oxime, or lower alkyl, wherein lower alkyl is optionally substituted with one or more substituents selected from the group consisting of hydroxy, lower alkoxy, lower haloalkoxy, lower haloalkyl, carboxy, amino, and halogen;

n is 0, 1, 2, or 3.

$Y^3$ is H, halogen, or lower alkyl, wherein lower alkyl is optionally substituted with one or more substituents selected from the group consisting of hydroxy, lower alkoxy, amino, and halogen;

m is 0 or 1;

$Y^4$ is $Y^{4a}$, $Y^{4b}$, $Y^{4c}$, or $Y^{4d}$; wherein $Y^{4a}$ is H or halogen; $Y^{4b}$ is lower alkyl, optionally substituted with one or more substituents selected from the group consisting of lower haloalkyl, halogen, hydroxy, amino, cyano, and lower alkoxy; $Y^{4c}$ is lower cycloalkyl, optionally substituted with one or more substituents selected from the group consisting of lower alkyl, lower haloalkyl, halogen, hydroxy, amino, cyano, and lower alkoxy; and $Y^{4d}$ is amino, optionally susbstituted with one or more lower alkyl, alkoxy lower alkyl, or hydroxy lower alkyl;

or a pharmaceutically acceptable salt thereof.

In one variation of Formula I, $Y^1$ is methyl.

In one variation of Formula I, X is CH.

In one variation of Formula I, n is 1 and m is 0.

In one variation of Formula I, $Y^3$ is H.

In one variation of Formula I, $Y^2$ is methyl. In one variation of Formula I, $Y^2$ is hydroxymethyl. In one variation of Formula I, $Y^2$ is hydroxyethyl. In one variation of Formula I, $Y^2$ is halogen.

In one variation of Formula I, $Y^4$ is

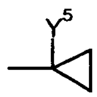

wherein $Y^5$ is halogen, lower alkyl or lower haloalkyl. In one variation of Formula I, $Y^4$ is

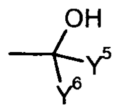

wherein $Y^5$ and $Y^6$ are independently H, lower alkyl, or lower haloalkyl. In one variation of Formula I, $Y^4$ is

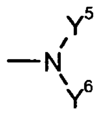

wherein $Y^5$ and $Y^6$ are independently H or lower alkyl. In one variation of Formula I, $Y^4$ is

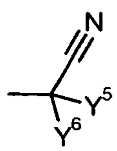

wherein $Y^5$ and $Y^6$ are independently H, lower alkyl, or lower haloalkyl.

In one variation of Formula I, R is $-R^1-R^2-R^3$; wherein $R^1$ is phenyl or pyridyl; $R^2$ is -C(=O); $R^3$ is $R^4$; and $R^4$ is morpholine or piperazine, optionally substituted with one or more lower alkyl.

In one variation, Formula I is 6-Dimethylamino-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (**I-2**) of formula

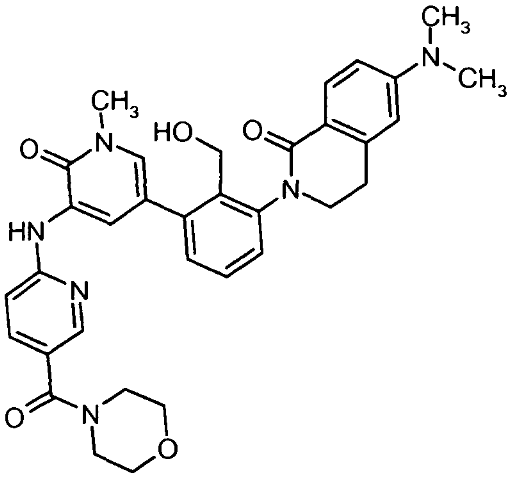

,

6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (**I-3**) of formula

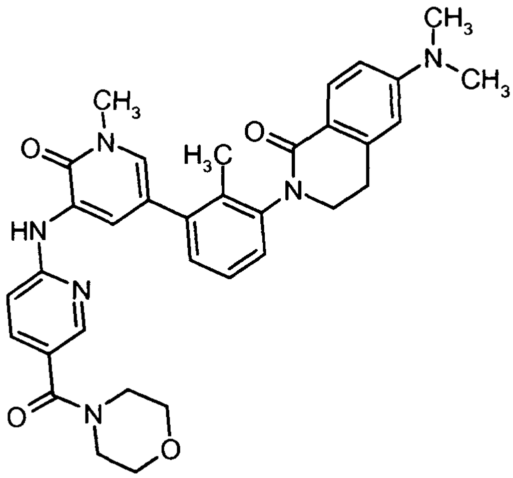

or 6-Dimethylamino-2-(3-{1-methyl-5-[5-(morpholine-4-carbonyl)-pyridin-2-ylamino]-6-oxo-1,6-dihydro-pyridin-3-yl}-phenyl)-3,4-dihydro-2H-Boquinolin-1-one (**I-1**) of formula

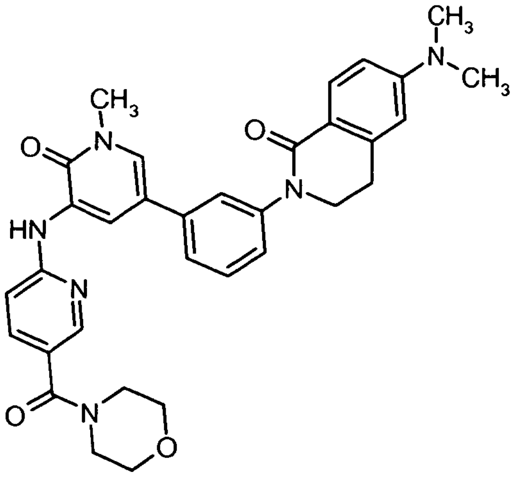

.

The application provides a compound of Formula II,

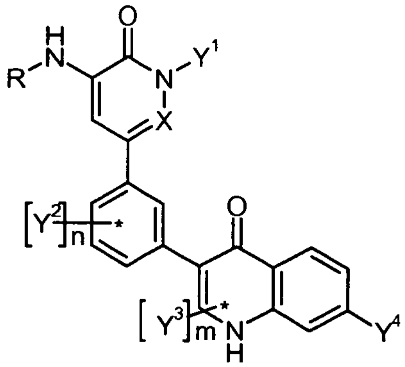

(II)

wherein:

R is H, $-R^1$, $-R^1-R^2-R^3$, or $-R^2-R^3$;

$R^1$ is aryl, heteroaryl, cycloalkyl, or heterocycloalkyl, and is optionally substituted with lower alkyl, hydroxy, lower alkoxy, halo, nitro, amino, cyano, or halo-lower alkyl;

$R^2$ is -C(=O), -C(=O)O, -C(=O)NH, or $-S(=O)_2$;

$R^3$ is H or $R^4$; wherein $R^4$ is lower alkyl, aryl, arylalkyl, alkylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with lower alkyl, hydroxy, lower alkoxy, halo, nitro, amino, cyano, or halo-lower alkyl;

X is CH or N;

$Y^1$ is H or lower alkyl;

each $Y^2$ is independently halogen or lower alkyl, wherein lower alkyl is optionally substituted with one or more substituents selected from the group consisting of hydroxy, lower alkoxy, and halogen;

n is 0, 1, 2, or 3.

$Y^3$ is H, halogen, or lower alkyl, wherein lower alkyl is optionally substituted with one or more substituents selected from the group consisting of hydroxy, lower alkoxy, amino, and halogen;

m is 0 or 1;

$Y^4$ is $Y^{4a}$, $Y^{4b}$, $Y^{4c}$, or $Y^{4d}$; wherein $Y^{4a}$ is H or halogen; $Y^{4b}$ is lower alkyl, optionally substituted with one or more substituents selected from the group consisting of lower haloalkyl, halogen, hydroxy, amino, and lower alkoxy; $Y^{4c}$ is lower cycloalkyl, optionally substituted with one or more substituents selected from the group consisting of lower alkyl, lower haloalkyl, halogen, hydroxy, amino, and lower alkoxy; and $Y^{4d}$ is amino, optionally substituted with one or more lower alkyl;

or a pharmaceutically acceptable salt thereof.

The application also provides a compound of Formula II,

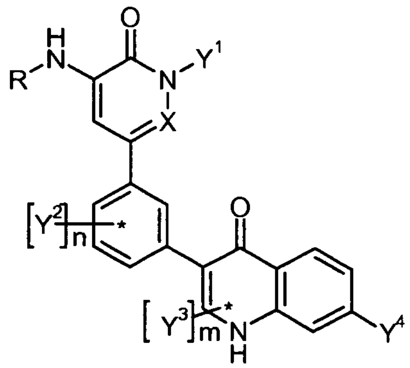

(II)

wherein:

| | |
|---|---|
| R | is H, $-R^1$, $-R^1-R^2-R^3$, $-R^1-R^3$, or$-R^2-R^3$; |
| $R^1$ | is aryl, heteroaryl, cycloalkyl, or heterocycloalkyl, and is optionally substituted with one or more lower alkyl, hydroxy, hydroxy lower alkyl, lower alkoxy, halo, nitro, amino, amido, cyano, oxo, or halo-lower alkyl; |
| $R^2$ | is -C(=O), -C(=O)O, -C(=O)$NR^2$, or $-S(=O)_2$; wherein $R^2$ is H or lower alkyl; |
| $R^3$ | is H or $R^4$; wherein $R^4$ is lower alkyl, amino, aryl, arylalkyl, alkylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with one or more lower alkyl, hydroxy, hydroxy lower alkyl, lower alkoxy, halo, nitro, amino, cyano, or halo-lower alkyl; |
| X | is CH or N; |
| $Y^1$ | is H, lower alkyl, or lower haloalkyl; |
| each $Y^2$ | is independently halogen or lower alkyl, wherein lower alkyl is optionally substituted with one or more substituents selected from the group consisting of hydroxy, lower alkoxy, lower haloalkoxy, lower haloalkyl, carboxy, amino, and halogen; |
| n | is 0, 1, 2, or 3. |
| $Y^3$ | is H, halogen, or lower alkyl, wherein lower alkyl is optionally substituted with one or more substituents selected from the group consisting of hydroxy, lower alkoxy, amino, and halogen; |
| m | is 0 or 1; |
| $Y^4$ | is $Y^{4a}$ $Y^{4b}$, $Y^{4c}$, or $Y^{4d}$; wherein $Y^{4a}$ is H or halogen; $Y^{4b}$ is lower alkyl, optionally substituted with one or more substituents selected from the group consisting of lower haloalkyl, halogen, hydroxy, amino, cyano, and lower alkoxy; $Y^{4c}$ is lower cycloalkyl, optionally substituted with one or more substituents selected from the group consisting of lower alkyl, lower haloalkyl, halogen, hydroxy, amino, cyano, and lower alkoxy; and $Y^{4d}$ is amino, optionally susbstituted with one or more lower alkyl, alkoxy lower alkyl, or hydroxy lower alkyl; |

or a pharmaceutically acceptable salt thereof.

In one variation of Formula II, $Y^1$ is methyl.

In one variation of Formula II, X is CH.

In one variation of the above compound, n is 1 and m is 0.

In one variation of Formula II, $Y^3$ is H.

In one variation of Formula II, $Y^2$ is methyl. In one variation of Formula II, $Y^2$ is hydroxymethyl. In one variation of Formula II, $Y^2$ is hydroxyethyl. In one variation of Formula II, $Y^2$ is halogen.

In one variation of Formula II, $Y^4$ is

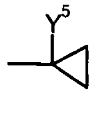

wherein $Y^5$ is halogen, lower alkyl or lower haloalkyl. In one variation of Formula II, $Y^4$ is

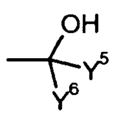

wherein $Y^5$ and $Y^6$ are independently H, lower alkyl, or lower haloalkyl. In one variation of Formula II, $Y^4$ is

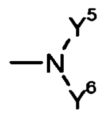

wherein $Y^5$ and $Y^6$ are independently H or lower alkyl. In one variation of Formula II, $Y^4$ is

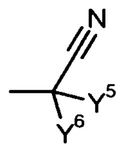

wherein, $Y^5$ and $Y^6$ are independently H, lower alkyl, or lower haloalkyl.

In one variation of Formula II, R is $-R^1-R^2-R^3$; $R^1$ is phenyl or pyridyl; $R^2$ is -C(=O); $R^3$ is $R^4$; and $R^4$ is morpholine or piperazine, optionally substituted with one or more lower alkyl.

In one variation, Formula II is 7-Dimethylamino-3-(2-methyl-3-{1-methyl-5-[5-(morpholine-4-carbonyl)-pyridin-2-ylamino]-6-oxo-1,6-dihydro-pyridin-3-yl}-phenyl)-1H-quinotin-4-one (**II-2**) of formula

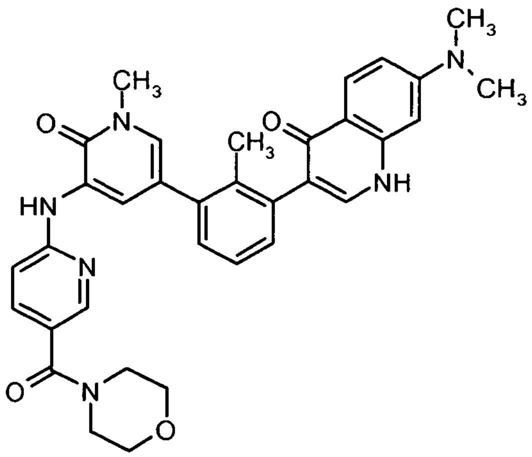

,

of formula

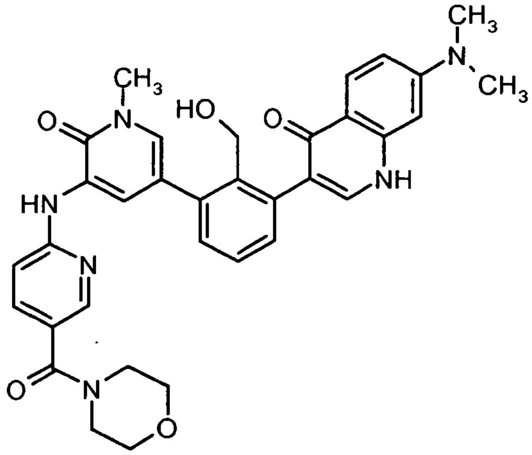

or 7-tert-Butyl-3-(3-{1-methyl-5-[5-(morpholine-4-carbonyl)-pyridin-2-ylamino]-6-oxo-1,6-dihydro-pyridin-3-yl}-phenyl)-1H-quinolin-4-one (II-I) of formula

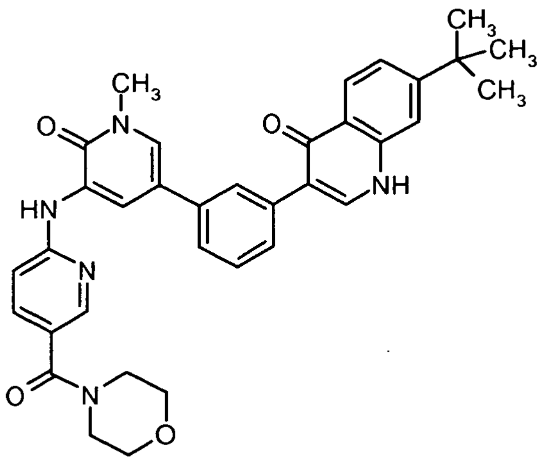

The application provides a compound of Formula III,

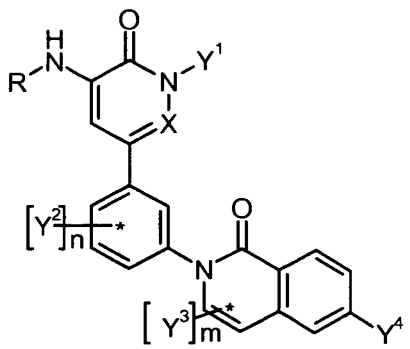

(III)

wherein:

| | |
|---|---|
| R | is H, $-R^1$, $-R^1-R^2-R^3$, or $-R^2-R^3$; |
| $R^1$ | is aryl, heteroaryl, cycloalkyl, or heterocycloalkyl, and is optionally substituted with lower alkyl, hydroxy, lower alkoxy, halo, nitro, amino, cyano, or halo-lower alkyl; |
| $R^2$ | is -C(=O), -C(=O)O, -C(=O)NH, or $-S(=O)_2$; |
| $R^3$ | is H or $R^4$; wherein $R^4$ is lower alkyl, aryl, arylalkyl, alkylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with lower alkyl, hydroxy, lower alkoxy, halo, nitro, amino, cyano, or halo-lower alkyl; |
| X | is CH or N; |
| $Y^1$ | is H or lower alkyl; |
| each $Y^2$ | is independently halogen or lower alkyl, wherein lower alkyl is optionally substituted with one or more substituents selected from the group consisting of hydroxy, lower alkoxy, and halogen; |
| n | is 0, 1, 2, or 3. |
| $Y^3$ | is H, halogen, or lower alkyl, wherein lower alkyl is optionally substituted with one or more substituents selected from the group consisting of hydroxy, lower alkoxy, amino, and halogen; |
| m | is 0 or 1; |
| $Y^4$ | is $Y^{4a}$, $Y^{4b}$, $Y^{4c}$, or $Y^{4d}$; $Y^{4a}$ is H or halogen; $Y^{4b}$ is lower alkyl, optionally substituted with one or more substituents selected from the group consisting of lower haloalkyl, halogen, hydroxy, amino, and lower alkoxy; $Y^{4c}$ is lower cycloalkyl, optionally substituted with one or more substituents selected from the group consisting of lower alkyl, lower haloalkyl, halogen, hydroxy, amino, and lower alkoxy; and $Y^{4d}$ is amino, optionally susbstituted with one or more lower alkyl; |

or a pharmaceutically acceptable salt thereof

The application also provides a compound of Formula III,

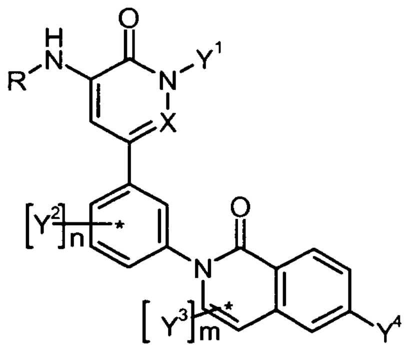

(III)

wherein:

| | |
|---|---|
| R | is H, $-R^1$, $-R^1-R^2-R^3$, $-R^1-R^3$, or $-R^2-R^3$; |
| $R^1$ | is aryl, heteroaryl, cycloalkyl, or heterocycloalkyl, and is optionally substituted with one or more lower alkyl, hydroxy, hydroxy lower alkyl, lower alkoxy, halo, nitro, amino, amido, cyano, oxo, or halo-lower alkyl; |
| $R^2$ | is -C(=O), -O, -C(=O)O, $-C(=O)NR^2$, or $-S(=O)_2$; wherein $R^2$ is H or lower alkyl; |
| $R^3$ | is H or $R^4$; wherein $R^4$ is lower alkyl, amino, aryl, arylalkyl, alkylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with one or more lower alkyl, hydroxy, hydroxy lower alkyl, lower alkoxy, halo, nitro, amino, amido, acyl, cyano, or halo-lower alkyl; |
| X | is CH or N; |
| $Y^1$ | is H, lower alkyl, or lower haloalkyl; |
| each $Y^2$ | is independently halogen or lower alkyl, wherein lower alkyl is optionally substituted with one or more substituents selected from the group consisting of hydroxy, lower alkoxy, lower haloalkoxy, lower haloalkyl, carboxy, amino, and halogen; |
| n | is 0, 1, 2, or 3. |
| $Y^3$ | is H, halogen, or lower alkyl, wherein lower alkyl is optionally substituted with one or more substituents selected from the group consisting of hydroxy, lower alkoxy, amino, and halogen; |
| m | is 0 or 1; |
| $Y^4$ | is $Y^{4a}$, $Y^{4b}$, $Y^{4c}$, or $Y^{4d}$; $Y^{4a}$ is H or halogen; $Y^{4b}$ is lower alkyl, optionally substituted with one or more substituents selected from the group consisting of haloalkyl, halogen, hydroxy, amino, cyano, and lower alkoxy; $Y^{4c}$ is lower cycloalkyl, optionally substituted with one or more substituents selected from the group consisting of lower alkyl, lower haloalkyl, halogen, hydroxy, hydroxy lower alkyl, amino, cyano, and lower alkoxy; and $Y^{4d}$ is amino, optionally substituted with one or more lower alkyl, alkoxy lower alkyl, or hydroxy lower alkyl; |

or a pharmaceutically acceptable salt thereof

In one variation of Formula III, $Y^1$ is methyl.

In one variation of Formula III, X is CH.

In one variation of Formula III, n is 1 and m is 0.

In one variation of Formula III, $Y^3$ is H.

In one variation of Formula III, $Y^2$ is methyl. In one variation of Formula III, $Y^2$ is hydroxymethyl. In one variation of Formula III, $Y^2$ is hydroxyethyl. In one variation of Formula III, $Y^2$ is halogen.

In one variation of Formula III, $Y^4$ is

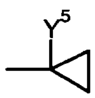

wherein $Y^5$ is halogen, lower alkyl or lower haloalkyl. In one variation of Formula III, $Y^4$ is

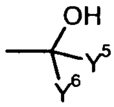

wherein $Y^5$ and $Y^6$ are independently H, lower alkyl, or lower haloalkyl. In one variation of Formula III, $Y^4$ is

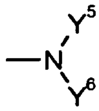

wherein $Y^5$ and $Y^6$ are independently H or lower alkyl. In one variation of Formula III, $Y^4$ is

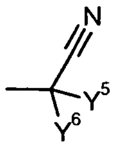

wherein, $Y^5$ and $Y^6$ are independently H, lower alkyl, or lower haloalkyl.

In one variation of Formula III, R is $-R^1-R^2-R^3$; $R^1$ is phenyl or pyridyl; $R^2$ is -C(=O); $R^3$ is $R^4$; and $R^4$ is morpholine or piperazine, optionally substituted with one or more lower alkyl.

In one variation, the compound of Formula III is 6-Dimethylamino-2-(2-hydroxymethyl-3-{1-methyl-5-[5-(morpholine-4-carbonyl)-pyridin-2-ylamino]-6-oxo-1,6-dihydro-pyridin-3-yl}-phenyl)-2H-isoquinolin-1-one (**III-3**) of formula

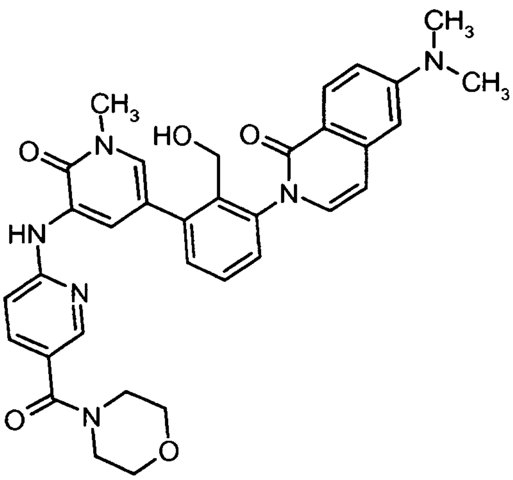

,

6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-2H-isoquinolin-1-one (**III-2**) of formula

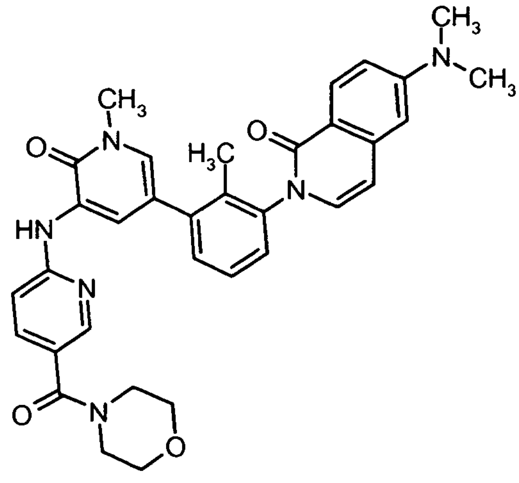

,

or 6-Dimethylamino-2-(3-{1-methyl-5-[5-(morpholine-4-carbonyl)-pyridin-2-ylamino]-6-oxo-1,6-dihydro-pyridin-3-yl}-phenyl)-2H-isoquinolin-1-one (**III-1**) of formula

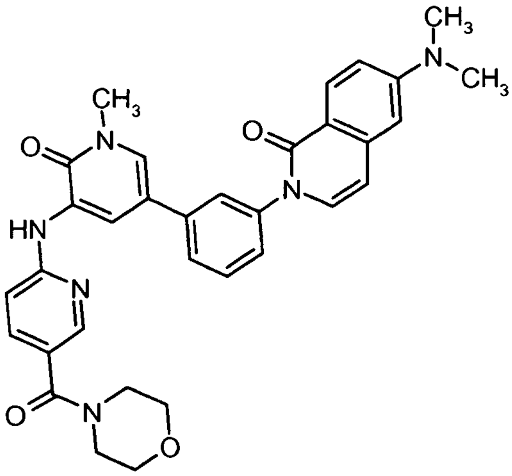

.

The application provides a method for treating an inflammatory and/or autoimmune condition comprising administering to a patient in need thereof a therapeutically effective amount of the Btk inhibitor compound of any one of the above Formulae or variations thereof.

The application provides a method for treating an arthritis comprising administering to a patient in need thereof a therapeutically effective amount of the Btk inhibitor compound of any one of the above Formulae or variations thereof

The application provides a method of inhibiting B-cell proliferation comprising administering to a patient in need thereof a therapeutically effective amount of the Btk inhibitor compound of any one of the above Formulae or variations thereof

The application provides a method for inhibiting Btk activity comprising administering the Btk inhibitor compound of any one of the above Formulae or variations thereof, wherein the Btk inhibitor compound exhibits an $IC_{50}$ of 50 micromolar or less in an *in vitro* biochemical assay of Btk activity.

In one variation of the above method, the Btk inhibitor compound exhibits an $IC_{50}$ of 100 nanomolar or less in an *in vitro* biochemical assay of Btk activity.

In one variation of the above method, the compound exhibits an $IC_{50}$ of 10 nanomolar or less in an *in vitro* biochemical assay of Btk activity.

The application provides a method for treating an inflammatory condition comprising co-administering to a patient in need thereof a therapeutically effective amount of an anti-inflammatory compound in combination with the Btk inhibitor compound of any one of the above Formulae or variations thereof.

The application provides a method for treating arthritis comprising co-administering to a patient in need thereof a therapeutically effective amount of an anti-inflammatory compound in combination with the Btk inhibitor compound of any one of the above Formulae or variations thereof.

The application provides a method for treating a lymphoma or a BCR-ABL1$^+$ leukemia cells by administering to a patient in need thereof a therapeutically effective amount of the Btk inhibitor compound of any one of the above Formulae or variations thereof

The application provides a pharmaceutical composition comprising the Btk inhibitor compound of any one of the above Formulae or variations thereof, admixed with at least one pharmaceutically acceptable carrier, excipient or diluent.

The present application provides compounds of generic Formulae I-III, wherein variables R, X, $Y^1$, $Y^2$, $Y^3$, $Y^4$, n, and m are as defined herein above.

In one embodiment of the present invention, there is provided a compound according to generic Formula I which comprises the exemplified Btk inhibitor compounds of Formulae I-1 to I-155. In one embodiment of the present invention, there is provided a compound according to generic Formula II which comprises the exemplified Btk inhibitor compounds of Formulae II-1 and II-2. In one embodiment of the present invention, there is provided a compound according to generic Formula III which comprises the exemplified Btk inhibitor compounds of Formulae III-1 to III-36.

The present application discloses 5-phenyl-1H-pyridin-2-one and 6-phenyl-2H-pyridazin-3-one derivatives according to generic Formulae I-III

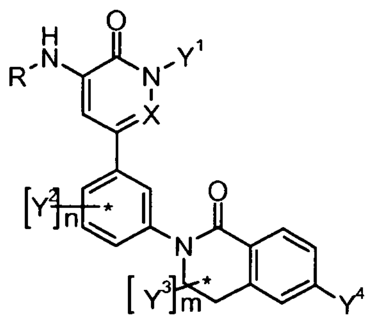

(I)

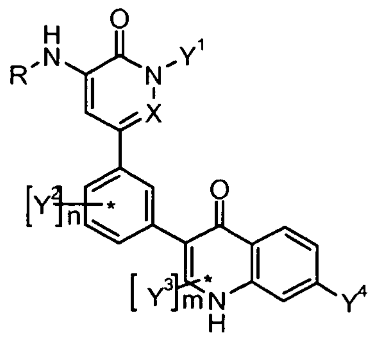

(II)

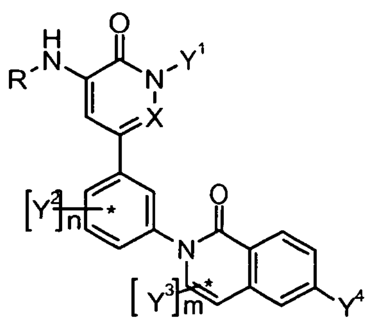

(III)

The phrase "as defined herein above" refers to the broadest definition for each group as provided herein or the broadest claim. In all other aspects, variations and embodiments provided, substituents which can be present in each embodiment and which are not explicitly defined retain the broadest definition provided in the Summary of the Invention.

The compounds of generic Formulae I-III inhibit Bruton's tyrosine kinase (Btk). Activation of Btk by upstream kinases results in activation of phospholipase-Cy which, in turn, stimulates release of pro-inflammatory mediators. The compounds of generic Formulae I-III, incorporating side chains of IH-quinolin-4-one, 3,4-dihydro-2H-isoquinolin-I-one, and 2H-isoquinolin-1-one on the 5-phenyl-1H-pyridin-2-one and 6-phenyl-2H-pyridazin-3-one ring systems, exhibit unexpectedly enhanced inhibitory activity compared to analogues with other side chains. Compounds of Formulae I-III are useful in the treatment of arthritis and other anti-inflammatory and auto-immune diseases. Compounds according to Formulae I-III are, accordingly, useful for the treatment of arthritis. Compounds of Formulae I-III are useful for inhibiting Btk in cells and for modulating B-cell development. The present invention further comprises pharmaceutical compositions containing compounds of Formulae I-III admixed with pharmaceutically acceptable carrier, excipients or diluents.

The phrase "a" or "an" entity as used herein refers to one or more of that entity; e.g., a compound refers to one or more compounds or at least one compound. As such, the terms "a" (or "an"), "one or more", and "at least one" can be used interchangeably herein.

The phrase "as defined herein above" refers to the broadest definition for each group as provided in the Summary of the Invention or the broadest claim. In all other embodiments provided below, substituents which can be present in each embodiment and which are not explicitly defined retain the broadest definition provided in the Summary of the Invention.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, unless specifically indicated otherwise, the word "or" is used in the "inclusive" sense of "and/or" and not the "exclusive" sense of "either/or".

The term "independently" is used herein to indicate that a variable is applied in any one instance without regard

to the presence or absence of a variable having that same or a different definition within the same compound. Thus, in a compound in which R" appears twice and is defined as "independently carbon or nitrogen", both R"s can be carbon, both R"s can be nitrogen, or one R" can be carbon and the other nitrogen.

When any variable occurs more than one time in any moiety or formula depicting and describing compounds employed or claimed in the present invention, its definition on each occurrence is independent of its definition at every other occurrence. Also, combinations of substituents and/or variables are permissible only if such compounds result in stable compounds.

The symbols "*" at the end of a bond or "------" drawn through a bond each refer to the point of attachment of a functional group or other chemical moiety to the rest of the molecule of which it is a part. Thus, e.g.

MeC(=O)OR$^4$ wherein R$^4$ = * 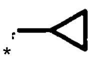 or 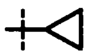 ⇒ MeC(=O)O 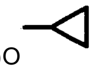 .

A bond drawn into ring system (as opposed to connected at a distinct vertex) indicates that the bond may be attached to any of the suitable ring atoms

The term "optional" or "optionally" as used herein means that a subsequently described event or circumstance may, but need not, occur, and that the description includes instances where the event or circumstance occurs and instances in which it does not. For example, "optionally substituted" means that the optionally substituted moiety may incorporate a hydrogen or a substituent.

The phrase "optional bond" means that the bond may or may not be present, and that the description includes single, double, or triple bonds. If a substituent is designated to be a "bond" or "absent", the atoms linked to the substituents are then directly connected.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Certain compounds of formulae I-III may exhibit tautomerism. Tautomeric compounds can exist as two or more interconvertable species. Prototropic tautomers result from the migration of a covalently bonded hydrogen atom between two atoms. Tautomers generally exist in equilibrium and attempts to isolate an individual tautomers usually produce a mixture whose chemical and physical properties are consistent with a mixture of compounds. The position of the equilibrium is dependent on chemical features within the molecule. For example, in many aliphatic aldehydes and ketones, such as acetaldehyde, the keto form predominates while; in phenols, the enol form predominates. Common prototropic tautomers include keto/enol (-C(=O)-CH- ↔ -C(-OH)=CH-), amide/imidic acid (-C(=O)-NH- ↔ -C(-OH)=N-) and amidine (-C(=NR)-NH-↔ -C(-NHR)=N-) tautomers. The latter two are particularly common in heteroaryl and heterocyclic rings and the present invention encompasses all tautomeric forms of the compounds.

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001). Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

The definitions described herein may be appended to form chemically-relevant combinations, such as "heteroalkylaryl," "haloalkylheteroaryl," "arylalkylheterocyclyl," "alkylcarbonyl," "alkoxyalkyl," and the like. When the term "alkyl" is used as a suffix following another term, as in "phenylalkyl," or "hydroxyalkyl," this is intended to refer to an alkyl group, as defined above, being substituted with one to two substituents selected from the other specifically-named group. Thus, e.g., "phenylalkyl" refers to an alkyl group having one to two phenyl substituents, and thus includes benzyl, phenylethyl, and biphenyl. An "alkylaminoalkyl" is an alkyl group having one to two alkylamino substituents. "Hydroxyalkyl" includes 2-hydroxyethyl, 2-hydroxypropyl, 1-(hydroxymethyl)-2-methylpropyl, 2-hydroxybutyl, 2,3-dihydroxybutyl, 2-(hydroxymethyl), 3-hydroxypropyl, and so forth. Accordingly, as used herein, the term "hydroxyalkyl" is used to define a subset of heteroalkyl groups defined below. The term -(ar)alkyl refers to either an unsubstituted alkyl or an aralkyl group. The term (hetero)aryl or (het)aryl refers to either an aryl or a heteroaryl group.

The term "acyl" as used herein denotes a group of formula -C(=O)R wherein R is hydrogen or lower alkyl as defined herein. The term "alkylcarbonyl" as used herein denotes a group of formula C(=O)R wherein R is alkyl as defined herein. The term $C_{1-6}$acyl refers to a group -C(=O)R wherein R is hydrogen or $C_{1-5}$alkyl. The term "arylcarbonyl" as used

herein means a group of formula C(=O)R wherein R is an aryl group; the term "benzoyl" as used herein an "arylcarbonyl" group wherein R is phenyl.

The term "alkyl" as used herein denotes an unbranched or branched chain, saturated, monovalent hydrocarbon residue containing 1 to 10 carbon atoms. The term "lower alkyl" denotes a straight or branched chain hydrocarbon residue containing 1 to 6 carbon atoms. "$C_{1-10}$ alkyl" as used herein refers to an alkyl composed of 1 to 10 carbons. Examples of alkyl groups include, but are not limited to, lower alkyl groups include methyl, ethyl, propyl, i-propyl, n-butyl, i-butyl, t-butyl or pentyl, isopentyl, neopentyl, hexyl, heptyl, and octyl.

When the term "alkyl" is used as a suffix following another term, as in "phenylalkyl," or "hydroxyalkyl," this is intended to refer to an alkyl group, as defined above, being substituted with one to two substituents selected from the other specifically-named group. Thus, e.g., "phenylalkyl" denotes the radical R'R"-, wherein R' is a phenyl radical, and R" is an alkylene radical as defined herein with the understanding that the attachment point of the phenylalkyl moiety will be on the alkylene radical. Examples of arylalkyl radicals include, but are not limited to, benzyl, phenylethyl, 3-phenylpropyl. The terms "arylalkyl" or "aralkyl" are interpreted similarly except R' is an aryl radical. The terms "(het) arylalkyl" or "(het)aralkyl" are interpreted similarly except R' is optionally an aryl or a heteroaryl radical.

The terms "haloalkyl" or "halo-lower alkyl" or "lower haloalkyl" refers to a straight or branched chain hydrocarbon residue containing 1 to 6 carbon atoms wherein one or more carbon atoms are substituted with one or more halogen atoms.

The term "alkylene" or "alkylenyl" as used herein denotes a divalent saturated linear hydrocarbon radical of 1 to 10 carbon atoms (*e.g.*, $(CH_2)_n$) or a branched saturated divalent hydrocarbon radical of 2 to 10 carbon atoms (*e.g.*, -CHMe- or $-CH_2CH(i\text{-}Pr)CH_2-$), unless otherwise indicated. Except in the case of methylene, the open valences of an alkylene group are not attached to the same atom. Examples of alkylene radicals include, but are not limited to, methylene, ethylene, propylene, 2-methyl-propylene, 1,1-dimethyl-ethylene, butylene, 2-ethyl-butylene.

The term "alkoxy" as used herein means an -O-alkyl group, wherein alkyl is as defined above such as methoxy, ethoxy, *n*-propyloxy, *i*-propyloxy, *n*-butyloxy, *i*-butyloxy, *t*-butyloxy, pentyl-oxy, hexyloxy, including their isomers. "Lower alkoxy" as used herein denotes an alkoxy group with a "lower alkyl" group as previously defined. "$C_{1-10}$ alkoxy" as used herein refers to an-O-alkyl wherein alkyl is $C_{1-10}$.

The term "hydroxyalkyl" as used herein denotes an alkyl radical as herein defined wherein one to three hydrogen atoms on different carbon atoms is/are replaced by hydroxyl groups.

The terms "alkylsulfonyl" and "arylsulfonyl" as used herein refers to a group of formula $-S(=O)_2R$ wherein R is alkyl or aryl respectively and alkyl and aryl are as defined herein. The term "heteroalkylsulfonyl" as used herein refers herein denotes a group of formula $-S(=O)_2R$ wherein R is "heteroalkyl" as defined herein.

The terms "alkylsulfonylamino" and "arylsulfonylamino"as used herein refers to a group of formula $-NR'S(=O)_2R$ wherein R is alkyl or aryl respectively, R' is hydrogen or $C_{1-3}$alkyl, and alkyl and aryl are as defined herein.

The term "cycloalkyl" as used herein refers to a saturated carbocyclic ring containing 3 to 8 carbon atoms, i.e. cyclopropyl, cyclobutyl, cyclopentyl, cyclohexyl, cycloheptyl or cyclooctyl. "$C_{3-7}$cycloalkyl" as used herein refers to an cycloalkyl composed of 3 to 7 carbons in the carbocyclic ring.

The term carboxy-alkyl as used herein refers to an alkyl moiety wherein one, hydrogen atom has been replaced with a carboxyl with the understanding that the point of attachment of the heteroalkyl radical is through a carbon atom. The term "carboxy" or "carboxyl" refers to a $-CO_2H$ moiety.

The term "heteroaryl" or "heteroaromatic" as used herein means a monocyclic or bicyclic radical of 5 to 12 ring atoms having at least one aromatic ring containing four to eight atoms per ring, incorporating one or more N, O, or S heteroatoms, the remaining ring atoms being carbon, with the understanding that the attachment point of the heteroaryl radical will be on an aromatic ring. As well known to those skilled in the art, heteroaryl rings have less aromatic character than their all-carbon counter parts. Thus, for the purposes of the invention, a heteroaryl group need only have some degree of aromatic character. Examples of heteroaryl moieties include monocyclic aromatic heterocycles having 5 to 6 ring atoms and 1 to 3 heteroatoms include, but is not limited to, pyridinyl, pyrimidinyl, pyrazinyl, pyrrolyl, pyrazolyl, imidazolyl, oxazol, isoxazole, thiazole, isothiazole, triazoline, thiadiazole and oxadiaxoline which can optionally be substituted with one or more, preferably one or two substituents selected from hydroxy, cyano, alkyl, alkoxy, thio, lower haloalkoxy, alkylthio, halo, lower haloalkyl, alkylsulfinyl, alkylsulfonyl, halogen, amino, alkylamino,dialkylamino, aminoalkyl, alkylaminoalkyl, and dialkylaminoalkyl, nitro, alkoxycarbonyl and carbamoyl, alkylcarbamoyl, dialkylcarbamoyl, arylcarbamoyl, alkylcarbonylamino and arylcarbonylamino. Examples ofbicyclic moieties include, but are not limited to, quinolinyl, isoquinolinyl, benzofuryl, benzothiophenyl, benzoxazole, benzisoxazole, benzothiazole and benzisothiazole. Bicyclic moieties can be optionally substituted on either ring; however the point of attachment is on a ring containing a heteroatom.

The term "heterocyclyl", "heterocycloalkyl" or "heterocycle" as used herein denotes a monovalent saturated cyclic radical, consisting of one or more rings, preferably one to two rings, including spirocyclic ring systems, of three to eight atoms per ring, incorporating one or more ring heteroatoms (chosen from N,O or $S(O)_{0-2}$), and which can optionally be independently substituted with one or more, preferably one or two substituents selected from hydroxy, oxo, cyano, lower alkyl, lower alkoxy, lower haloalkoxy, alkylthio, halo, lower haloalkyl, hydroxyalkyl, nitro, alkoxycarbonyl, amino,

alkylamino, alkylsulfonyl, arylsulfonyl, alkylaminosulfonyl, aryl-aminosulfonyl, alkylsulfonylamino, arylsulfonylamino, alkylaminocarbonyl, arylaminocarbonyl, alkylcarbonylamino, arylcarbonylamino, unless otherwise indicated. Examples of heterocyclic radicals include, but are not limited to, azetidinyl, pyrrolidinyl, hexahydroazepinyl, oxetanyl, tetrahydrofuranyl, tetrahydrothiophenyl, oxazolidinyl, thiazolidinyl, isoxazolidinyl, morpholinyl, piperazinyl, piperidinyl, tetrahydropyranyl, thiomorpholinyl, quinuclidinyl and imidazolinyl.

Commonly used abbreviations include: acetyl (Ac), azo-*bis*-isobutyrylnitrile (AIBN), atmospheres (Atm), 9-borabicyclo[3.3.1]nonane (9-BBN or BBN), *tert*-butoxycarbonyl (Boc), di-*tert*-butyl pyrocarbonate or boc anhydride ($BOC_2O$), benzyl (Bn), butyl (Bu), Chemical Abstracts Registration Number (CASRN), benzyloxycarbonyl (CBZ or Z), carbonyl diimidazole (CDI), 1,4-diazabicyclo[2.2.2]octane (DABCO), diethylaminosulfur trifluoride (DAST), dibenzylideneacetone (dba), 1,5-diazabicyclo[4.3.0]non-5-ene (DBN), 1,8-diazabicyclo[5.4.0]undec-7-ene (DBU), N,N'-dicyclohexylcarbodiimide (DCC), 1,2-dichloroethane (DCE), dichloromethane (DCM), diethyl azodicarboxylate (DEAD), di-*iso*-propylazodicarboxylate (DIAD), di-*iso*-butyl-aluminumhydride (DIBAL or DIBAL-H), di-iso-propylethylamine (DIPEA), N,N-dimethyl acetamide (DMA), 4-N,N-dimethylaminopyridine (DMAP), N,N-dimethylformamide (DMF), dimethyl sulfoxide (DMSO), 1,1'-*bis*-(diphenylphosphino)ethane (dppe), 1,1'-*bis*-(diphenyl-phosphino)ferrocene (dppf), 1-(3-dimethylaminopropyl)-3-ethylcarbodiimide hydrochloride (EDCI), ethyl (Et), ethyl acetate (EtOAc), ethanol (EtOH), 2-ethoxy-2*H*-quinoline-1-carboxylic acid ethyl ester (EEDQ), diethyl ether ($Et_2O$), O-(7-azabenzotriazole-1-yl)-N, N,N'N'-tetra-methyluronium hexafluorophosphate acetic acid (HATU), acetic acid (HOAc), 1-N-hydroxybenzotriazole (HOBt), high pressure liquid chromatography (HPLC), *iso*-propanol (IPA), lithium hexamethyl disilazane (LiHMDS), methanol (MeOH), melting point (mp), $MeSO_2$- (mesyl or Ms), , methyl (Me), acetonitrile (MeCN), *m*-chloroperbenzoic acid (MCPBA), mass spectrum (ms), methyl *t*-butyl ether (MTBE), N-bromosuccinimide (NBS), N-carboxyanhydride (NCA), N-chlorosuccinimide (NCS), N-methylmorpholine (NMM), N-methylpyrrolidone (NMP), pyridinium chlorochromate (PCC), pyridinium dichromate (PDC), phenyl (Ph), propyl (Pr), *iso*propyl (*i*-Pr), pounds per square inch (psi), pyridine (pyr), room temperature (rt or RT), *tert*butyldimethylsilyl or *t*-$BuMe_2Si$ (TBDMS), triethylamine (TEA or $Et_3N$), 2,2,6,6-tetramethylpiperidine 1-oxyl (TEMPO), triflate or $CF_3SO_2$- (Tf), trifluoroacetic acid (TFA), 1,1'-*bis*-2,2,6,6-tetramethylheptane-2,6-dione (TMHD), O-benzotriazol-1-yl-N,N,N',N'-tetramethyluronium tetrafluoroborate (TBTU), thin layer chromatography (TLC), tetrahydrofuran (THF), trimethylsilyl or $Me_3Si$ (TMS), *p*-toluenesulfonic acid monohydrate (TsOH or pTsOH), 4-Me-$C_6H_4SO_2$-or tosyl (Ts), N-urethane-N-carboxyanhydride (UNCA). Conventional nomenclature including the prefixes *normal* (*n*), *iso* (*i*-), *secondary* (*sec*-), *tertiary* (*tert*-) and *neo* have their customary meaning when used with an alkyl moiety. (Rigaudy and Klesney, Nomenclature in Organic Chemistry, IUPAC 1979 Pergamon Press, Oxford.).

Examples of representative compounds encompassed by the present invention and within the scope of the invention are provided in the following Table. These examples and preparations which follow are provided to enable those skilled in the art to more clearly understand and to practice the present invention. They should not be considered as limiting the scope of the invention, but merely as being illustrative and representative thereof.

In general, the nomenclature used in this Application is based on AUTONOMTM v.4.0, a Beilstein Institute computerized system for the generation of IUPAC systematic nomenclature. If there is a discrepancy between a depicted structure and a name given that structure, the depicted structure is to be accorded more weight. In addition, if the stereochemistry of a structure or a portion of a structure is not indicated with, e.g., bold or dashed lines, the structure or portion of the structure is to be interpreted as encompassing all stereoisomers of it.

TABLE I depicts examples ofpyridinone compounds according to generic Formulae **I-III** wherein **X** either is CH or N. The compounds may be prepared according to the procedures as described in the Examples.

TABLE I.

| | **Nomenclature** | **$(M+H)^+$** |
|---|---|---|
| I-1 | 6-Dimethylamino-2-(3-{1-methyl-5-[5-(morpholine-4-carbonyl)-pyridin-2-ylamino]-6-oxo-1,6-dihydro-pyridin-3-yl}-phenyl)-3,4-dihydro-2H-isoquin-olin-1-one | 579.2 |
| I-2 | 6-Dimethylamino-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoqumotin-1-one | 609.1 |
| I-3 | 6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one | 593.3 |
| II-1 | 7-tert-Butyl-3-(3-{1-methyl-5-[5-(morpholine-4-carbonyl)-pyridin-2-yl-amino]-6-oxo-1,6-dihydro-pyridin-3-yl}-phenyl)-1H-quinolin-4-one | 590.1 |
| II-2 | 7-Dimethylamino-3-(2-methyl-3-{1-methyl-5-[5-(morpholine-4-carbonyl)-pyridin-2-ylamino]-6-oxo-1,6-dihydro-pyridin-3-yl}-phenyl)-1H-quinolin-4-one | 591.1 |

(continued)

| | Nomenclature | $(M+H)^+$ |
|---|---|---|
| III-1 | 6-Dimethylamino-2-(3-{1-methyl-5-[5-(morpholine-4-carbonyl)-pyridin-2-ylamino]-6-oxo-1,6-dihydro-pyridin-3-yl}-phenyl)-2H-isoquinolin-1-one | 577.1 |
| III-2 | 6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-2H-isoquin-olin-1-one | 591.1 |
| III-3 | 6-Dimethylamino-2-(2-hydroxymethyl-3-{1-methyl-5-[5-(morpholine-4-carbonyl)-pyridin-2-ylamino]-6-oxo-1,6-dihydro-pyridin-3-yl}-phenyl)-2H-isoquinolin-1-one | 607.2 |
| I-4 | 1-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-yl}-3-ethyl-urea | |
| I-5 | 6-Dimethylamino-2-{2-hydroxymethyl-3-[1-methyl-5-(1-methyl-1*H*-pyrazol-3-ylamino)-6-oxo-1,6-dihydro-pyridin-3-yl]-phenyl}-3,4-dihydro-2*H*-isoquinolin-1-one | |
| I-6 | 6-Cyclopropyl-2-(2-hydroxymethyl-3-{1-methyl-5-[5-(morpholine-4-carb-onyl)-pyridin-2-ylamino]-6-oxo-1,6-dihydro-pyridin-3-yl}-phenyl)-3,4-di-hydro-2*H*-isoquinolin-1-one | |
| I-7 | 2-(2-Difluoromethoxy-3-{1-methyl-5-[5-(morpholine-4-carbonyl)-pyridin-2-ylamino]-6-oxo-1,6-dihydro-pyridin-3-yl}-phenyl)-6-dimethylamino-3,4-dihydro-2*H*-isoquinolin-1-one | |
| I-8 | 6-Dimethylamino-2-{2-hydroxymethyl-3-[1-methyl-5-(5-morpholin-4-yl-methyl-pyridin-2-ylamino)-6-oxo-1,6-dihydro-pyridin-3-yl]-phenyl}-3,4-di-hydro-2*H*-isoquinolin-1-one | |
| I-9 | 6-Dimethylamino-2-(2-hydroxymethyl-3-{1-methyl-5-[5-(4-methyl-piper-azine-1-carbonyl)-pyridin-2-ylamino]-6-oxo-1,6-dihydro-pyridin-3-yl}-phenyl)-3,4-dihydro-2*H*-isoquinolin-1-one | |
| I-10 | 2-(2-Hydroxymethyl-3-{1-methyl-5-[5-(morpholine-4-carbonyl)-pyridin-2-ylamino]-6-oxo-1,6-dihydro-pyridin-3-yl}-phenyl)-6-methylamino-3,4-di-hydro-2*H*-isoquinolin-1-one | |
| I-11 | 2-{3-[5-(1-*tert*-Butyl-1*H*-pyrazol-3-ylamino)-1-methyl-6-oxo-1,6-dlhydro-pyridin-3-yl]-2-hydroxymethyl-phenyl}-6-cyclopropyl-3,4-dihydro-2*H*-iso-quinolin-1-one | |
| I-12 | 6-Cyclopropyl-2-{3-[5-(1-ethyl-1*H*-pyrazol-3-ylamino)-1-methyl-6-oxo-1,6-dihydro-pyridin-3-yl]-2-hydroxymethyl-phenyl}-3,4-dihydro-2H-iso-quinolin-1-one | |
| I-13 | 2-{3-[5-(1-Cyctohexyl-1*H*-pyrazol-3-ylamino)-1-methyl-6-oxo-1,6-di-hydro-pyridin-3-yl]-2-hydroxymethyl-phenyl}-6-cyclopropyl-3,4-dihydro-2*H*-isoquinolin-1-one | |
| I-14 | 6-Cyclopropyl-2-(2-hydroxymethyl-3-{1-methyl-6-oxo-5-[1-(2,2,2-tri-fluoro-ethyl)-1*H*-pyrazol-3-ylamino]-1,6-dihydro-pyridin-3-yl}-phenyl)-3,4-dihydro-2*H*-isoquinolin-1-one | |
| I-15 | 6-Cyclopropyl-2-{3-[5-(1-cyclopropyl-1*H*-pyrazol-3-ylamino)-1-methyl-6-oxo-1,6-dihydro-pyridin-3-yl]-2-hydroxymethyl-phenyl}-3,4-dihydro-2*H*-isoquinolin-1-one | |
| I-16 | 6-Dimethylamino-2-(2-hydroxymethyl-3-{1-methyl-5-[5-(4-methyl-piper-azin-1-yl)-pyridin-2-ylamino]-6-oxo-1,6-dihydro-pyridin-3-yl}-phenyl)-3,4-dihydro-2*H*-isoquinolin-1-one | |
| I-17 | 6-Cyclopropyl-2-{2-hydroxymethyl-3-[1-methyl-5-(1-methyl-1*H*-pyrazol-3-ylamino)-6-oxo-1,6-dihydro-pyridin-3-yl]-phenyl}-3,4-dihydro-2*H*-iso-quinolin-1-one | |
| I-18 | 6-Cyclopropyl-2-{2-hydroxymethyl-3-[5-(2-isopropyl-2*H*-[1,2,3]triazol-4-ylamino)-1-methyl-6-oxo-1,6-dihydro-pyridin-3-yl]-phenyl}-3,4-dihydro-2*H*-isoquinolin-1-one | |
| I-19 | 6-Cyclopropyl-2-{2-hydroxymethyl-3-[5-(1-isopropyl-1*H*-pyrazol-3-yl-amino)-1-methyl-6-oxo-1,6-dihydro-pyridin-3-yl]-phenyl}-3,4-dihydro-2*H*-isoquinolin-1-one | |
| I-20 | 6-(1-Fluoro-cyclopropyl)-2-(2-hydroxymethyl-3-{1-methyl-5-[5-(morpho-line-4-carbonyl)-pyridin-2-ylamino]-6-oxo-1,6-dihydro-pyridin-3-yl}-phenyl)-3,4-dihydro-2*H*-isoquinolin-1-one | |
| I-21 | 6-Dimethylamino-2-{2-hydroxymethyl-3-[1-methyl-5-(5-morpholin-4-yl-pyridin-2-ylamino)-6-oxo-1,6-dihydro-pyridin-3-yl]-phenyl}-3,4-dihydro-2*H*-isoquinolin-1-one | |
| 1-22 | 6-(Ethyl-methyl-amino)-2-(2-hydroxymethyl-3-{1-methyl-5-[5-(morpho-line-4-carbonyl)-pyridin-2-ylamino]-6-oxo-1,6-dihydro-pyridin-3-yl}-phenyl)-3,4-dihydro-2*H*-isoquinolin-1-one | |

(continued)

| | Nomenclature | $(M+H)^+$ |
|---|---|---|
| I-23 | 6-*tert*-Butyl-2-(2-hydroxymethyl-3-{1-methyl-5-[5-(morpholine-4-carbon-yl)-pyridin-2-ylamino]-6-oxo-1,6-dihydro-pyridin-3-yl}-phenyl)-3,4-di-hydro-2*H*-isoquinolin-1-one | |
| I-24 | 6-(1-Difluoromethyl-cyclopropyl)-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2*H*-isoquinolin-1-one | |
| I-25 | 6-Cyclopropyl-2-(2-hydroxymethyl-3-{1-methyl-5-[5-(4-methyl-piperazin-1-yl)-pyridin-2-ylamino]-6-oxo-1,6-dihydro-pyridin-3-yl}-phenyl)-3,4-di-hydro-2*H*-isoquinolin-1-one | |
| I-26 | 6-Fluoro-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2*H*-isoquinolin-1-one | |
| I-27 | 6-*tert*-Butyl-2-(2-hydroxymethyl-3-{1-methyl-5-[5-(4-methyl-piperazin-1-yl)-pyridin-2-ylamino]-6-oxo-1,6-dihydro-pyridin-3-yl}-phenyl)-3,4-di-hydro-2*H*-isoquinolin-1-one | |
| I-28 | 6-Cyclopropyl-2-(2-hydroxymethyl-3-{1-methyl-5-[6-(4-methyl-piperazin-1-yl)-pyridazin-3-ylamino]-6-oxo-1,6-dihydro-pyridin-3-yl}-phenyl)-3,4-dihydro-2*H*-isoquinolin-1-one | |
| I-29 | 2-(3-{5-[5-(4,4-Difluoro-pipendine-1-carbonyl)-pyridin-2-ylamino]-1-methyl-6-oxo-1,6-dihydro-pyridin-3-yl}-2-hydroxymethyl-phenyl)-6-di-methylamino-3,4-dihydro-2*H*-isoquinolin-1-one | |
| I-30 | 6-Dimethylamino-2-{2-hydroxymethyl-3-[1-methyl-5-(1-methyl-5-oxo-4,5-dihydro-1*H*-[1,2,4] triazol-3-ylamino)-6-oxo-1,6-dihydro-pyridin-3-yl]-phenyl}-3,4-dihydro-2*H*-isoquinolin-1-one | |
| I-31 | 6-Dimethylamino-2-(2-hydroxymethyl-3-{1-methyl-5-[5-(2-oxa-6-aza-spiro[3.3]heptane-6-carbonyl)-pyridin-2-ylamino]-6-oxo-1,6-dihydro-pyridin-3-yl}-phenyl)-3,4-dihydro-2*H*-isoquinolin-1-one | |
| I-32 | 6-Dimethylamino-2-(2-hydroxymethyl-3-{1-methyl-5-[6-(4-methyl-piperazin-1-yl)-pyridazin-3-ylamino]-6-oxo-1,6-dihydro-pyridin-3-yl}-phenyl)-3,4-dihydro-2*H*-isoqutnotin-1-one | |
| I-33 | 6-Dimethylamino-2-(3-{5-[5-(1,1-dioxo-1$\lambda^6$-thiomorpholine-4-carbonyl)-pyridin-2-ylamino]-1-methyl-6-oxo-1,6-dihydro-pyridin-3-yl}-2-hydroxy-methyl-phenyl)-3,4-dihydro-2*H*-isoquinolin-1-one | |
| I-34 | 6-Cyclopropyl-2-(2-hydroxymethyl-3-{5-[1-(2-hydroxy-2-methyl-propyl)-1*H*-pyrazol-3-ylamino]-1-methyl-6-oxo-1,6-dihydro-pyridin-3-yl}-phenyl)-3,4-dihydro-2*H*-isoquinolin-1-one | |
| I-35 | 6-Dimethylamino-2-{3-[5-(5-ethoxymethyl-pyridin-2-ylamino)-1-methyl-6-oxo-1,6-dihydro-pyridin-3-yl]-2-hydroxymethyl-phenyl}-3,4-dihydro-2*H*-isoquinolin-1-one | |
| I-36 | 6-Dimethylamino-2-(2-hydroxymethyl-3-{5-[5-(2-methoxy-ethoxymethyl)-pyridin-2-ylamino]-1-methyl-6-oxo-1,6-dihydro-pyridin-3-yl}-phenyl)-3,4-dihydro-2*H*-isoquinolin-1-one | |
| I-37 | 6-Dimethylamino-2-(3-{5-[5-(2-hydroxy-ethoxymethyl)-pyridin-2-yl-amino]-1-methyl-6-oxo-1,6-dihydro-pyridin-3-yl}-2-hydroxymethyl-phenyl)-3,4-dihydro-2*H*-isoquinolin-1-one | |
| I-38 | 6-Cyclopropyl-2-(3-{5-[1-(2-hydroxy-ethyl)-1*H*-pyrazol-3-ylamino]-1-methyl-6-oxo-1,6-dihydro-pyridin-3-yl}-2-hydroxymethyl-phenyl)-3,4-di-hydro-2*H*-isoquinolin-1-one | |
| I-39 | 6-D imethylamino-2-{2-hydroxymethyl-3-[5-(5-methanesulfonylmethyl-pyridin-2-ylamino)-1-methyl-6-oxo-1,6-dihydro-pyridin-3-yl]-phenyl}-3,4-dihydro-2*H*-isoquinolin-1-one | |
| I-40 | 6-Dimethylamino-2-(2-hydroxymethyl-3-{1-methyl-6-oxo-5-[5-(2-oxo-pyrrolidin-1-ylmethyl)-pyridin-2-ylamino]-1,6-dihydro-pyridin-3-yl}-phenyl)-3,4-dihydro-2*H*-isoquinolin-1-one | |
| I-41 | 6-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-*N*-(2-methoxy-ethyl)-*N*-methyl-nicotinamide | |
| I-42 | 2-(3-{5-[5-(4,4-Difluoro-pipendin-1-ylmethyl)-pyridin-2-ylamino]-1-methyl-6-oxo-1,6-dihydro-pyridin-3-yl}-2-hydroxymethyl-phenyl)-6-di-methylamino-3,4-dihydro-2*H*-isoquinolin-1-one | |
| I-43 | 6-Cyclopropyl-2-(3-{5-[1-(2-hydroxy-3-methoxy-propyl)-1*H*-pyrazol-3-yl-amino]-1-methyl-6-oxo-1,6-dihydro-pyridin-3-yl}-2-hydroxymethyl-phenyl)-3,4-dihydro-2*H*-isoquinolin-1-one | |

(continued)

| | Nomenclature | $(M+H)^+$ |
|---|---|---|
| I-44 | 6-Cyclopropyl-2-(2-hydroxymethyl-3-{1-methyl-5-[1-(2-morpholin-4-yl-methyl)-1*H*-pyrazol-3-ylamino]-6-oxo-1,6-dihydro-pyridin-3-yl}-phenyl)-3,4-dihydro-2*H*-isoquinolin-1-one | |
| I-45 | 6-Dimethylamino-2-[3-(5-{5-[4-(2-hydroxy-ethyl)-piperazin-1-yl]-pyridin-2-ylamino}-1-methyl-6-oxo-1,6-dihydro-pyridin-3-yl)-2-hydroxymethyl-phenyl]-3,4-dihydro-2*H*-isoquinolin-1-one | |
| I-46 | 2-(2-Hydroxymethyl-3-{1-methyl-5-[5-(morpholine-4-carbonyl)-pyridin-2-ylamino]-6-oxo-1,6-dihydro-pyridin-3-yl}-phenyl)-6-(1-methyl-cyclo-propyl)-3,4-dihydro-2*H*-isoquinolin-1-one | |
| I-47 | 6-Dimethylamino-2-(3-{5-[5-(4-ethoxy-piperidine-1-carbonyl)-pyridin-2-ylamino]-1-methyl-6-oxo-1,6-dihydro-pyridin-3-yl}-2-hydroxymethyl-phenyl)-3,4-dihydro-2*H*-isoquinolin-1-one | |
| I-48 | 6-Dimethylamino-2-(2-hydroxymethyl-3-{5-[5-(4-methoxymethyl-piperi-dine-1-carbonyl)-pyridin-2-ylamino]-1-methyl-6-oxo-1,6-dihydro-pyridin-3-yl}-phenyl)-3,4-dihydro-2*H*-isoquinolin-1-one | |
| I-49 | 2-(3-{5-[5-(Azetid ine-1-carbonyl)-pyridin-2-ylamino]-1-methyl-6-oxo-1,6-dihydro-pyridin-3-yl}-2-hydroxymethyl-phenyl)-6-dimethylamino-3,4-di-hydro-2*H*-isoquinolin-1-one | |
| I-50 | 2-(3-{5-[5-(3,3-Difluoro-azetidine-1-carbonyl)-pyridin-2-ylamino]-1-methyl-6-oxo-1,6-dihydro-pyridin-3-yl}-2-hydroxymethyl-phenyl)-6-di-methylamino-3,4-dihydro-2*H*-isoquinolin-1-one | |
| I-51 | 6-Dimethylamino-2-(3-{5-[5-(1,1-dioxo-1$\lambda^6$-isothiazolidin-2-ylmethyl)-pyridin-2-ylamino]-1-methyl-6-oxo-1,6-dihydro-pyridin-3-yl}-2-hydroxy-methyl-phenyl)-3,4-dihydro-2*H*-isoquinolin-1-one | |
| I-52 | 6-*tert*-Butyl-2-{2-hydroxymethyl-3-[1-methyl-5-(5-morpholin-4-yl-pyridin-2-ylamino)-6-oxo-1,6-dihydro-pyridin-3-yl]-phenyl}-3,4-dihydro-2*H*-iso-quinolin-1-one | |
| I-53 | 6-*tert*-Butyl-2-(2-hydroxymethyl-3-{1-methyl-5-[5-(2-oxa-6-aza-spiro[3.3]-heptane-6-carbonyl)-pyridin-2-ylamino]-6-oxo-1,6-dihydro-pyridin-3-yl}-phenyl)-3,4-dihydro-2*H*-isoquinolin-1-one | |
| I-54 | 6-Dimethylamino-2-(3-{5-[5-(4-ethyl-piperazin-1-yl)-pyridin-2-ylamino]-1-methyl-6-oxo-1,6-dihydro-pyridin-3-yl}-2-hydroxymethyl-phenyl)-3,4-dihydro-2*H*-isoquinolin-1-one | |
| I-55 | 6-Dimethylamino-2-(3-{5-[5-(4-ethyl-piperazin-1-yl)-pyridin-2-ylamino]-1-methyl-6-oxo-1,6-dihydro-pyridin-3-yl}-2-hydroxymethyl-phenyl)-3,4-di-hydro-2*H*-isoquinolin-1-one | |
| I-56 | 6-Dimethylamino-2-(2-hydroxymethyl-3-(5-[5-(4-hydroxy-4-methyl-piperidine-1-carbonyl)-pyridin-2-ylamino]-1-methyl-6-oxo-1,6-dihydro-pyridin-3-yl}-phenyl)-3,4-dihydro-2*H*-isoquinolin-1-one | |
| I-57 | *N*-(6-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-3-ylmethyl)-methanesulfonamide | |
| I-58 | (6-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-3-ylmethyl)-carbamic acid methyl ester | |
| 1-59 | 6-Cyclopropyl-2-(2-hydroxymethyl-3-{1-methyl-5-[5-(2-oxa-6-aza-spiro-[3.3]heptane-6-carbonyl)-pyridin-2-ylamino]-6-oxo-1,6-dihydro-pyridin-3-yl}-phenyl)-3,4-dihydro-2*H*-isoquinolin-1-one | |
| I-60 | 6-Cyclopropyl-2-(3-{5-[5-(1,1-dioxo-1$\lambda^6$-thiomorpholine-4-carbonyl)-pyri-din-2-ylamino]-1-methyl-6-oxo-1,6-dihydro-pyridin-3-yl}-2-hydroxy-methyl-phenyl)-3,4-dihydro-2*H*-isoquinolin-1-one | |
| I-61 | 6-(Ethyl-methyl-amino)-2-{2-hydroxymethyl-3-[1-methyl-5-(5-morpholin-4-yl-pyridin-2-ylamino)-6-oxo-1,6-dihydro-pyridin-3-yl]-phenyl}-3,4-di-hydro-2*H*-isoquinolin-1-one | |
| I-62 | 6-Gyclopropyl-2-(3-{5-[1-(2-dimethylamino-ethyl)-1*H*-pyrazol-3-ylamino]-1-methyl-6-oxo-1,6-dihydro-pyridin-3-yl}-2-hydroxymethyl-phenyl)-3,4-dihydro-2*H*-isoquinolin-1-one | |
| I-63 | 6-(1-Hydroxy-1-methyl-ethyl)-2-(2-hydroxymethyl-3-{1-methyl-5-[5-(mor-pholine-4-carbonyl)-pyridin-2-ylamino]-6-oxo-1,6-dihydro-pyridin-3-yl}-phenyl)-3,4-dihydro-2*H*-isoquinolin-1-one | |

(continued)

| | Nomenclature | $(M+H)^+$ |
|---|---|---|
| I-64 | 6-[(2-Hydroxy-ethyl)-methyl-amino]-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2*H*-isoquinolin-1-one | |
| I-65 | 6-Dimethylamino-2-[3-(5-{5-[(2-hydroxy-ethyl)-methyl-amino]-pyridin-2-ylamino}-1-methyl-6-oxo-1,6-dihydro-pyridin-3-yl)-2-hydroxymethyl-phenyl]-3,4-dihydro-2*H*-isoquinolin-1-one | |
| I-66 | 6-Dimethylamino-2-{2-hydroxymethyl-3-[5-(4-hydroxy-3,4,5,6-tetrahydro-2*H*-[1,3']bipyridinyl-6'-ylamino)-1-methyl-6-oxo-1,6-dihydro-pyridin-3-yl]-phenyl}-3,4-dihydro-2*H*-isoquinolin-1-one | |
| I-67 | 6-Dimethylamino-2-(3-{5-[5-(1,1-dioxo-1-thiomorpholin-4-yl)-pyridin-2-ylamino]-1-methyl-6-oxo-1,6-dihydro-pyridin-3-yl}-2-hydroxymethyl-phenyl)-3,4-dihydro-2*H*-isoquinolin-1-one | |
| I-68 | 2-(3-{5-[5-(3,3-Difluoro-azetidin-1-yl)-pyridin-2-ylamino]-1-methyl-6-oxo-1,6-dihydro-pyridin-3-yl}-2-hydroxymethyl-phenyl)-6-dimethylamino-3,4-dihydro-2*H*-isoquinolin-1-one | |
| I-69 | 6-Dimethylamino-2-(3-{5-[5-(3-hydroxy-azetidin-1-yl)-pyridin-2-ylamino]-1-methyl-6-oxo-1,6-dihydro-pyridin-3-yl}-2-hydroxymethyl-phenyl)-3,4-dihydro-2*H*-isoquinolin-1-one | |
| I-70 | 2-{3-[5-(5-Diethylamino-pyridin-2-ylamino)-1-methyl-6-oxo-1,6-dihydro-pyridin-3-yl]-2-hydroxymethyl-phenyl}-6-dimethylamino-3,4-dihydro-2*H*-isoquinolin-1-one | |
| I-71 | 2-{2-Hydroxymethyl-3-[1-methyl-5-(5-morpholin-4-yl-pyridin-2-ylamino)-6-oxo-1,6-dihydro-pyridin-3-yl]-phenyl}-6-(1-methyl-cyclopropyl)-3,4-dihydro-2*H*-isoquinolin-1-one | |
| I-72 | 2-{3-[5-(4,4-Difluoro-3,4,5,6-tetrahydro-2*H*-[1,3']bipyridinyl-6'-ylamino)-1-methyl-6-oxo-1,6-dihydro-pyridin-3-yl]-2-hydroxymethyl-phenyl}-6-di-methylamino-3,4-dihydro-2H isoquinolin-1-one | |
| I-73 | 6-Dimethylamino-2-{2-hydroxymethyl-3-[5-(1-methanesulfonylmethyl-1*H*-pyrazol-3-ylamino)-1-methyl-6-oxo-1,6-dihydro-pyridin-3-yl]-phenyl}-3,4-dihydro-2*H*-isoquinolin-1-one | |
| I-74 | 6-Dimethylamino-2-{2-hydroxymethyl-3-[1-methyl-6-oxo-5-(2-propyl-2*H*-[1,2,3]triazol-4-ylamino)-1,6-dihydro-pyridin-3-yl]-phenyl}-3,4-dihydro-2*H*-isoquinolin-1-one | |
| I-75 | 6-Dimethylamino-2-(2-hydroxymethyl-3-{1-methyl-6-oxo-5-[5-(2-oxo-pyrrolidin-1-yl)-pyridin-2-ylamino]-1,6-dihydro-pyridin-3-yl}-phenyl)-3,4-dihydro-2*H*-isoquinolin-1-one | |
| I-76 | 6'-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-3,4,5,6-tetrahydro-[1,3']bipyridinyl-2-one | |
| I-77 | 6-Dimethylamino-2-(3-{5-[5-(1,1-dioxo-1$\lambda^6$-[1,2]thiazinan-2-yl)-pyridin-2-ylamino]-1-methyl-6-oxo-1,6-dihydro-pyridin-3-yl}-2-hydroxymethyl-phenyl)-3,4-dihydro-2*H*-isoquinolin-1-one | |
| I-78 | 6-Dimethylamino-2-(3-{5-[5-(1,1-dioxo-1$\lambda^6$-isothiazolidin-2-yl)-pyridin-2-ylamino]-1-methyl-6-oxo-1,6-dihydro-pyridin-3-yl}-2-hydroxymethyl-phenyl)-3,4-dihydro-2*H*-isoquinolin-I-one | |
| I-79 | 2-(6-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-3-yloxy)-acetamide | |
| I-80 | 6-Cyclopropyl-2-(2-hydroxymethyl-3-{1-methyl-5-[5-(2-oxa-6-aza-spiro[3.3]hept-6-yl)-pyridin-2-ylamino]-6-oxo-1,6-dihydro-pyridin-3-yl}-phenyl)-3,4-dihydro-2*H*-isoquinolin-1-one | |
| I-81 | 1-(6-{5-[3-(6-Cyclopropyl-1-oxo-3,4-dihydro-1*H*-isoquinolin-2-yl)-2-hydroxymethyl-phenyl]-1-methyl-2-oxo-1,2-dihydro-pyridin-3-ylamino}-pyridine-3-carbonyl)-piperidine-4-carbonitrile | |
| I-82 | 6-Cyclopropyl-2-(3-{5-[5-(3-ethoxy-pyrrolidine-1-carbonyl)-pyridin-2-yl-amino]-1-methyl-6-oxo-1,6-dihydro-pyridin-3-yl} -2-hydroxymethyl-phenyl)-3,4-dihydro-2*H*-isoquinolin-1-one | |
| I-83 | 6-Dimethylamino-2-(2-hydroxymethyl-3-{5-[5-(3-methanesulfonyl-prop-oxy)-pyridin-2-ylamino]-1-methyl-6-oxo-1,6-dihydro-pyridin-3-yl}-phenyl)-3,4-dihydro-2*H*-isoquinolin-1-one | |
| I-84 | 6-Dimethylamino-2-(3-{5-[5-(2-hydroxy-ethoxy)-pyridin-2-ylamino]-1-methyl-6-oxo-1,6-dihydro-pyridin-3-yl}-2-hydroxymethyl-phenyl)-3,4-di-hydro-2*H*-isoquinolin-1-one | |

(continued)

| | Nomenclature | $(M+H)^+$ |
|---|---|---|
| I-85 | 2-(3-{5-[5-(4-Acetyl-piperazin-1-yl)-pyridin-2-ylamino]-1-methyl-6-oxo-1,6-dihydro-pyridin-3-yl}-2-hydroxymethyl-phenyl)-6-dimethylamino-3,4-dihydro-2H-isoquinolin-1-one | |
| I-86 | 1-(6-{5-[3-(6-Cyclopropyl-1-oxo-3,4-dihydro-1*H*-isoquinolin-2-yl)-2-hydroxymethyl-phenyl]-1-methyl-2-oxo-1,2-dihydro-pyridin-3-ylamino}-pyridine-3-carbonyl)-piperidine-3-carbonitrile | |
| I-87 | 2-(2-acetaldehyde oxime -3-{1-methyl-5-[5-(morpholine-4-carbonyl)-pyri-din-2-ylamino]-6-oxo-1,6-dihydro-pyridin-3-yl}-phenyl)-6-dimethylamino-3,4-dihydro-2*H*-isoquinolin-1-one | |
| I-88 | 2-[6-(1-Chloro-cyclopropyl)-1-oxo-3,4-dihydro-1*H*-isoquinolin-2-yl]-6-{1-methyl-5-[5-(morpholine-4-carbonyl)-pyridin-2-ylamino]-6-oxo-1,6-di-hydro-pyridin-3-yl}-benzaldehyde | |
| I-89 | 6-(1-Chloro-cyclopropyl)-2-(2-hydroxymethyl-3-{1-methyl-5-[5-(morpho-line-4-carbonyl)-pyridin-2-ylamino]-6-oxo-1,6-dihydro-pyridin-3-yl}-phenyl)-3,4-dihydro-2*H*-isoquinolin-1-one | |
| I-90 | 6-Dimethylamino-2-{2-hydroxymethyl-3-[5-(4-hydroxy-4-methyl-3,4,5,6-tetrahydro-2*H*-[1,3'] bipyridinyl-6'-ylamino)-1-methyl-6-oxo-1,6-dihydro-pyridin-3-yl]-phenyl}-3,4-dihydro-2*H*-isoquinolin-1-one | |
| I-91 | 2-[2-(2-Hydroxymethyl-3- {1-methyl-5-[5-(morpholine-4-carbonyl)-pyridin-2-ylamino]-6-oxo-1,6-dihydro-pyridin-3-yl}-phenyl)-1-oxo-1,2,3,4-tetra-hydro-isoquinolin-6-yl]-2-methyl-propionitrile | |
| I-92 | 6-(1-Chloro-cyclopropyl)-2-{2-hydroxymethyl-3-[1-methyl-5-(5-morpho-lin-4-yl-pyridin-2-ylamino)-6-oxo-1,6-dihydro-pyridin-3-yl]-phenyl}-3,4-dihydro-2*H*-isoquinolin-1-one | |
| I-93 | 6-Dimethylamino-2-(2-hydroxymethyl-3-{1-methyl-5-[5-(2-oxa-6-aza-spiro[3.3]hept-6-yl)-pyridin-2-ylamino]-6-oxo-1,6-dihydro-pyridin-3-yl}-phenyl)-3,4-dihydro-2*H*-isoquinolin-1-one | |
| I-94 | 1-(2-{2-Hydroxymethyl-3-[1-methyl-5-(5-morpholin-4-yl-pyridin-2-yl-amino)-6-oxo-1,6-dihydro-pyridin-3-yl]-phenyl]-1-oxo-1,2,3,4-tetrahydro-isoquinolin-6-yl)-cyclopropanecarbonitrile | |
| I-95 | 2-(2-{2-Hydroxymethyl-3-[1-methyl-5-(5-morpholin-4-yl-pyridin-2-yl-amino)-6-oxo-1,6-dihydro-pyridin-3-yl]-phenyl}-1-oxo-1,2,3,4-tetrahydro-isoquinolin-6-yl)-2-methyl-propionitrile | |
| I-96 | 6-Ditnethylamino-2-{2-hydroxymethyl-3-[1-methyl-6-oxo-5-(5-piperazin-1-yl-pyridin-2-ylamino)-1,6-dihydro-pyridin-3-yl]-phenyl}-3,4-dihydro-2*H*-isoquinolin-1-one | |
| I-97 | 6-Dimethylamino-2-{2-hydroxymethyl-3-[5-(5-hydroxymethyl-pyridin-2-ylamino)-1-methyl-6-oxo-1,6-dihydro-pyridin-3-yl]-phenyl}-3,4-dihydro-2*H*-isoquinolin-1-one | |
| I-98 | 6-Dimethylamino-2-{2-hydroxymethyl-3-[1-methyl-5-(6-morpholin-4-yl-pyrimidin-4-ylamino)-6-oxo-1,6-dihydro-pyridin-3-yl]-phenyl}-3,4-di-hydro-2*H*-isoquinolin-1-one | |
| I-99 | 6-Dimethylamino-2-{2-hydroxymethyl-3-[1-methyl-5-(5-methyl-isoxazol-3-ylamino)-6-oxo-1,6-dihydro-pyridin-3-yl]-phenyl}-3,4-dihydro-2*H*-iso-quinolin-1-one | |
| I-100 | 6-Dimethylamino-2-{2-hydroxymethyl-3-[5-(isoxazol-3-ylamino)-1-methyl-6-oxo-1,6-dihydro-pyridin-3-yl]-phenyl}-3,4-dihydro-2*H*-iso-quinolin-1-one | |
| I-101 | 6-(1-Chloro-cyclopropyl)-2-[3-(5-{5-[4-(2-hydroxy-ethyl)-piperazin-1-yl]-pyridin-2-ylamino}-1-methyl-6-oxo-1,6-dihydro-pyridin-3-yl)-2-hydroxy-methyl-phenyl]-3,4-dihydro-2*H*-isoquinolin-1-one | |
| I-102 | 1-[2-(2-Hydroxymethyl-3-{1-methyl-5-[5-(morpholine-4-carbonyl)-pyridin-2-ylamino]-6-oxo-1,6-dihydro-pyridin-3-yl}-phenyl)-1-oxo-1,2,3,4-tetra-hydro-isoquinolin-6-yl]-cyclopropanecarbonitrile | |
| I-103 | 2-(6-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-3-yloxy)-*N,N*-dimethyl-acetamide | |
| I-104 | 6-(1-Hydroxy-1-methyl-ethyl)-2-{2-hydroxymethyl-3-[1-methyl-5-(5-mor-pholin-4-yl-pyridin-2-ylamino)-6-oxo-1,6-dihydro-pyridin-3-yl]-phenyl}-3,4-dihydro-2*H*-isoquinolin-1-one | |

(continued)

| | Nomenclature | (M+H)+ |
|---|---|---|
| I-105 | 6-Dimethylamino-2-(2-hydroxymethyl-3-{5-[5-(2-hydroxy-propoxy-methyl)-pyridin-2-ylamino]-1-methyl-6-oxo-1,6-dihydro-pyridin-3-yl}-phenyl)-3,4-dihydro-2*H*-isoquinolin-1-one | |
| I-106 | (2-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-4-yl)-carbamic acid benzyl ester | |
| I-107 | 2-{3-[5-(4-Amino-pyridin-2-ylamino)-1-methyl-6-oxo-1,6-dihydro-pyridin-3-yl]-2-hydroxymethyl-phenyl}-6-dimethylamino-3,4-dihydro-2*H*-iso-quinolin-1-one | |
| I-108 | N-(2-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-4-yl)-acetamide | |
| I-109 | 6-Dimethylamino-2-(2-hydroxymethyl-3-{1-methyl-5-[6-(4-methyl-piper-azin-1-yl)-pyrimidin-4-ylamino]-6-oxo-1,6-dihydro-pyridin-3-yl} -phenyl)-3,4-dihydro-2*H*-isoquinolin-1-one | |
| I-110 | 6-[(2-Hydroxy-ethyl)-methyl-amino]-2-{2-hydroxymethyl-3-[1-methyl-5-(5-morpholin-4-yl-pyridin-2-ylamino)-6-oxo-1,6-dihydro-pyridin-3-yl]-phenyl}-3,4-dihydro-2*H*-isoquinolin-1-one | |
| I-111 | 2-(3-{1-Difluoromethyl-5-[5-(morpholine-4-carbonyl)-pyridin-2-ylamino]-6-oxo-1,6-dihydro-pyrid in-3-yl}-2-hydroxymethyl-phenyl)-6-dimethyl-amino-3,4-dihydro-2*H*-isoquinolin-1-one | |
| I-112 | 6-Dimethylamino-2-(2-hydroxymethyl-3-{5-[5-(2-methoxy-ethoxy)-pyri-din-2-ylamino]-1-methyl-6-oxo-1,6-dihydro-pyridin-3-yl}-phenyl)-3,4-di-hydro-2*H*-isoquinolin-1-one | |
| I-113 | 2-(3-{5-[5-(3,3-Difluoro-azetidin-1-ylmethyl)-pyridin-2-ylamino]-1-methyl-6-oxo-1,6-dihydro-pyridin-3-yl}-2-hydroxymethyl-phenyl)-6-di-methylamino-3,4-dihydro-2*H*-isoquinolin-1-one | |
| I-114 | 6-Dimethylamino-2-(3-{5-[5-(1,1-dioxo-1$\lambda^6$-thiomorpholin-4-ylmethyl)-pyridin-2-ylamino]-1-methyl-6-oxo-1,6-dihydro-pyrid in-3-yl}-2-hydroxy-methyl-phenyl)-3,4-dihydro-2*H*-isoquinolin-1-one | |
| I-115 | 1-(5-{3-[6-(1-Chloro-cyclopropyl)-1-oxo-3,4-dihydro-1*H*-isoquinolin-2-yl]-2-hydroxymethyl-phenyl}-1-methyl-2-oxo-1,2-dihydro-pyridin-3-yl)-3-ethyl-urea | |
| I-116 | 6-Dimethylamino-2-{2-hydroxymethyl-3-[1-methyl-6-oxo-5-(5-trifluoro-methyl-isoxazol-3-ylamino)-1,6-dihydro-pyridin-3-yl]-phenyl}-3,4-di-hydro-2*H*-isoquinolin-1-one | |
| I-117 | 1-{5-[3-(6-*tert*-Butyl-1-oxo-3,4-dihydro-1*H*-isoquinolin-2-yl)-2-hydroxy-methyl-phenyl]-1-methyl-2-oxo-1,2-dihydro-pyridin-3-yl} -3-methyl-urea | |
| I-118 | 6-Dimethylamino-2-(2-hydroxymethyl-3- {5-[5-(3-hydroxy-3-methyl-azetidin-1-yl)-pyridin-2-ylamino]-1-methyl-6-oxo-1,6-dihydro-pyridin-3-yl}-phenyl)-3,4-dihydro-2*H*-isoquinolin-1-one | |
| I-119 | 6-(1-Chloro-cyclopropyl)-2-{2-hydroxymethyl-3-[5-(4-hydroxy-4-methyl-3,4,5,6-tetrahydro-2*H*-[1,3']bipyridinyl-6'-ylamino)-1-methyl-6-oxo-1,6-dihydro-pyridin-3-yl]-phenyl}-3,4-dihydro-2*H*-isoqunolin-1-one | |
| I-120 | 2-(3-{5-[5-(4-Acetyl-piperazin-1-yl)-pyridin-2-ylamino]-1-methyl-6-oxo-1,6-dihydro-pyridin-3-yl}-2-hydroxymethyl-phenyl)-6-(1-chloro-cyclo-propyl)-3,4-dihydro-2*H*-isoquinolin-1-one | |
| I-121 | 6-(1-Chloro-cyclopropyl)-2-{2-hydroxymethyl-3-[1-methyl-6-oxo-5-(5-piperazin-1-yl-pyridin-2-ylamino)-1,6-dihydro-pyridin-3-yl]-phenyl}-3,4-dihydro-2*H*-isoquinolin-1-one | |
| I-122 | 1-{5-[3-(6-*tert*-Butyl-1-oxo-3,4-dihydro-1*H*-isoquinolin-2-yl)-2-hydroxy-methyl-phenyl]-1-methyl-2-oxo-1,2-dihydro-pyridin-3-yl}-3-ethyl-urea | |
| I-123 | 6-[Bis-(2-hydroxy-ethyl)-amino]-2-{2-hydroxymethyl-3-[1-methyl-5-(5-morpholin-4-yl-pyridin-2-ylamino)-6-oxo-1,6-dihydro-pyridin-3-yl]-phenyl}-3,4-dihydro-2*H*-isoquinolin-1-one | |
| I-124 | 2-(6-Dimethylamino-1-oxo-3,4-dihydro-1*H*-isoquinolin-2-yl)-6-{1-methyl-5-[5-(morpholine-4-carbonyl)-pyridin-2-ylamino]-6-oxo-1,6-dihydro-pyridin-3-yl}-benzoic acid | |
| I-125 | 6-Dimethylamino-2-{5-fluoro-2-hydroxymethyl-3-[1-methyl-5-(5-morpho-lin-4-yl-pyridin-2-ylamino)-6-oxo-1,6-dihydro-pyridin-3-yl]-phenyl}-3,4-dihydro-2*H*-isoquinolin-1-one | |

(continued)

| | Nomenclature | (M+H)+ |
|---|---|---|
| I-126 | 1-(6-{5-[3-(6-Dimethylamino-1-oxo-3,4-dihydro-1*H*-isoduinolin-2-yl)-2-hydroxymethyl-phenyl]-1-methyl-2-oxo-1,2-dihydro-pyridin-3-ylamino}-pyridin-3-yl)-pyrrolidine-3-carboxylic acid methyl ester | |
| I-127 | 1-(6-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-ylamino}-pyridin-3-yl)-pyrrolidine-3-carboxylic acid | |
| I-128 | 6-Dimethylamino-2-(2-hydroxymethyl-3-{5-[6-(2-methoxy-1-methyl-ethyl-amino)-pyridin-2-ylamino]-1-methyl-6-oxo-1,6-dihydro-pyridin-3-yl}-phenyl)-3,4-dihydro-2*H*-isoquinolin-1-one | |
| I-129 | 6-Dimethylamino-2-{2-hydroxymethyl-3-[5-(1*H*-imidazol-2-ylamino)-1-methyl-6-oxo-1,6-dihydro-pyridin-3-yl]-phenyl}-3,4-dihydro-2*H*-iso-quinolin-1-one | |
| I-130 | N-{5-[3-(6-Dimethylamino-1-oxo-3,4-dihydro-1H-isoquinolin-2-yl)-2-hydroxymethyl-phenyl]-1-methyl-2-oxo-1,2-dihydro-pyridin-3-yl}-N'-carb-oxylic acid butyl ester-guanidine | |
| I-131 | *N*-{5-[3-(6-Dimethylamino-1-oxo-3,4-dihydro-1*H*-isoquinolin-2-yl)-2-hydroxymethyl-phenyl]-1-methyl-2-oxo-1,2-dihydro-pyridin-3-yl}-guanidine | |
| I-132 | 2-(2-Aminomethyl-3-{1-methyl-5-[5-(morpholine-4-carbonyl)-pyridin-2-ylamino]-6-oxo-1,6-dihydro-pyridin-3-yl}-phenyl)-6-dimethylamino-3,4-dihydro-2*H*-isoquinolin-1-one | |
| I-133 | 6-Dimethylamino-2-(2-dimethylaminomethyl-3-{1-methyl-5-[5-(morpho-line-4-carbonyl)-pyridin-2-ylamino]-6-oxo-1,6-dihydro-pyridin-3-yl}-phenyl)-3,4-dihydro-2*H*-isoquinolin-1-one | |
| I-134 | 1-(5-{2-Hydroxymethyl-3-[6-(1-hydroxy-1-methyl-ethyl)-1-oxo-3,4-di-hydro-1*H*-isoquinolin-2-yl]-phenyl}-1-methyl-2-oxo-1,2-dihydro-pyridin-3-yl)-3-methyl-urea | |
| I-135 | 1-(5-{3-[6-(2-Cyano-1,1-dimethyl-ethyl)-1-oxo-3,4-dihydro-1*H*-isoquino-lin-2-yl]-2-hydroxymethyl-phenyl}-1-methyl-2-oxo-1,2-dihydro-pyridin-3-yl)-3-methyl-urea | |
| I-136 | 2-[2-Hydroxymethyl-3-(5-{5-[4-(2-methoxy-ethyl)-piperazin-1-yl]-pyridin-2-ylamino}-1-methyl-6-oxo-1,6-dihydro-pyridin-3-yl)-phenyl]-6-(1-methyl-cyclopropyl)-3,4-dihydro-2*H*-isoquinolin-1-one | |
| I-137 | 6-Dimethylamino-2-(2-hydroxymethyl-3-{5-[6-((R)-3-hydroxy-pyrrolidin-1-yl)-pyridin-2-ylamino]-1-methyl-6-oxo-1,6-dihydro-pyridin-3-yl}-phenyl)-3,4-dihydro-2*H*-isoquinolin-1-one | |
| I-138 | 6-Dimethylamino-2-{3-[5-(5-ethyl-isoxazol-3-ylamino)-1-methyl-6-oxo-1,6-dihydro-pyridin-3-yl]-2-hydroxymethyl-phenyl}-3,4-dihydro-2*H*-iso-quinolin-1-one | |
| I-139 | 6-Cyclopropyl-2-{2-hydroxymethyl-3-[1-methyl-5-(5-morpholin-4-yl-pyridin-2-ylamino)-6-oxo-1,6-dihydro-pyridin-3-yl]-phenyl}-3,4-dihydro-2*H*-isoquinolin-1-one | |
| I-140 | 6-Azetidin-1-yl-2-(2-hydroxymethyl-3-{1-methyl-5-[5-(morpholine-4-carb-onyl)-pyridin-2-ylamino]-6-oxo-1,6-dihydro-pyridin-3-yl}-phenyl)-3,4-di-hydro-2*H*-isoquinolin-I-one | |
| I-141 | 1-{5-[3-(6-Dimethytamino-1-oxo-3,4-dihydro-1*H*-isoquinolin-2-yl)-4-fluoro-2-hydroxymethyl-phenyl]-1-methyl-2-oxo-1,2-dihydro-pyridin-3-yl}-3-methyl-urea | |
| I-142 | 6-tert-Butyl-2-{2-hydroxymethyl-3-[1-methyl-6-oxo-5-(5-piperazin-1-yl-pyridin-2-ylamino)-1,6-dihydro-pyridin-3-yl]-phenyl}-3,4-dihydro-2*H*-iso-quinolin-1-one | |
| I-143 | 2-Cyano-*N*-{5-[3-(6-dimethylamino-1-oxo-3,4-dihydro-1*H*-isoquinolin-2-yl)-2-hydroxymethyl-phenyl]-1-methyl-2-oxo-1,2-dihydro-pyridin-3-yl}-acetamide | |
| I-144 | 6-[2-(2-Hydroxy-ethoxy)-1,1-dimethyl-ethyl]-2-{2-hydroxymethyl-3-[1-methyl-5-(5-morpholin-4-yl-pyridin-2-ylamino)-6-oxo-1,6-dihydro-pyridin-3-yl]-phenyl}-3,4-dihydro-2*H*-isoquinolin-1-one | |
| I-145 | 6-(1-Chloro-cyclopropyl)-2-(2-hydroxymethyl-3-{1-methyl-5-[5-(4-methyl-piperazin-1-yl)-pyridin-2-ylamino]-6-oxo-1,6-dihydro-pyridin-3-yl}-phenyl)-3,4-dihydro-2*H*-isoquinolin-1-one | |
| I-146 | 6-(1-Hydroxy-1-methyl-ethyl)-2-(2-hydroxymethyl-3-{1-methyl-5-[5-(4-methyl-piperazin-1-yl)-pyridin-2-ylamino]-6-oxo-1,6-dihydro-pyridin-3-yl}-phenyl)-3,4-dihydro-2*H*-isoquinolin-1-one | |

(continued)

| | Nomenclature | $(M+H)^+$ |
|---|---|---|
| I-147 | 6-(2-Hydroxy-1,1-dimethyl-ethyl)-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2*H*-isoquinolin-1-one | |
| I-148 | 2-[2-(2-Hydroxymethyl-3-{1-methyl-5-[5-(4-methyl-piperazin-1-yl)-pyri-din-2-ylamino]-6-oxo-1,6-dihydro-pyridin-3-yl}-phenyl)-1-oxo-1,2,3,4-tetrahydro-isoquinolin-6-yl]-2-methyl-propionitrile | |
| I-149 | *N*-{5-[3-(6-Dimethylammo-1-oxo-3,4-dihydro-1*H*-isoquinolin-2-yl)-2-hydroxymethyl-phenyl]-1-methyl-2-oxo-1,2-dihydro-pyridin-3-yl}-3-oxo-butyramide | |
| I-150 | 6-Dimethylamino-2-[2-hydroxymethyl-3-(5-{5-[(2-methoxy-ethylamino)-methyl]-pyridin-2-ylamino}-1-methyl-6-oxo-1,6-dihydro-pyridin-3-yl)-phenyl]-3,4-dihydro-2*H*-isoquinolin-1-one | |
| I-151 | 6-Dimethylamino-2-{3-[5-(5-ethyl-4-oxo-4,5-dihydro-1*H*-imidazol-2-yl-amino)-1-methyl-6-oxo-1,6-dihydro-pyridin-3-yl]-2-hydroxymethyl-phenyl}-3,4-dihydro-2*H*-isoquinolin-1-one | |
| I-152 | 1-{5-[3-(6-*tert*-Butyl-1-oxo-3,4-dihydro-1*H*-isoquiolin-2-yl)-2-hydroxy-methyl-phenyl]-1-methyl-2-oxo-1,2-dihydro-pyridin-3-yl}-3-(3-dimethyl-amino-propyl)-urea | |
| I-153 | 6-Dimethylamino-2-{3-[5-(5-ethyl-1*H*-imidazol-2-ylamino)-1-methyl-6-oxo-1,6-dihydro-pyridin-3-yl]-2-hydroxymethyl-phenyl}-3,4-dihydro-2*H*-isoquinolin-1-one | |
| 1-154 | 6-Cyclopropyl-2-(2-hydroxymethyl-3-{1-methyl-5-[5-(morpholine-4-carb-onyl)-pyridin-2-ylamino]-6-oxo-1,6-dihydro-pyridazin-3-yl}-phenyl)-3,4-dihydro-2*H*-isoquinolin-1-one | |
| 1-155 | 6-Cyclopropyl-2-{2-hydroxymethyl-3-[1-methyl-5-(1-methyl-1*H*-pyrazol-3-ylamino)-6-oxo-1,6-dihydro-pyridazin-3-yl]-phenyl}-3,4-dihydro-2*H*-isoquinolin-1-one | |
| III-4 | 6-Dimethylamino-2-{2-hydroxymethyl-3-[1-methyl-5-(1-methyl-1*H*-pyrazol-3-ylamino)-6-oxo-1,6-dihydro-pyridin-3-yl]-phenyl}-2*H*-iso-quinolin-1-one | |
| III-5 | 1-{5-[3-(6-Dimethylamino-1-oxo-1*H*-isoquinolin-2-yl)-2-hydroxymethyl-phenyl]-1-methyl-2-oxo-1,2,-dihydro-pyridin-3-yl}-3-ethyl-urea | |
| III-6 | 2-(2-Hydroxymethyl-3-{1-methyl-5-[5-(morpholine-4-carbonyl)-pyridin-2-ylamino]-6-oxo-1,6-dihydro-pyridin-3-yl}-phenyl)-6-(1-methyl-cyclo-propyl)-2*H*-isoquinolin-1-one | |
| III-7 | 2-{2-Hydroxymethyl-3-[1-methyl-5-(5-morpholin-4-ylmethyl-pyridin-2-ylamino)-6-oxo-1,6-dihydro-pyridin-3-yl]-phenyl}-6-(1-methyl-cyclo-propyl)-2*H*-isoquinolin-1-one | |
| III-8 | 2-(2-Hydroxymethyl-3-{1-methyl-5-[5-(4-methyl-piperazine-1-carbonyl)-pyridin-2-ylamino]-6-oxo-1,6-dihydro-pyridin-3-yl}-phenyl)-6-(1-methyl-cyclopropyl)-2*H*-isoquinolin-1-one | |
| III-9 | 6-Cyclopropyl-2-{2-hydroxymethyl-3-[1-methyl-5-(5-morpholin-4-yl-methyl-pyridin-2-ylamino)-6-oxo-1,6-dihydro-pyridin-3-yl]-phenyl}-2*H*-isoquinolin-1-one | |
| III-10 | 6-Cyclopropyl-2-(2-hydroxymethyl-3-{1-methyl-5-[5-(4-methyl-piper-azine-1-carbonyl)-pyridin-2-ylamino]-6-oxo-1,6-dihydro-pyridin-3-yl}-phenyl)-2*H*-isoquinolin-1-one | |
| III-11 | 6-Cyclopropyl-2-(2-hydroxymethyl-3-{1-methyl-5-[5-(morpholine-4-carb-onyl)-pyridin-2-ylamino]-6-oxo-1,6-dihydro-pyridin-3-yl}-phenyl)-2*H*-isoquinolin-1-one | |
| III-12 | 6-Cyclopropyl-2-{2-hydroxymethyl-3-[1-methyl-5-(5-morpholin-4-yl-pyri-din-2-ylamino)-6-oxo-1,6-dihydro-pyridin-3-yl]-phenyl}-2*H*-isoquinolin-1-one | |
| III-13 | 6-Cyclopropyl-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3-methyl-2*H*-isoquinolin-1-one | |
| III-14 | 2-{2-Hydroxymethyl-3-[1-methyl-5-(5-morpholin-4-yl-pyridin-2-ylamino)-6-oxo-1,6-dihydro-pyridin-3-yl]-phenyl}-6-(1-methyl-cyclopropyl)-2*H*-isoquinolin-1-one | |
| III-15 | 6-Dimethylamino-2-{2-hydroxymethyl-3-[1-methyl-5-(5-morpholin-4-yl-pyridin-2-ylamino)-6-oxo-1,6-dihydro-pyridin-3-yl]-phenyl}-2*H*-iso-quinolin-1-one | |

(continued)

| | Nomenclature | $(M+H)^+$ |
|---|---|---|
| III-16 | 6-*tert*-Butyl-2-(2-hydroxymethyl-3-{1-methyl-5-[5-(morpholine-4-carbon-yl)-pyridin-2-ylamino]-6-oxo-1,6-dihydro-pyridin-3-yl}-phenyl)-2*H*-iso-quinolin-1-one | |
| III-17 | 6-Cyclopropyl-3-hydroxymethyl-2-(2-hydroxymethyl-3-{1-methyl-5-[5-(morpholine-4-carbonyl)-pyridin-2-ylamino]-6-oxo-1,6-dihydro-pyridin-3-yl}-phenyl)-2*H*-isoquinolin-1-one | |
| III-18 | 6-Cyclopropyl-3-hydroxymethyl-2-{2-hydroxymethyl-3-[1-methyl-5-(5-morpholin-4-yl-pyridin-2-ylamino)-6-oxo-1,6-dihydro-pyridin-3-yl]-phenyl}-2*H*-isoquinolin-1-one | |
| III-19 | 6-Cyclopropyl-3-dimethylaminomethyl-2-{2-hydroxymethyl-3-[1-methyl-5-(5-morpholin-4-yl-pyridin-2-ylamino)-6-oxo-1,6-dihydro-pyridin-3-yl]-phenyl}-2*H*-isoquinolin-1-one | |
| III-20 | 3-*tert*-Butoxymethyl-6-cyclopropyl-2-{3-[5-(6-fluoro-pyridin-2-ylamino)-1-methyl-6-oxo-1,6-dihydro-pyridin-3-yl]-2-hydroxymethyl-phenyl}-2*H*-isoquinolin-1-one | |
| III-21 | 6-Dimethylamino-2-{2-hydroxymethyl-3-[1-methyl-5-(6-methylamino-pyridin-2-ylamino)-6-oxo-1,6-dihydro-pyridin-3-yl]-phenyl}-2*H*-iso-quinolin-1-one | |
| III-22 | 6-Dimethylamino-2-(2-hydroxymethyl-3-{1-methyl-5-[6-(4-methyl-piper-azin-1-yl)-pyridin-2-ylamino]-6-oxo-1,6-dihydro-pyridin-3-yl}-phenyl)-2*H*-isoquinolin-1-one | |
| III-23 | 2-{3-[5-(6-Amino-pyridin-2-ylamino)-1-methyl-6-oxo-1,6-dihydro-pyridin-3-yl]-2-hydroxymethyl-phenyl}-6-dimethylamino-2*H*-isoquinolin-1-one | |
| III-24 | 2-(6-{5-[3-(6-Dimethylamino-1-oxo-1*H*-isoquinolin-2-yl)-2-hydroxy-methyl-phenyl]-1-methyl-2-oxo-1,2-dihydro-pyridin-3-ylamino}-pyridin-3-yloxy)-*N*-methyl-acetamide | |
| III-25 | 2-{3-[5-(5,6-Dimethoxy-pyridin-2-ylamino)-1-methyl-6-oxo-1,6-dihydro-pyridin-3-yl]-2-hydroxymethyl-phenyl}-6-dimethylamino-2*H*-isoquinolin-1-one | |
| III-26 | 6-Dimethylamino-2-(2-hydroxymethyl-3-{5-[5-methoxy-6-(2-methoxy-ethoxy)-pyridin-2-ylamino]-1-methyl-6-oxo-1,6-dihydro-pyridin-3'-yl}-phenyl)-2*H*-isoquinolin-1-one | |
| III-27 | 6-Dimethylamino-2-(2-hydroxymethyl-3-{5-[6-methoxy-5-(2-methoxy-eth-oxy)-pyridin-2-ylamino]-1-methyl-6-oxo-1,6-dihydro-pyridin-3-yl}-phenyl)-2*H*-isoquinolin-1-one | |
| III-28 | 2-(3-{5-[5,6-Bis-(2-methoxy-ethoxy)-pyridin-2-ylamino]-1-methyl-6-oxo-1,6-dihydro-pyridin-3-yl}-2-hydroxymethyl-phenyl)-6-dimethylamino-2*H*-isoquinolin-1-one | |
| III-29 | 6-Dimethylamino-2-{2-hydroxymethyl-3-[1-methyl-5-(2-morpholin-4-yl-pyrimidin-4-ylamino)-6-oxo-1,6-dihydro-pyridin-3-yl]-phenyl}-2*H*-iso-quinolin-1-one | |
| III-30 | 6-Dimethylamino-2-(2-hydroxymethyl-3-{1-methyl-5-[2-(4-methyl-piper-azin-1-yl)-pyrimidin-4-ylamino]-6-oxo-1,6-dihydro-pyridin-3-yl}-phenyl)-2*H*-isoquinolin-1-one | |
| III-31 | 2-[4-(6-{5-[3-(6-Dimethylamino-1-oxo-1*H*-isoquinolin-2-yl)-2-hydroxy-methyl-phenyl]-1-methyl-2-oxo-1,2-dihydro-pyridin-3-ylamino}-pyridin-3-yl)-piperazin-1-yl]-isobutyramide | |
| III-32 | 2-(3-{5-[6-(4-Acetyl-piperazin-1-yl)-pyridin-2-ylamino]-1-methyl-6-oxo-1,6-dihydro-pyridin-3-yl}-2-hydroxymethyl-phenyl)-6-dimethylamino-2*H*-isoquinolin-1-one | |
| III-33 | 6-Dimethylamino-2-{3-[5-(5-ethyl-1*H*-pyrazol-3-ylamino)-1-methyl-6-oxo-1,6-dihydro-pyridin-3-yl]-2-hydroxymethyl-phenyl}-2*H*-isoquinolin-1-one | |
| III-34 | 6-Dimethylamino-2-(3-{5-[5-(2-hydroxy-ethoxy)-6-(2-methoxy-ethoxy)-pyridin-2-ylamino]-1-methyl-6-oxo-1,6-dihydro-pyridin-3-yl}-2-hydroxy-methyl-phenyl)-2*H*-isoquinolin-1-one | |
| III-35 | 6-Cyclopropyl-2-(2-hydroxymethyl-3-{1-methyl-5-[5-(morpholine-4-carb-onyl)-pyridin-2-ylamino]-6-oxo-1,6-dihydro-pyridazin-3-yl}-phenyl)-2*H*-isoquinolin-1-one | |
| III-36 | 2-(2-Hydroxymethyl-3-{1-methyl-5-[5-(morpholine-4-carbonyl)-pyridin-2-ylamino]-6-oxo-1,6-dihydro-pyridazin-3-yl}-phenyl)-6-(1-methyl-cyclo-propyl)-2*H*-isoquinolin-1-one | |

The pyridinone and pyridazinone derivatives described herein are kinase inhibitors, in particular Btk inhibitors. These inhibitors can be useful for treating one or more diseases responsive to kinase inhibition, including diseases

responsive to Btk inhibition and/or inhibition of B-cell proliferation, in mammals. Without wishing to be bound to any particular theory, it is believed that the interaction of the compounds of the invention with Btk results in the inhibition of Btk activity and thus in the pharmaceutical utility of these compounds. Accordingly, the invention includes a method of treating a mammal, for instance a human, having a disease responsive to inhibition of Btk activity, and/or inhibiting B-cell proliferation, comprising administrating to the mammal having such a disease, an effective amount of at least one chemical entity provided herein. An effective concentration may be ascertained experimentally, e.g. by assaying blood concentration of the compound, or theoretically, by calculating bioavailability. Other kinases that may be affected in addition to Btk include, but are not limited to, other tyrosine kinases and serine/- threonine kinases.

Kinases play notable roles in signaling pathways controlling fundamental cellular processes such as proliferation, differentiation, and death (apoptosis). Abnormal kinase activity has been implicated in a wide range of diseases, including multiple cancers, autoimmune and/or inflammatory diseases, and acute inflammatory reactions. The multifaceted role of kinases in key cell signaling pathways provides a significant opportunity to identify novel drugs targeting kinases and signaling pathways.

An embodiment includes a method of treating a patient having an autoimmune and/or inflammatory disease, or an acute inflammatory reaction responsive to inhibition of Btk activity and/or B-cell proliferation.

Autoimmune and/or inflammatory diseases that can be affected using compounds and compositions according to the invention include, but are not limited to: psoriasis, allergy, Crohn's disease, irritable bowel syndrome, Sjogren's disease, tissue graft rejection, and hyperacute rejection of transplanted organs, asthma, systemic lupus erythematosus (and associated glomerulonephritis), dermatomyositis, multiple sclerosis, scleroderma, vasculitis (ANCA-associated and other vasculitides), autoimmune hemolytic and thrombocytopenic states, Goodpasture's syndrome (and associated glomerulonephritis and pulmonary hemorrhage), atherosclerosis, rheumatoid arthritis, chronic Idiopathic thrombocytopenic purpura (ITP), Addison's disease, Parkinson's disease, Alzheimer's disease, diabetes, septic shock, and myasthenia gravis.

Included herein are methods of treatment in which at least one chemical entity provided herein is administered in combination with an anti-inflammatory agent. Anti-inflammatory agents include but are not limited to NSAIDs, non-specific and COX-2 specific cyclooxgenase enzyme inhibitors, gold compounds, corticosteroids, methotrexate, tumor necrosis factor receptor (TNF) receptors antagonists, immunosuppressants and methotrexate.

Examples of NSAIDs include, but are not limited to, ibuprofen, flurbiprofen, naproxen and naproxen sodium, diclofenac, combinations of diclofenac sodium and misoprostol, sulindac, oxaprozin, diflunisal, piroxicam, indomethacin, etodolac, fenoprofen calcium, ketoprofen, sodium nabumetone, sulfasalazine, tolmetin sodium, and hydroxychloroquine. Examples of NSAIDs also include COX-2 specific inhibitors such as celecoxib, valdecoxib, lumiracoxib and/or etoricoxib.

In some embodiments, the anti-inflammatory agent is a salicylate. Salicylates include by are not limited to acetylsalicylic acid or aspirin, sodium salicylate, and choline and magnesium salicylates.

The anti-inflammatory agent may also be a corticosteroid. For example, the corticosteroid may be cortisone, dexamethasone, methylprednisolone, prednisolone, prednisolone sodium phosphate, or prednisone.

In additional embodiments the anti-inflammatory agent is a gold compound such as gold sodium thiomalate or auranofin.

The invention also includes embodiments in which the anti-inflammatory agent is a metabolic inhibitor such as a dihydrofolate reductase inhibitor, such as methotrexate or a dihydroorotate dehydrogenase inhibitor, such as leflunomide.

Other embodiments of the invention pertain to combinations in which at least one anti-inflammatory compound is an anti-C5 monoclonal antibody (such as eculizumab or pexelizumab), a TNF antagonist, such as entanercept, or infliximab, which is an anti-TNF alpha monoclonal antibody.

Still other embodiments of the invention pertain to combinations in which at least one active agent is an immunosuppressant compound such as an immunosuppressant compound chosen from methotrexate, leflunomide, cyclosporine, tacrolimus, azathioprine, and mycophenolate mofetil.

B-cells and B-cell precursors expressing BTK have been implicated in the pathology of B-cell malignancies, including, but not limited to, B-cell lymphoma, lymphoma (including Hodgkin's and non-Hodgkin's lymphoma), hairy cell lymphoma, multiple myeloma, chronic and acute myelogenous leukemia and chronic and acute lymphocytic leukemia.

BTK has been shown to be an inhibitor of the Fas/APO-1 (CD-95) death inducing signaling complex (DISC) in B-lineage lymphoid cells. The fate of leukemia/lymphoma cells may reside in the balance between the opposing proapoptotic effects of caspases activated by DISC and an upstream anti-apoptotic regulatory mechanism involving BTK and/or its substrates (Vassilev et al., J. Biol. Chem. 1998, 274, 1646-1656).

It has also been discovered that BTK inhibitors are useful as chemosensitizing agents, and, thus, are useful in combination with other chemotherapeutic drugs, in particular, drugs that induce apoptosis. Examples of other chemotherapeutic drugs that can be used in combination with chemosensitizing BTK inhibitors include topoisomerase I inhibitors (camptothecin or topotecan), topoisomerase II inhibitors (e.g. daunomycin and etoposide), alkylating agents (e.g. cyclophosphamide, melphalan and BCNU), tubulin directed agents (e.g. taxol and vinblastine), and biological agents (e.g.

antibodies such as anti CD20 antibody, IDEC 8, immunotoxins, and cytokines).

Btk activity has also be associated with some leukemias expressing the *bcr-abl* fusion gene resulting from translocation of parts of chromosome 9 and 22. This abnormality is commonly observed in chronic myelogenous leukemia. Btk is constitutively phosphorylated by the *bcr-abl* kinase which initiates downstream survival signals which circumvents apoptosis in *bcr-abl* cells. (Feldhahn et al. J. Exp. Med. 2005 201(11):1837-1852)

The compounds of the present invention may be formulated in a wide variety of oral administration dosage forms and carriers. Oral administration can be in the form of tablets, coated tablets, dragées, hard and soft gelatine capsules, solutions, emulsions, syrups, or suspensions. Compounds of the present invention are efficacious when administered by other routes of administration including continuous (intravenous drip) topical parenteral, intramuscular, intravenous, subcutaneous, transdermal (which may include a penetration enhancement agent), buccal, nasal, inhalation and suppository administration, among other routes of administration. The preferred manner of administration is generally oral using a convenient daily dosing regimen which can be adjusted according to the degree of affliction and the patient's response to the active ingredient.

A compound or compounds of the present invention, as well as their pharmaceutically useable salts, together with one or more conventional excipients, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. A typical preparation will contain from about 5% to about 95% active compound or compounds (w/w). The term "preparation" or "dosage form" is intended to include both solid and liquid formulations of the active compound and one skilled in the art will appreciate that an active ingredient can exist in different preparations depending on the target organ or tissue and on the desired dose and pharmacokinetic parameters.

The term "excipient" as used herein refers to a compound that is useful in preparing a pharmaceutical composition, generally safe, non-toxic and neither biologically nor otherwise undesirable, and includes excipients that are acceptable for veterinary use as well as human pharmaceutical use. The compounds of this invention can be administered alone but will generally be administered in admixture with one or more suitable pharmaceutical excipients, diluents or carriers selected with regard to the intended route of administration and standard pharmaceutical practice.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

A "pharmaceutically acceptable salt" form of an active ingredient may also initially confer a desirable pharmacokinetic property on the active ingredient which were absent in the non-salt form, and may even positively affect the pharmacodynamics of the active ingredient with respect to its therapeutic activity in the body. The phrase "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like.

Solid form preparations include powders, tablets, pills, capsules, cachets, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Solid form preparations may contain, in addition to the active component, colorants, flavors, stabilizers, buffers, artificial and natural sweeteners, dispersants, thickeners,

solubilizing agents, and the like.

Liquid formulations also are suitable for oral administration include liquid formulation including emulsions, syrups, elixirs, aqueous solutions, aqueous suspensions. These include solid form preparations which are intended to be converted to liquid form preparations shortly before use. Emulsions may be prepared in solutions, e.g., in aqueous propylene glycol solutions or may contain emulsifying agents such as lecithin, sorbitan monooleate, or acacia. Aqueous solutions can be prepared by dissolving the active component in water and adding suitable colorants, flavors, stabilizing, and thickening agents. Aqueous suspensions can be prepared by dispersing the finely divided active component in water with viscous material, such as natural or synthetic gums, resins, methylcellulose, sodium carboxymethylcellulose, and other well known suspending agents.

The compounds of the present invention may be formulated for parenteral administration (e.g., by injection, e.g. bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, e.g. solutions in aqueous polyethylene glycol. Examples of oily or nonaqueous carriers, diluents, solvents or vehicles include propylene glycol, polyethylene glycol, vegetable oils (*e.g.,* olive oil), and injectable organic esters (*e.g.,* ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilisation from solution for constitution before use with a suitable vehicle, *e.g.*, sterile, pyrogen-free water.

The compounds of the present invention may be formulated for topical administration to the epidermis as ointments, creams or lotions, or as a transdermal patch. Ointments and creams may, e.g., be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

The compounds of the present invention may be formulated for administration as suppositories. A low melting wax, such as a mixture of fatty acid glycerides or cocoa butter is first melted and the active component is dispersed homogeneously, e.g., by stirring. The molten homogeneous mixture is then poured into convenient sized molds, allowed to cool, and to solidify.

The compounds of the present invention may be formulated for vaginal administration. Pessaries, tampons, creams, gels, pastes, foams or sprays containing in addition to the active ingredient such carriers as are known in the art to be appropriate.

The compounds of the present invention may be formulated for nasal administration. The solutions or suspensions are applied directly to the nasal cavity by conventional means, e.g., with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved e.g. by means of a metering atomizing spray pump.

The compounds of the present invention may be formulated for aerosol administration, particularly to the respiratory tract and including intranasal administration. The compound will generally have a small particle size e.g. of the order of five (5) microns or less. Such a particle size may be obtained by means known in the art, e.g. by micronization. The active ingredient is provided in a pressurized pack with a suitable propellant such as a chlorofluorocarbon (CFC), e.g., dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, e.g. a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form e.g. in capsules or cartridges of e.g., gelatin or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to an skin-adhesive solid support. The compound of interest can also be combined with a penetration enhancer, e.g., Azone (1-dodecylaza-cyclo-heptan-2-one). Sustained release delivery systems are inserted subcutaneously into to the subdermal layer by surgery or injection. The subdermal implants encapsulate the compound in a lipid soluble membrane, e.g., silicone rubber, or a biodegradable polymer, e.g., polyactic acid.

Suitable formulations along with pharmaceutical carriers, diluents and expcipients are described in Remington:

The Science and Practice of Pharmacy 1995, edited by Martin, Mack Publishing Company, 19th edition, Easton, Pennsylvania. A skilled formulation scientist may modify the formulations within the teachings of the specification to provide numerous formulations for a particular route of administration without rendering the compositions of the present invention unstable or compromising their therapeutic activity.

The modification of the present compounds to render them more soluble in water or other vehicle, e.g., may be easily accomplished by minor modifications (salt formulation, esterification, etc.), which are well within the ordinary skill in the art. It is also well within the ordinary skill of the art to modify the route of administration and dosage regimen of a particular compound in order to manage the pharmacokinetics of the present compounds for maximum beneficial effect in patients.

The term "therapeutically effective amount" as used herein means an amount required to reduce symptoms of the disease in an individual. The dose will be adjusted to the individual requirements in each particular case. That dosage can vary within wide limits depending upon numerous factors such as the severity of the disease to be treated, the age and general health condition of the patient, other medicaments with which the patient is being treated, the route and form of administration and the preferences and experience of the medical practitioner involved. For oral administration, a daily dosage of between about 0.01 and about 1000 mg/kg body weight per day should be appropriate in monotherapy and/or in combination therapy. A preferred daily dosage is between about 0.1 and about 500 mg/kg body weight, more preferred 0.1 and about 100 mg/kg body weight and most preferred 1.0 and about 10 mg/kg body weight per day. Thus, for administration to a 70 kg person, the dosage range would be about 7 mg to 0.7 g per day. The daily dosage can be administered as a single dosage or in divided dosages, typically between 1 and 5 dosages per day. Generally, treatment is initiated with smaller dosages which are less than the optimum dose of the compound. Thereafter, the dosage is increased by small increments until the optimum effect for the individual patient is reached. One of ordinary skill in treating diseases described herein will be able, without undue experimentation and in reliance on personal knowledge, experience and the disclosures of this application, to ascertain a therapeutically effective amount of the compounds of the present invention for a given disease and patient.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

EXAMPLES

**Example 1:** 2-(3-Bromo-phenyl)-3-(3-t-butyl-phenylamino)-acrylic acid ethyl ester

(3-Bromo-phenyl)-acetic acid benzyl ester (1 g, 4.12 mmol) was dissolved in ethyl formate (8 mL, 99 mmol). Sodium hydride (60%, 660 mg, 16.5 mmol) was added. After stirring overnight, this was quenched with 2 M aq. HCl. This was partitioned between ethyl acetate and water. The ethyl acetate layer was washed with water, washed with brine, dried over anhydrous magnesium sulfate, and concentrated *in vacuo.*

This material (0.65 g) and 3-t-butyl-aniline (0.37 mL, 2.48 mmol) were stirred in 1 mL ethanol for 18 hours. This was concentrated *in vacuo* and purified by flash chromatography (gradient elution 5 to 20% ethyl acetate/hexanes) to yield 2-(3-Bromo-phenyl)-3-(3-t-butyl-phenylamino)-acrylic acid ethyl ester (0.5 mg). MS (ESI) 402 $(M+H)^+$.

**Example 2:** 3-(3-Bromo-phenyl)-7-tert-butyl-1H-quinolin-4-one

To 2-(3-Bromo-phenyl)-3-(3-tert-butyl-phenylamino)-acrylic acid ethyl ester (151mg, 0.388 mmol) was added 10g ofpolyphosphoric acid. The resulting mixture was heated at 140°C for 90 minutes. 80 mL of water was added. The mixture was stirred for 40 minutes. The resulting precipitate was filtered, washed with water, and air dried for 3 days to yield 3-(3-Bromo-phenyl)-7-tert-butyl-1H-quinolin-4-one (123mg. 0.345mmol). MS (ESI) 356 $(M+H)^+$.

**Example 3:** 7-tert-Butyl-3-[3-(4,4,5,5-tetramethyl-[1,3,2]dioxaborolan-2-yl)-phenyl]-1H-quinolin-4-one

3-(3-Bromo-phenyl)-7-tert-butyl-1H-quinolin-4-one (119mg, 0.334mmol), bis(pinacolato)diboron (102mg, 0.401mmol), and potassium acetate (98mg, 1.0mmol) were deposited in a sealed vessel with 2mL of DMSO. Argon was bubbled through the mixture for 1 minute. [1,1'-Bis(diphenylphosphino)ferrocene]dichloropalladium(II) complex with DCM (8.0mg, 0.0098 mmol) was added. Argon was bubbled through the mixture for one minute and the lid was tightly sealed. The resulting mixture was heated at 80°C for 18 hours prior to being partitioned between ethylacetate and water. The ethylacetate layer was washed with brine, dried over anhydrous magnesium sulfate, concentrated *in vacuo,* and purified by flash chromatography (gradient elution 25 to 50% ethylacetate/hexanes) to yield 7-tert-Butyl-3-[3-(4,4,5,5-tetramethyl-[1,3,2]dioxaborolan-2-yl)-phenyl]-1H-quinolin-4-one (77mg, 0.19mmol). MS (ESI) 404.1 $(M+H)^+$.

**Example 4:** 7-tert-Butyl-3-(3-{1-methyl-5-[5-(morpholine-4-carbonyl)-pyridin-2-ylamino]-6-oxo-1,6-dihydro-pyridin-3-yl}-phenyl)-1H-quinolin-4-one (II-1)

A solution of 5-Bromo-1-methyl-3-[5-(morpholine-4-carbonyl)-pyridin-2-ylamino]-1H-pyridin-2-one (19mg, 0.050mmol), 7-tert-Butyl-3-[3-(4,4,5,5-tetramethyl-[1,3,2]dioxaborolan-2-yl)-phenyl]-1H-quinolin-4-one (20mg, 0.05mmol), tetrakis(triphenylphosphine)palladium(0) (6.0mg, 0.0052mmol), and sodium carbonate (16mg, 0.15mmol) in 2mL 1,2-dimethoxyethane and 1mL water was microwaved at 170°C for 12.5 minutes. The resulting mixture was partitioned between ethyl acetate and water. The ethyl acetate layer was washed with brine, dried over anhydrous magnesium sulfate, concentrated *in vacuo,* and purified by preparative TLC (5% methanol/DCM) to yield 7-tert-Butyl-3-(3-{1-methyl-5-[5-(morpholine-4-carbonyl)-pyridin-2-ylamino]-6-oxo-1,6-dihydro-pyridin-3-yl]-phenyl)-1H-quinolin-4-one (5.7mg, 0.0085mmol). MS (ESI) 590.1 $(M+H)^+$.

**Example 5:** 2-(3-Bromo-2-methyl-phenyl)-3-(3-dimethylamino-phenylamino)-acrylic acid ethyl ester

(3-Bromo-2-methyl-phenyl)-acetic acid benzyl ester (421mg, 1.32mmol) was dissolved in ethyl formate (2.5mL, 31mmol). Sodium hydride (95%, 67mg, 2.6mmol) was added. After stirring for 30 minutes, this was quenched with 1M aq. HCl. This was partitioned between ethyl acetate and water. The ethyl acetate layer was washed with water, washed with brine, dried over anhydrous magnesium sulfate, and concentrated *in vacuo.*
A portion of this material and N,N-Dimethyl-benzene-1,3-diamine (96mg, 0.70mmol) were stirred in 1 mL ethanol for 18 hours. This was concentrated *in vacuo* and purified by flash chromatography (gradient elution 5 to 20% ethyl acetate/hexanes) to yield 2-(3-Bromo-2-methylphenyl)-3-(3-dimethylamino-phenylamino)-acrylic acid ethyl ester (164mg, 0.407mmol). MS (ESI) 405.0 $(M+H)^+$.

**Example 6:** 3-(3-Bromo-2-methyl-phenyl)-7-dimethylamino-1H-quinolin-4-one

To 2-(3-Bromo-2-methyl-phenyl)-3-(3-dimethylamino-phenylamino)-acrylic acid ethyl ester (100mg, 0.248mmol) was added 4g polyphosphoric acid. This stirred at 140°C for 10 minutes. 50 ml water was added and the mixture was stirred. The resulting precipitate was filtered and washed with water. The filtrate was extracted with 10%methanol/DCM solution. The organic layer was dried over anhydrous magnesium sulfate and concentrated *in vacuo.* The resulting residue was combined with the precipitate and purified by flash chromatography (gradient elution 2 to 5% methanol/DCM) to yield 3-(3-Bromo-2-methyl-phenyl)-7-dimethylamino-1H-quinolin-4-one (22mg, 0.062mmol). MS (ESI) 357.0 $(M+H)^+$.

**Example 7:** 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one

5-Bromo-1-methyl-3-[5-(morpholine-4-carbonyl)-pyridin-2-ylamino]-1H-pyridin-2-one (1.00g, 2.55mmol), bis-(pinacolato)diboron (1.94g, 7.64mmol), potassium acetate (750mg, 7.64mmol), 2-(dicyclohexylphoshphino)-2',4',6'-tri-i-propyl-1,1'-biphenyl(121mg, 0.254mmol), and bis(dibenzylidineacetone)palladium(0) (73mg, 0.13mmol) were dissolved in 15 ml degassed 1,4-dioxane. The headspace of the vessel was evacuated and backfilled with argon 5 times. This was heated at 110°C for 3 hours. This was partitioned between ethyl acetate and water. The ethyl acetate layer was washed with brine, dried over anhydrous magnesium sulfate, concentrated *in vacuo,* and purified by flash chromatography (gradient elution 2 to 8% methanol/DCM) to yield 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (0.798g, 1.81mmol). MS (ESI) 441.2 $(M+H)^+$.

**Example 8:** 7-Dimethylamino-3-(2-methyl-3- {1-methyl-5-[5-(morpholine-4-carbonyl)-pyridin-2-ylamino]-6-oxo-1,6-dihydro-pyridin-3-yl}-phenyl)-1H-quinolin-4-one **(II-2)**

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (27mg, 0.062mmol), 3-(3-Bromo-2-methyl-phenyl)-7-dimethylamino-1H-quinolin-4-one (22mg, 0.062mmol), potassium phosphate (26mg, 0.12mmol), 2-(dicyclohexylphoshphino)-2',4',6'-tri-i-propyl-1,1'-biphenyl (1.7mg, 0.0036mmol), and bis-(dibenzylidineacetone)palladium(0) (1.0mg, 0.0018mmol) was added 4mL of degassed 1:3 water/n-butanol. The headspace of the vessel was evacuated and backfilled with argon 4 times. This was heated at 100°C for 1 hour. This was partitioned between ethyl acetate and water. The ethyl acetate layer was washed with brine, dried over anhydrous magnesium sulfate, concentrated *in vacuo,* and purified by preparative TLC (5% methanol/DCM) to yield 7-Dimethyl-amino-3-(2-methyl-3-{1-methyl-5-[5-(morpholine-4-carbonyl)-pyridin-2-ylamino]-6-oxo-1,6-dihydro-pyridin-3-yl}-phenyl)-1H-quinolin-4-one (14mg, 0.024mmol). MS (ESI) 591.1 $(M+H)^+$.

**Example 9:** 6-Fluoro-3,4-dihydro-2H-isoquinolin-1-one

5-Fluoro-indan-1-one (4.00g, 26.6mmol) was dissolved in 40mL DCM and 40mL methanesulfonic acid. This was cooled to 0°C and sodium azide (3.46g, 53.2mmol) was added. After 2 hours, the solution was made basic by slowly adding 20% aq. sodium hydroxide. The resulting mixture was partitioned between DCM and water. The DCM layer was dried over anhydrous magnesium sulfate, *concentrated in vacuo,* and purified by flash chromatography (gradient elution 50 to 100% ethyl acetate/hexanes) to yield 6-Fluoro-3,4-dihydro-2H-isoquinolin-1-one (2.72g, 16.5mmol). MS (ESI) 166.1 $(M+H)^+$.

**Example 10:** 6-Dimethylamino-3,4-dihydro-2H-isoquinolin-1-one

6-Fluoro-3,4-dihydro-2H-isoquinolin-1-one (1.56g, 9.45mmol) was deposited in a sealed vessel with 25mL 33%dimethylamine in ethanol. This was heated at 150°C for 7 hours. The resulting solution was partitioned between ethyl acetate and water. The ethyl acetate layer was washed with brine, dried over anhydrous magnesium sulfate, concentrated *in vacuo,* and purified by flash chromatography (elution with ethyl acetate) to yield 6-Dimethylamino-3,4-dihydro-2H-isoquinolin-1-one (787mg, 4.14mmol). MS (ESI) 191.1 $(M+H)^+$.

**Example 11:** 2-(3-Bromo-phenyl)-6-dimethylamino-3,4-dihydro-2H-isoquinolin-1-one

6-Dimethylamino-3,4-dihydro-2H-isoquinolin-1-one (762mg, 4.01mmol), cuprous iodide (153mg, 0.802mmol) and potassium carbonate (554mg, 4.01mmol)were deposited in a sealed type vessel. 6 mL DMSO and 1,3-dibromobenzene (1.89g, 8.01mmol) were added. Argon was bubbled through the mixture for 2 minutes and the lid was tightly closed. This was heated at 150°C for 24 hours. Cuprous iodide (153mg, 0.802mmol) was added and the mixture was heated at 150°C for an additional 24 hours. This was diluted with dichoromethane and filtered through a pad of celite. The filtrate was partitioned between DCM and 5% aq. ammonium hydroxide. The DCM layer was dried over anhydrous magnesium sulfate, concentrated *in vacuo,* and purified by flash chromatography (gradient elution 25 to 100% ethyl acetate/hexanes) to yield 2-(3-Bromo-phenyl)-6-dimethylamino-3,4-dihydro-2H-isoquinolin-1-one (939mg, 2.72mmol). MS (ESI) 345.0 $(M+H)^+$.

**Example 12:** 6-Dimethylamino-2-[3-(4,4,5,5-tetramethyl-[1,3,2]dioxaborolan-2-yl)-phenyl]-3,4-dihydro-2H-isoquinolin-1-one

2-(3-Bromo-phenyl)-6-dimethylamino-3,4-dihydro-2H-isoquinolin-1-one (163mg, 0.472mmol), bis(pinacolato)diboron (144mg, 0.567mmol), and potassium acetate (138mg, 1.42mmol) were deposited in a sealed vessel with 2mL DMSO. Argon was bubbled through the mixture for 1 minute. [1, 1'-Bis(diphenylphosphino)ferrocene]dichloropalladium (II) complex with DCM (12mg, 0.015mmol)) was added. Argon was continuted to bubble through the mixture for one more minute and the lid was tightly closed. This was heated at 80°C for 18 hours. This was partitioned between ethyl acetate and water. The ethyl acetate layer was washed with brine, dried over anhydrous magnesium sulfate, concentrated *in vacuo,* and purified by flash chromatography (30% ethyl acetate/hexanes) to yield 6-Dimethylamino-2-[3-(4,4,5,5-tetramethyl-[1,3,2]-dioxaborolan-2-yl)-phenyl]-3,4-dihydro-2H-isoquinolin-1-one (137mg, 0.349mmol). MS (ESI) 393.2 $(M+H)^+$.

**Example 13:** 6-Dimethylamino-2-(3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one **(I-1)**

5-Bromo-1-methyl-3-[5-(morpholine-4-carbonyl)-pyridin-2-ylamino]-1H-pyridin-2-one (67mg, 0.17mmol), 6-Dimethylamino-2-[3-(4,4,5,5-tetramethyl-[1,3,2]dioxaborolan-2-yl)-phenyl]-3,4-dihydro-2H-isoquinolin-1-one (67mg, 0.17 mmol), tetrakis(triphenylphosphine)palladium(0) (20mg, 0.017mmol), and sodium carbonate (54mg, 0.51mmol) in 2mL 1,2-dimethoxyethane and 1mL water was heated to 170°C for 12.5 minutes in the microwave. The resulting mixture was partitioned between ethyl acetate and water. The ethyl acetate layer was washed with brine, dried over anhydrous magnesium sulfate, concentrated *in vacuo,* and purified by flash chromatography (gradient elution 2 to 5% methanol/DCM) to yield 6-Dimethylamino-2-(3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (40mg, 0.069mmol). MS (ESI) 579.2 $(M+H)^+$.

**Example 14:** 6-Fluoro-2H-isoquinolin-1-one

6-Fluoro-3,4-dihydro-2H-isoquinolin-1-one (149mg, 0.903mmol) was dissolved in 3mL 1,4-dioxane. Argon was bubbled through this solution for 1 minute and 2,3-dichloro-5,6-dicyano-*p*-benzoquinone (205mg, 0.903mmol) was added. This was heated at 100°C for 24 hours. The resulting mixture was partitioned between ethyl acetate and 1M aq. sodium hydroxide. The ethyl acetate layer was washed with brine, dried over anhydrous magnesium sulfate, concentrated *in vacuo,* and purified by flash chromatography (50% ethyl acetate/hexanes) to yield 6-Fluoro-2H-isoquinolin-1-one (54mg, 0.33mmol). MS (ESI) 164.1 $(M+H)^+$.

**Example 15:** 6-Dimethylamino-2H-isoquinolin-1-one

6-Fluoro-2H-isoquinolin-1-one (54mg, 0.33mmol) was deposited in a sealed tube with 5mL 33% dimethylamine in ethanol. This was heated at 150°C for 3.5 hours. This was concentrated *in vacuo* and purified by flash chromatography (gradient elution 50 to 100% ethyl acetate/hexanes) to yield 6-Dimethylamino-2H-isoquinolin-1-one (39mg, 0.21mmol). MS (ESI) 189.1 $(M+H)^+$.

**Example 16:** 2-(3-Bromo-phenyl)-6-dimethylamino-2H-isoquinolin-1-one

6-Dimethylamino-2H-isoquinolin-1-one (39mg, 0.21mmol), cuprous iodide (8.0mg, 0.041 mmol), and potassium carbonate (29mg, 0.21mmol) were deposited in sealed vessel. 3mL DMSO and 1,3-dibromobenzene (98mg, 0.42mmol) were added. Argon was bubbled through the mixture for 2 minutes and the lid was tightly closed. This was heated at 150°C for 5 hours. The resulting mixture was partitioned between ethyl acetate and water. The organic layer was washed with brine, dried over anhydrous magnesium sulfate, concentrated *in vacuo,* and purified by flash chromatography (25% ethyl acetate/hexanes) to yield 2-(3-Bromo-phenyl)-6-dimethyl-amino-2H-isoquinolin-1-one (45mg, 0.13mmol). MS (ESI) 345.0 $(M+H)^+$.

**Example 17:** 6-Dimethylamino-2-(3-{1-methyl-5-[5-(morpholine-4-carbonyl)-pyridin-2-ylamino]-6-oxo-1,6-dihydro-pyridin-3-yl}-phenyl)-2H-isoquinolin-1-one **(III-1)**

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (58mg, 0.13mmol), 2-(3-Bromo-phenyl)-6-dimethylamino-2H-isoquinolin-1-one (45mg, 0.13mmol), potassium phosphate (56mg, 0.26mmol), 2-(dicyclohexylphoshphino)-2',4',6'-tri-i-propyl-1,1'-biphenyl (3.7mg, 0.0078 mmol), and bis(dibenzylidineacetone)palladium(0) (2.2mg, 0.0038mmol) was added 4mL of degassed 1:3 water/n-butanol. The headspace of the vessel was evacuated and backfilled with argon 4 times. This was heated at 100°C for 2 hours. This was partitioned between ethyl acetate and water. The ethyl acetate layer was washed with brine, dried over anhydrous magnesium sulfate, concentrated *in vacuo,* and purified by preparative TLC (5% methanol/DCM) to yield 6-Dimethylamino-2-(3-{1-methyl-5-[5-(morpholine-4-carbonyl)-pyridin-2-ylamino]-6-oxo-1,6-dihydro-pyridin-3-yl}-phenyl)-2H-isoquinolin-1-one (45mg, 0.078mmol). MS (ESI) 577.1 $(M+H)^+$.

**Example 18:** 1,3-Dibromo-2-bromomethyl-benzene

2,6-dibromotoluene (2.50g, 10.0mmol) was dissolved in 20 mL carbontetrachloride. N-bromosuccinimide (1.87g, 10.5mmol) was added followed by benzoyl peroxide (73mg, 0.30mmol). The resulting mixture was heated at reflux for 90 minutes. 50 mL petroleum ether was added. This was filtered and concentrated *in vacuo* to yield 1,3-Dibromo-2-bromomethyl-benzene (3.52g, 10.7mmol). $^1$HNMR (300MHz, CDCl;) $\delta$ 4.83 (s, 3H), 7.02 (t, *J*= 8 Hz, 1H), and 7.55 (d, *J*= 8 Hz, 2H).

**Example 19:** Acetic acid 2,6-dibromo-benzyl ester

To 1,3-Dibromo-2-bromomethyl-benzene (3.35g, 10.2mmol) was added potassium acetate (4.00g, 40.8mmol) and 25mL N,N-dimethylformamide. This was heated at 70°C for 20 minutes. The resulting mixture was partitioned between water and ethyl acetate. The ethyl acetate layer was washed with water, washed with brine, dried over anhydrous magnesium sulfate, concen*trated in vacuo,* and purified by flash chromatography (gradient elution 0 to 5% ethyl acetate hexanes) to yield Acetic acid 2,6-dibromo-benzyl ester (1.92g, 6.23mmol). $^1$HNMR (300MHz, $CDCl_3$) $\delta$ 2.12 (s, 3H), 5.42 (s, 2H), 7.08 (t, *J*= 8 Hz, 1H), and 7.58 (d, *J*= 8 Hz, 2H).

**Example 20:** Acetic acid 2-bromo-6-(6-dimethylamino-1-oxo-1H-isoquinolin-2-yl)-benzyl ester

6-Dimethylamino-2H-isoquinolin-1-one (50mg, 0.27mmol), Acetic acid 2,6-dibromo-benzyl ester (164mg, 532mmol), cuprous iodide (10mg, 0.053mmol), and potassium carbonate (37mg, 0.27mmol) were deposited in sealed vessel. 3mL DMSO was added. Argon was bubbled through the mixture for 2 minutes and the lid was tightly closed. This was heated at 150°C for 5 hours. The resulting mixture was partitioned between ethyl acetate and water. The organic layer was washed with brine, dried over anhydrous magnesium sulfate, concentrated *in vacuo,* and purified by flash chromatography (gradient elution 30 to 40% ethylacetate/hexanes) to yield Acetic acid 2-bromo-6-(6-dimethylamino-1-oxo-1H-isoquinolin-2-yl)-benzyl ester (48mg, 0.12mmol). MS (ESI) 417.0 $(M+H)^+$.

**Example 21:** Acetic acid 2-(6-dimethylamino-1-oxo-1H-isoquinolin-2-yl)-6-(1-methyl-5-[5-(morpholine-4-carbonyl)-pyridin-2-ylamino]-6-oxo-1,6-dihydro-pyridin-3-yl}-benzyl ester

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (43mg, 0.097mmol), Acetic acid 2-bromo-6-(6-dimethyl-amino-1-oxo-1H-isoquinolin-2-yl)-benzyl ester (40mg, 0.097mmol), potassium phosphate (41mag, 0.19mmol), 2-(dicyclohexylphoshphino)-2',4',6'-tri-i-propyl-1,1'-biphenyl (2.7mg, 0.0057mmol), and bis(dibenzylidineacetone)palladium(0) (1.6mg, 0.0028mmol) was added 4mL of degassed 1:3 water/n-butanol. The headspace of the vessel was evacuated and backfilled with argon 4 times. This was heated at 100°C for 110 minutes. This was partitioned between ethyl acetate and water. The ethyl acetate layer was washed with brine, dried over anhydrous magnesium sulfate, concentrated *in vacuo,* and purified by flash chromatography (gradient elution 2 to 5% methanol/DCM) to yield Acetic acid 2-(6-dimethylamino-1-oxo-1H-isoquinolin-2-yl)-6-{1-methyl-5-[5-(morpholine-4-carbonyl)-pyridin-2-ylamino]-6-oxo-1,6-dihydro-pyridin-3-yl}-benzyl ester (33mg, 0.051mmol). MS (ESI) 649.2 $(M+H)^+$.

**Example 22:** 6-Dimethylamino-2-(2-hydroxymethyl-3- {1-methyl-5-[5-(morpholine-4-carbonyl)-pyridin-2-ylamino]-6-oxo-1,6-dihydro-pyridin-3-yl}-phenyl)-2H-isoquinolin-1-one **(III-3)**

To Acetic acid 2-(6-dimethylamino-1-oxo-1H-isoquinolin-2-yl)-6-{1-methyl-5-[5-(morpholine-4-carbonyl)-pyridin-2-ylamino]-6-oxo-1,6-dihydro-pyridin-3-yl}-benzyl ester (29mg, 0.045 mmol) in 2ml THF, 1ml methanol, and 1 ml water was added 1M aq. lithium hydroxide solution (0.13mL, 0.13mmol). After stirring for 18 hours, this was partitioned between ethyl acetate and water. The ethyl acetate layer was washed with brine, dried over anhydrous magnesium sulfate, concentrated *in vacuo,* and purified by preparative TLC (5% methanol/DCM) to yield 6-Dimethylamino-2-(2-hydroxymethyl-3-{1-methyl-5-[5-(morpholine-4-carbonyl)-pyridin-2-ylamino]-6-oxo-1,6-dihydro-pyridin-3-yl}-phenyl)-2H-isoquinolin-1-one (20mg, 0.033mmol). MS (ESI) 607.2 $(M+H)^+$.

**Example 23:** 2-(3-Bromo-2-methyl-phenyl)-6-dimethylamino-2H-isoquinolin-1-one

6-Dimethylamino-2H-isoquinolin-1-one (50mg, 0.27mmol), cuprous iodide (10mg, 0.053mmol), and potassium carbonate (37mg, 0.27mmol) were deposited in sealed vessel. 3mL DMSO and 2,6-dibromotoluene (133mg, 0.532mmol) were added. Argon was bubbled through the mixture for 2 minutes and the lid was tightly closed. This was heated at 150°C for 5 hours. The resulting mixture was partitioned between ethyl acetate and water. The organic layer was washed with brine, dried over anhydrous magnesium sulfate, concentrated *in vacuo,* and purified by flash chromatography (30% ethyl acetate/hexanes) to yield 2-(3-Bromo-2-methyl-phenyl)-6-dimethyl-amino-2H-isoquinolin-1-one (43mg, 0.12mmol). MS (ESI) 357 $(M+H)^+$.

**Example 24:** 6-Dimethylamino-2-(2-methy)-3-{1-methyl-5-[5-(morpholine-4-carbonyl)-pyridin-2-ylamino]-6-oxo-1,6-dihydro-pyridin-3-yl}-phenyl)-2H-isoquinolin-1-one **(III-2)**

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (69mg, 0.16mmol), 2-(3-Bromo-2-methyl-phenyl)-6-dimethylamino-2H-isoquinolin-1-one (36mg, 0.10mmol), potassium phosphate (43mg, 0.20mmol), 2-(dicyclohexylphoshphino)-2',4',6'-tri-i-propyl-1,1'-biphenyl (2.9mg, 0.0061 mmol), and bis-(dibenzylidineacetone)palladium(0) (1.7mg, 0.0030mmol) was added 4mL of degassed 1:3 water/n-butanol. The headspace of the vessel was evacuated and backfilled with argon 4 times. This was heated at 100°C for 110 minutes. This was partitioned between ethyl acetate and water. The ethyl acetate layer was washed with brine, dried over anhydrous magnesium sulfate, concentrated *in vacuo,* and purified by flash chromatography (gradient elution 2 to 10% methanol/DCM) to yield 6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-2H-isoquinolin-1-one (46mg, 0.078mmol). MS (ESI) 591.1 $(M+H)^+$.

**Example 25:** 2-(3-Bromo-2-methyl-phenyl)-6-dimethylamino-3,4-dihydro-2H-isoquinolin-1-one

6-Dimethylamino-3,4-dihydro-2H-isoquinolin-1-one (150mg, 0.789mmol), cuprous iodide (30mg, 0.16mmol) and potassium carbonate (109mg, 0.789mmol) were deposited in a sealed vessel. 3 mL DMSO and 2,6-dibromotoluene (395mg, 1.58mmol) were added. Argon was bubbled through the mixture for 2 minutes and the lid was tightly closed. This was heated at 150°C for 24 hours. Cuprous iodide (30mg, 0.16mmol) was added and the mixture was heated at 150°C for an additional 24 hours. This was diluted with dichoromethane and filtered through a pad of celite. The filtrate was partitioned between DCM and 5% aq. ammonium hydroxide. The DCM layer was washed with brine. The combined aqueous layers were washed with DCM. The combined DCM layers were dried over anhydrous magnesium sulfate, concentrated *in vacuo,* and purified by flash chromatography (gradient elution 25 to 50% ethyl acetate/hexanes) to yield 2-(3-Bromo-2-methyl-phenyl)-6-dimethylamino-3,4-dihydro-2H-isoquinolin-1-one (181mg, 0.504mmol). MS (ESI) 361.1 $(M+H)^+$.

**Example 26:** 6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one **(1-3)**

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (77mg, 0.18mmol), 2-(3-Bromo-2-methyl-phenyl)-6-dimethylamino-3,4-dihydro-2H-isoquinolin-1-one (63mg, 0.18mmol), potassium phosphate (74mg, 0.35mmol), 2-(dicyclohexylphoshphino)-2',4',6'-tri-i-propyl-1,1'-biphenyl (5.0mg, 0.010mmol), and bis(dibenzylidineacetone)palladium(0) (3.0mg, 0.0052mmol) was added 4mL of degassed 1:3 water/n-butanol. The headspace of the vessel was evacuated and backfilled with argon 4 times. This was heated at 100°C for 110 minutes. This was partitioned between ethyl acetate and water. The ethyl acetate layer was washed with brine, dried over anhydrous magnesium sulfate, concentrated *in vacuo,* and purified by flash chromatography (gradient elution 2 to 5% methanol DCM) to yield 6-Dimethylamino-2-(2-methyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (33mg, 0.056mmol). MS (ESI) 593.3 $(M+H)^+$.

**Example 27:** Acetic acid 2-bromo-6-(6-dimethylamino-1-oxo-3,4-dihydro-1H-isoquinolin-2-yl)-benzyl ester

6-Dimethylamino-3,4-dihydro-2H-isoquinolin-1-one (150mg, 0.789mmol), Acetic acid 2,6-dibromo-benzyl ester (487mg, 1.58mmol), cuprous iodide (30mg, 0.16mmol) and potassium carbonate (109mg, 0.789mmol) were deposited in a sealed vessel. 3 mL DMSO was added. Argon was bubbled through the mixture for 2 minutes and the lid was tightly closed. This was heated at 150°C for 24 hours. Cuprous iodide (30mg, 0.16mmol) was added and the mixture was heated at 150°C for an additional 24 hours. This was diluted with dichoromethane and filtered through a pad of celite. The filtrate was partitioned between DCM and 5% aq. ammonium hydroxide. The DCM layer was dried over anhydrous magnesium sulfate, concentrated *in vacuo,* and purified by flash chromatography (gradient elution 25 to 50% ethyl acetate/hexanes) to yield Acetic acid 2-bromo-6-(6-dimethylamino-1-oxo-3,4-dihydro-1H-isoquinolin-2-yl)-benzyl ester (93mg, 0.22mmol). MS (ESI) 417.1 $(M+H)^+$.

**Example 28:** 6-Dimethylamino-2-(2-hydroxymethyl-3- {1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one **(I-2)**

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (60mg, 0.14mmol), Acetic acid 2-bromo-6-(6-dimethyl-amino-1-oxo-3,4-dihydro-1H-isoquinolin-2-yl)-benzyl ester (46mg, 0.11mmol), potassium phosphate (47mg, 0.22mmol), 2-(dicyclohexylphoshphino)-2',4',6'-tri-i-propyl-1,1'-biphenyl (3.1mg, 0.0065mmol), and bis(dibenzylidineacetone)palladium(0) (1.9mag, 0033mmol) was added 4mL of degassed 1:3 water/n-butanol. The headspace of the vessel was evacuated and backfilled with argon 4 times. This was heated at 100°C for 110 minutes. This was partitioned between ethyl acetate and water. The ethyl acetate layer was washed with brine, dried over anhydrous magnesium sulfate, and concentrated *in vacuo.* This was dissolved in 2mL THF, 1 mL methanol, and 1 mL water. 1M aq. lithium hydroxide solution (0.33mL, 0.33mmol) was added. After stirring for 18 hours, the resulting mixture was partitioned between ethyl acetate and water. The ethyl acetate layer was washed with brine, dried over anhydrous magnesium sulfate, concentrated *in vacuo,* and purified by flash chromatography (gradient elution 2 to 5% methanol/DCM) to yield 6-Dimethylamino-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (36mg, 0.059 mmol). MS (ESI) 609.1 $(M+H)^+$.

**Example 29:** Acetic acid 2-bromo-6-(6-methylamino-1-oxo-3,4-dihydro-1H-isoquinolin-2-yl)-benzyl ester

To a solution of acetic acid 2-bromo-6-(6-dimethylamino-1-oxo-3,4-dihydro-1H-isoquinolin-2-yl)-benzyl ester (224mg, 0.482mmol) in 5mL 1,4-dioxane was added 2,3-dichloro-5,6-dicyano-p-benzoquinone (109mg, 0.48mmol). After stirring for 4 hours, this was partitioned between ethylacetate and 1M aq. NaOH. The organic layer was washed with water, washed with brine, dried over anhydrous magnesium sulfate, concentrated *in vacuo,* and purified by flash chromatography (gradient elution 40 to 50% ethylacetate/hexanes) to yield acetic acid 2-bromo-6-(6-methylamino-1-oxo-3,4-dihydro-1H-isoquinolin-2-yl)-benzyl ester (93mg, 0.23mmol). MS (ESI) 404.8 $(M+H)^+$.

**Example 30:** 2-(2-Hydroxymethyl-3-{1-methyl-5-[5-(morpholine-4-carbonyl)-pyridin-2-ylamino]-6-oxo-1,6-dihydro-pyridin-3-yl)-phenyl)-6-methylamino-3,4-dihydro-2H-isoquinolin-1-one

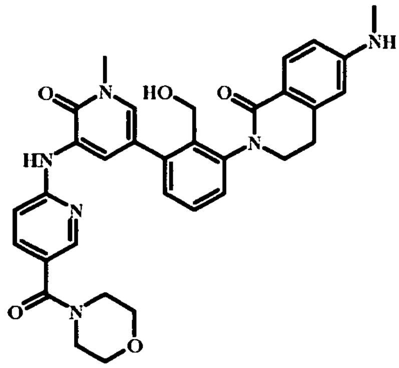

To 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (96mg, 0.22mmol), acetic acid 2-bromo-6-(6-methylamino-1-oxo-3,4-dihydro-1H-isoquinolin-2-yl)-benzyl ester (88mg, 0.22mmol), potassium phosphate (46mg, 0.22mmol), 2-(dicyclohexylphoshphino)-2',4',6'-tri-i-propyl-1,1'-biphenyl (6.2mg, 0.013mmol), and bis(dibenzylidineacetone)palladium(0) (3.7mg, 0064mmol) was added 4mL of degassed 1:3 water/*n*-butanol. The headspace of the vessel was evacuated and backfilled with argon 4 times. This was heated at 100°C for 2 hours. This was partitioned between ethyl acetate and water. The ethyl acetate layer was washed with brine, dried over anhydrous magnesium sulfate, and concentrated *in vacuo.* This was dissolved in 2mL THF, 1 mL methanol, and 1 mL water. 1M aq. lithium hydroxide solution (0.65mL, 0.65mmol) was added. After stirring for 18 hours, the resulting mixture was partitioned between ethyl acetate and water. The ethyl acetate layer was washed with brine, dried over anhydrous magnesium sulfate, concentrated *in vacuo,* and purified by preparative TLC (5% methanol/DCM) to yield 2-(2-Hydroxymethyl-3-{1-methyl-5-[5-(morpholine-4-carbonyl)-pyridin-2-ylamino]-6-oxo-1,6-dihydro-pyridin-3-yl}-phenyl)-6-methylamino-3,4-dihydro-2H-isoquinolin-1-one (43mg, 0.072mmol). MS (ESI) 595 $(M+H)^+$.

**Example 31:** Acetic acid 2-(6-dimethylamino-1-oxo-3,4-dihydro-1H-isoquinolin-2-yl)-6-(4,4,5,5-tetramethyl-[1,3,2]dioxaborolan-2-yl)-benzyl ester

To acetic acid 2-bromo-6-(6-dimethylamino-1-oxo-3,4-dihydro-1H-isoquinolin-2-yl)-benzyl ester (422mg, 1.01mmol), bis(pinacolato)diboron (308mg, 1.21mmol), and potassium acetate (298mg, 3.03mmol) in a sealed tube was added 5 mL dimethylsulfoxide. Argon was bubbled through this mixture for 3 minutes. [1,1'-Bis(diphenylphosphino) ferrocene]dichloropalladium-(II) complex with DCM (25mg, 0.030mmol) was added. Argon was continuted to bubble through the mixture for one more minute and the lid was tightly closed. This was heated at 80°C for 18 hours. This was partitioned between ethyl acetate and water. The ethyl acetate layer was washed with brine, dried over anhydrous magnesium sulfate, concentrated *in vacuo,* and purified by flash chromatography (gradient elution 30 to 50% ethyl acetate/hexanes) to yield acetic acid 2-(6-dimethylamino-1-oxo-3,4-dihydro-1H-isoquinolin-2-yl)-6-(4,4,5,5-tetramethyl-[1,3,2]dioxaborolan-2-yl)-benzyl ester (251mg, 0.541mmol). MS (ESI) 487.2 $(M+Na)^+$.

**Example 32:** 6-Dimethylamino-2-{2-hydroxymethyl-3-[1-methyl-5-(1-methyl-1H-pyrazol-3-ylamino)-6-oxo- 1,6-dihydro-pyridin-3-yl]-phenyl}-3,4-dihydro-2H-isoquinolin-1-one

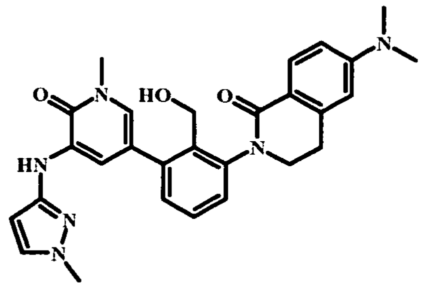

5-Bromo-1-methyl-3-(1-methyl-1H-pyrazol-3-ylamino)-1H-pyridin-2-one (35mg, 0.13mmol), acetic acid 2-(6-dimethylamino-1-oxo-3,4-dihydro-1H-isoquinolin-2-yl)-6-(4,4,5,5-tetramethyl-[1,3,2]dioxaborolan-2-yl)-benzyl ester (58 mg, 0.13mmol), tetrakis(triphenylphosphine)palladium(0) (14mg, 0.012mmol), and sodium carbonate (40mg, 0.38mmol) were dissolved in 2mL 1,2-dimethoxyethane and 1mL water. This was microwaved at 120°C for 30 minutes. This was partitioned between ethylacetate and water. The organic layer was washed with brine, dried over anhydrous magnesium sulfate, and concentrated *in vacuo.* This was dissolved in 3mL THF, 1.5 mL methanol, and 1.5mL water. 1M aq. Lithium hydroxide solution (0.38mL, 0.38mmol) was added. This stirred for 3 hours. This was partitioned between ethylacetate and water. The organic layer was washed with brine, dried over anhydrous magnesium sulfate, concentrated *in vacuo,* and purified by preparative TLC (5% methanol/DCM) to yield 6-Dimethylamino-2-{2-hydroxymethyl-3-[1-methyl-5-(1-methyl-1H-pyrazol-3-ylamino)-6-oxo-1,6-dihydro-pyridin-3-yl]-phenyl}-3,4-dihydro-2H-isoquinolin-1-one (39mg, 0.078 mmol). MS (ESI) 499.2 $(M+H)^+$.

**Example 33:** 1-Methyl-4-(6-nitro-pyridin-3-yl)-piperazine

To 5-Bromo-2-nitro-pyridine (2.00g, 9.85mmol) in 10 mL dimethylsulfoxide was added potassium carbonate (2.72g, 19.7mmol), 1-methylpiperazine (1.64mL, 14.8mmol), and tetrabutylammonium iodide (36mg, 0.097mmol) and was heated at 120°C for 18 hours. The mixture was made acidic with 1M aq. HCl and was partitioned between DCM and water. The aqueous layer was made basic with 2M aq. sodium carbonate and was extracted with DCM. The organic layer was dried over anhydrous magnesium sulfate, concentrated *in vacuo,* and was triturated with water to yield 1-Methyl-4-(6-nitro-pyridin-3-yl)-piperazine (1.82g, 8.19mmol). MS (ESI) 223.1 $(M+H)^+$.

**Example 34**: 5-(4-Methyl-piperazin-1-yl)-pyridin-2-ylamine

1-Methyl-4-(6-nitro-pyridin-3-yl)-piperazine (1.748g, 7.865mmol) was stirred in 30mL methanol with 175mg 10% palladium on carbon under an atmosphere of hydrogen gas for 5 hours. This was filtered and concentrated *in vacuo* to yield 5-(4-Methyl-piperazin-1-yl)-pyridin-2-ylamine (1.485g, 7.724mmol). MS (ESI) 193.1 $(M+H)^+$.

**Example 35:** 5-Bromo-1-methyl-3-[5-(4-methyl-piperazin-1-yl)-pyridin-2-ylamino]-1H-pyridin-2-one

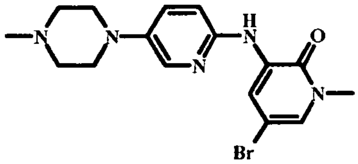

To 5-(4-Methyl-piperazin-1-yl)-pyridin-2-ylamine (1.06g, 5.53mmol), 3,5-Dibromo-1-methyl-1H-pyridin-2-one (1.23g, 4.61mmol), 4,5-bis(diphenylphosphino)-9,9-dimethylphosphino (400mg, 0.691mmol), and cesium carbonate (4.50g, 13.8mmol) was added 45mL 1,4-dioxane and tris(dibenzylidineacetone)dipalladium(0) (422mg, 0.461mmol). This was heated in a 120°C oil bath for 6 hours under argon. This was partitioned between ethylacetate and dilute aqueous sodium bicarbonate. The organic layer was washed with brine, dried over anhydrous magnesium sulfate, concentrated *in vacuo,* and purified by flash chromatography (gradient elution with 2 to 5% methanol/DCM) to yield 5-Bromo-1-methyl-3-[5-(4-methyl-piperazin-1-yl)-pyridin-2-ylamino]-1H-pyridin-2-one (484mg, 1.28mmol). MS (ESI) 380.0 $(M+H)^+$.

**Example 36:** 5-Bromo-1-methyl-3-(5-morpholin-4-yl-pyridin-2-ylamino)-1H-pyridin-2-one

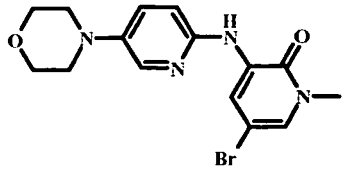

This compound was made analogously to 5-Bromo-1-methyl-3-[5-(4-methyl-piperazin-1-yl)-pyridin-2-ylamino]-1H-pyridin-2-one. MS (ESI) 365.0 $(M+H)^+$,

**Example 37:** 6-(Ethyl-methyl-amino)-3,4-dihydro-2H-isoquinolin-1-one

6-Fluoro-3,4-dihydro-2H-isoquinolin-1-one (2.00g, 12.1mmol) was deposited in a sealed tube with N-ethylmethylamine (4.0mL, 47mmol). This was heated at 150°C for 24 hours. This was concentrated *in vacuo* and purified by flash chromatography (elution with ethylacetate) to yield 6-(Ethyl-methyl-amino)-3,4-dihydro-2H-isoquinolin-1-one (2.10g, 10.3mmol). MS (ESI) 205.1 $(M+H)^+$.

**Example 38:** Acetic acid 2-bromo-6-[6-(ethyl-methyl-amino)-1-oxo-3,4-dihydro-1H-isoquinolin-2-yl]-benzyl ester

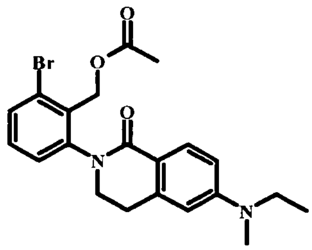

6-(Ethyl-methyl-amino)-3,4-dihydro-2H-isoquinolin-1-one (2.07g, 10.1 mmol), acetic acid 2,6-dibromo-benzyl ester (6.25, 20.3mmol), cuprous iodide (386mg, 2.03 mmol) and potassium carbonate (1.40, 10.1 mmol) were deposited in a sealed vessel. 30 mL DMSO was added. Argon was bubbled through the mixture for 3 minutes and the lid was tightly closed. This was heated at 150°C for 24 hours. Cuprous iodide (386mg, 2.03mmol) was added and the mixture was heated at 150°C for an additional 24 hours. This was diluted with 300mL ethyl acetate and 300mL water. After stirring for 20 minutes, this was filtered through a pad of celite. The layers were separated. The ethylacetate layer was dried over anhydrous magnesium sulfate, concentrated *in vacuo,* and purified by flash chromatography (gradient elution 25 to 50% ethyl acetate/- hexanes) to yield acetic acid 2-bromo-6-[6-(ethyl-methyl-amino)-1-oxo-3,4-dihydro-1H-isoquinolin-2-yl]-benzyl ester (1.21g, 2.81 mmol). MS (ESI) 433.0 $(M+H)^+$.

**Example 39:** 5-Bromo-1-methyl-3-(1-methyl-1H-pyrazol-3-ylamino)-1H-pyridin-2-one

3,5-Dibromo-1-methyl-1H-pyridin-2-one (469mg, 1.76mmol), 1-Methyl-1H-pyrazol-3-ylamine (205mg, 2.11 mmol), tris(dibenzylidineacetone)dipalladium(0) (80mg, 0.087mmol), 2,2'-bis(diphenylphosphino-1,1'-binaphthalene (82mg, 0.13mmol), and cesium carbonate (801mg, 2.46mmol) were deposited in a sealed vial with 10mL toluene. This was heated at 130°C for 18 hours. The resulting mixture was poured into 50 mL water. This was extracted with ethylacetate. The ethylacetate layer was washed with brine, dried over anhydrous magnesium sulfate, filtered, concentrated *in vacuo,* and purified by flash chromatography (eluted with ethylacete/- hexanes) to yield 5-Bromo-1-methyl-3-(1-methyl-1H-pyrazol-3-ylamino)-1H-pyridin-2-one (271mg, 0.957mmol). MS (ESI) 284.9 $(M+H)^+$.

**Example 40:** Acetic acid 2-(6-dimethylamino-1-oxo-1H-isoquinolin-2-yl)-6-(4,4,5,5-tetramethyl-[1,3,2]dioxaborolan-2-yl)-benzyl ester

To Acetic acid 2-bromo-6-(6-dimethylamino-1-oxo-1H-isoquinolin-2-yl)-benzyl ester (420mg, 1.01mmol), bis(pinacolato)diboron (308mg, 1.21mmol), and potassium acetate (298mg, 3.03 mmol) in a sealed tube was added 5 mL dimethylsulfoxide. Argon was bubbled through this mixture for 3 minutes. [1, 1'-Bis(diphenylphosphino)ferrocene]dichloropalladium(II) complex with DCM (25mg, 0.030mmol) was added. Argon was continuted to bubble through the mixture for one more minute and the lid was tightly closed. This was heated at 80°C for 18 hours. This was partitioned between ethyl acetate and water. The ethyl acetate layer was washed with brine, dried over anhydrous magnesium sulfate, concentrated *in vacuo,* and purified by flash chromatography (gradient elution 25 to 50% ethyl acetate/hexanes) to yield Acetic acid 2-(6-dimethyl-amino-1-oxo-1H-isoquinolin-2-yl)-6-(4,4,5,5-tetramethyl-[1,3,2]dioxaborolan-2-yl)-benzyl ester (183mg, 0.396mmol). MS (ESI) 463.1 $(M+H)^+$.

**Example 41:** 6-Dimethylamino-2-{2-hydroxymethyl-3-[1-methyl-5-(1-methyl-1H-pyrazol-3-ylamino)-6-oxo-1,6-dihydro-pyridin-3-yl]-phenyl}-2H-isoquinolin-1-one

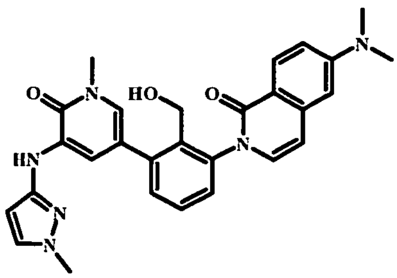

5-Bromo-1-methyl-3-(1-methyl-1H-pyrazol-3-ylamino)-1H-pyridin-2-one (47mg, 0.17mmol), Acetic acid 2-(6-dimethylamino-1-oxo-1H-isoquinolin-2-yl)-6-(4,4,5,5-tetramethyl-[1,3,2]dioxa-borolan-2-yl)-benzyl ester (77mg, 0.17 mmol), tetrakis(triphenylphosphine)palladium(0) (19mg, 0.016mmol), and sodium carbonate (53mg, 0.50mmol) were dissolved in 2mL 1,2-dimethoxyethane and 1 mL water. This was microwaved at 120°C for 30 minutes. This was partitioned between ethylacetate and water. The organic layer was washed with brine, dried over anhydrous magnesium sulfate, and concentrated *in vacuo.* This was dissolved in 3mL THF, 1.5mL methanol, and 1.5mL water. 1M aq. Lithium hydroxide solution (0.5mL, 0.5mmol) was added. This stirred for 3 hours. This was partitioned between ethylacetate and water. The organic layer was washed with brine, dried over anhydrous magnesium sulfate, concentrated *in vacuo,* and purified by preparative TLC (elution with 5% methanol/DCM) to yield 6-Dimethylamino-2-{2-hydroxymethyl-3-[1-methyl-5-(1-methyl-1H-pyrazol-3-ylamino)-6-oxo-1,6-dihydro-pyridin-3-yl]-phenyl}-2H-isoquinolin-1-one (37mg, 0.075mmol). MS (ESI) 497.1 $(M+H)^+$.

**Example 42:** 1-{5-[3-(6-Dimethylamino-1-oxo-1H-isoquinolin-2-yl)-2-hydroxymethyl-phenyl]-1-methyl-2-oxo-1,2-dihydro-pyridin-3-yl} -3-ethyl-urea

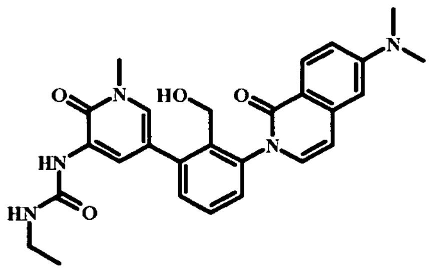

To 1-Ethyl-3-[1-methyl-2-oxo-5-(4,4,5,5-tetramethyl-[1,3,2]dioxaborolan-2-yl)-1,2-dihydro-pyridin-3-yl]-urea (50mg, 0.16mmol), acetic acid 2-bromo-6-(6-dimethylamino-1-oxo-1H-isoquinolin-2-yl)-benzyl ester (65mg, 0.16mmol), potassium phosphate (66mg, 0.31mmol), 2-(dicyclohexylphoshphino)-2',4',6'-tri-i-propyl-1,1'-biphenyl (4.4mg, 0.0092 mmol), and bis-(dibenzylidineacetone)palladium(0) (2.6mg, 0.0045mmol) was added 4mL of degassed 1:3 water/n-butanol. The headspace of the vessel was evacuated and backfilled with argon 4 times. This was heated at 100°C for 2 hours. This was partitioned between ethyl acetate and water. The ethyl acetate layer was washed with brine, dried over anhydrous magnesium sulfate, and concentrated *in vacuo.* This was dissolved in 2mL THF, 1 mL methanol, and

1 mL water. 1M aq. lithium hydroxide solution (0.47mL, 0.47mmol) was added. After stirring for 3 hours, the resulting mixture was partitioned between ethyl acetate and water. The ethyl acetate layer was washed with brine, dried over anhydrous magnesium sulfate, concentrated *in vacuo,* and purified by preparative TLC (5% methanol/DCM) to yield 1-{5-[3-(6-Dimethylamino-1-oxo-1H-isoquinolin-2-yl)-2-hydroxymethyl-phenyl]-1-methyl-2-oxo-1,2-dihydro-pyridin-3-yl{-3-ethyl-urea (33mg, 0.068mmol). MS (ESI) 488.1(M+H)$^+$.

**Example 43:** 5-Bromo-1-methyl-3-(5-morpholin-4-ylmethyl-pyridin-2-ylamino)-1H-pyridin-2-one

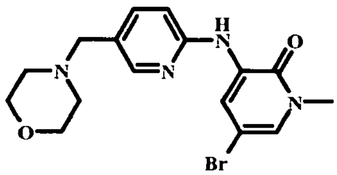

5-Bromo-1-methyl-3-[5-(morpholine-4-carbonyl)-pyridin-2-ylamino]-1H-pyridin-2-one (2.3g, 5.9mmol) was dissolved in 30mL THF. Borane THF complex (2.5g, 29mmol) was added. After stirring for 18 hours, this was concentrated *in vacuo.* Ethanol was added. This was refluxed for one hour. This was concentrated *in vacuo* and purified by flash chromatography to yield 5-Bromo-1-methyl-3-(5-morpholin-4-ylmethyl-pyridin-2-ylamino)-1H-pyridin-2-one (500mg, 1.32mmol). MS (ESI) 381.0 (M+H)$^+$.

**Example 44:** (6-Chloro-pyridin-3-yl)-(4-methyl-piperazin-1-yl)-methanone

To a solution of 6-Chloro-nicotinic acid (3.00g, 19.0mmol) in 30mL dimethylformamide was added (benzotriazol-1-yloxy)tripyrrolidinophosphonium hexafluorophosphate (10.9g, 20.9 mmol), 1-methylpiperazine (2.30g, 22.1 mmol). and triethylamine (2.18g, 21.5mmol). After stirring for 18 hours, theis was partitioned between ethyl acetate and water. The ethylacetate layer was dried over anhydrous sodium sulfate, *concentrated in vacuo,* and purified by flash chromatography (elution with 3% methanol/DCM) to yield (6-Chloro-pyridin-3-yl)-(4-methyl-piperazin-1-yl)-methanone (2.50g, 9.33mmol).

**Example 45:** 5-Bromo-1-methyl-3-[5-(4-methyl-piperazine-1-carbonyl)-pyridin-2-ylamino]-1H-pyridin-2-one

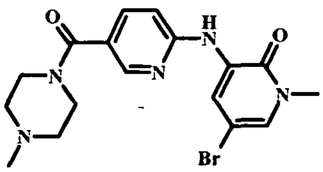

To a solution of (6-Chloro-pyridin-3-yl)-(4-methyl-piperazin-1-yl)-methanone (2.00g, 7.46 mmol) in 10 mL dimethylformamide was added 3-Amino-5-bromo-1-methyl-1H-pyridin-2-one (1.80g, 8.95mmol) and sodium hydiride (537mg, 22.4mmol). After stirring for 18 hours, this was quenched with water. This was extracted with ethylacetate. The ethylacetate layer was dired over anhydrous sodium sulfate, concentrated *in vacuo,* and purified by flash chromatography (gradient elution 0 to 5% methanol/DCM) to yield 5-Bromo-1-methyl-3-[5-(4-methylpiperazine-1-carbonyl)-pyridin-2-ylamino]-1H-pyridin-2-one (900mg, 1.94mmol). MS (ESI) 406.0 (M+H)$^+$.

**Example 56:** 6-Chloro-pyridazin-3-ylamine

3,6-Dichloro-pyridazine (7.5 g, 50.35 mmol) was dissolved in ethanolic ammonia (100 mL) and heated at (130 °C) for overnight in pressure vessel. Then the ethanol was evaporated under reduced pressure and crude purified by silica gel (230-400 mesh) flash chromatography using EtOAc/Hexane (6:4) to afford the title compound (4 g, 61 %) as a solid.

**Example 57:** 4-Bromo-6-chloro-pyridazin-3-ylamine

To a solution of 6-Chloro-pyridazin-3-ylamine (4 g, 31 mmol) in methanol (60 mL) was added $NaHCO_3$ (5.2 g, 62 mmol). The reaction mixture was stirred for 30 minutes at RT then $Br_2$ (4.9 g, 31 mmol) was added drop wise. Then the resulting reaction mixture was stirred additionally for 16 h at RT. After completion of reaction, the reaction mass concentrated under reduced pressure, crude purified by silica gel (100-200 mesh) chromatography using EtOAc/Hexane (8:2) to afford 4-Bromo-6-chloro-pyridazin-3-ylamine (2.3 g, 36 %) as a solid.

**Example 58:** 4-Bromo-6-chloro-2*H*-pyridazin-3-one

To a cooled solution (0-5 °C) of $NaNO_2$ (1 g, 13.20 mmol) in conc. $H_2SO_4$ (15 mL) was added 4-Bromo-6-chloro-pyridazin-3-ylamine (2.3 g, 11 mmol) in 50 mL of acetic acid. Then the reaction mixture was stirred for 1h at 20 °C followed by addition of water (75 mL) and stirring continued for 5 h at RT. The reaction mixture extracted with EtOAc, dried over $Na_2SO_4$, concentrated under reduced pressure and crude purified by silica gel (100-200 mesh) chromatography using EtOAc/Hexane (8:2) to afford **4** (2.2 g, 95 %) yellowish solid.

**Example 59:** 4-Bromo-6-chloro-2-methyl-2H-pyridazine-3-one

4-Bromo-6-chloro-2H-pyridazin-3-one (5.02g, 23.97 mmol) was dissolved in 40 ml dimethylformamide. Cesium carbonate (9.37g, 28.76 mmol) was added. After 5 min, iodomethane (5.103g, 35.95 mmol) was added dropwise over 20 min. The reaction mixture was stirred 3 hours at RT. The precipitate was filtered off and concentrated and the resulting residue was treated with 20 ml DCM. The insoluble material was filtered off again and washed with DCM. The filtrate was concentrated in vacuo to yield 4-Bromo-6-chloro-2-methyl-2H-pyridazine-3-one (5.223 g, 23.37 mmol). MS (ESI) 224.9 $(M+H)^+$

**Example 60:** 6-Chloro-2-methyl-4-(1-methyl-1H-pyrazol-3-ylamino)-2H-pyridazin-3-one

1-Methyl-1H-pyrazol-3-amine (806 mg, 8.3 mmol) was dissolved in 40 ml dioxane. Potassium tert-butoxide (1.793g, 15.98 mmol) was added. Finally 4-Bromo-6-chloro-2-methyl-2H-pyridazine-3-one (1.7 g, 7.61 mmol) was added and the mixture was stirred for 3 hours at RT. The reaction mixture was transfered into an 150 ml Erlenmeyer flask and acidified with 15 ml 1 M aqueous hydrochloric solution, then treated with a saturated sodium bicarbonate solution until the ph reached about 8. It was extracted twice with each 100 ml of DCM; and the organic phase was dried with sodium sulfate, filtered, and concentrated in vacuo to give 1.5 g of a light orange solid. This crude material was triturated with a mixture of DCM and hexane. The suspension was filtered off and the resulting filter cake was dried under high vacuum to yield 6-Chloro-2-methyl-4-(1-methyl-1H-pyrazol-3-ylamino)-2H-pyridazin-3-one (967 mg, 4.03 mmol). MS (ESI) 240.0 $(M+H)^+$

**Example 61:** Acetic acid 2-(6-cyclopropyl-1-oxo-3,4-dihydro-1H-isoquinolin-2yl)-6-[1-methyl-5-(1-methyl-1H-pyrazol-3-ylamino)-6-oxo-1,6-dihydro-pyridazin-3-yl]-benzyl ester

6-Chloro-2-methyl-4-(1-methyl-1H-pyrazol-3-ylamino)-2H-pyridazin-3-one (0.09g, 0.376 mmol), acetic acid 2-(6cyclopropyl-1-oxo-3,4-dihydro-1H-isoquinolin-2yl)-6-(4,4,5,-tetramethyl-[1,3,2]dioxaborolan-2yl)-benzyl ester (0.191g, 0.414 mmol) and cesium carbonate (0.428g, 1.31 mmol) were treated with a degassed solution of 2 ml dioxane/0.2 ml water. After 5 min stirring [1,1'-bis(diphenylphosphino)ferrocene]dichloropalladium(II) complex (0.031g, 0.038 mmol) was added and heated to 135°C for 30 min in the microwave. The reaction mixture was filtered over cellulose, washed with 10 ml of dioxane and concentrated in vacuo. The residue was purified by silica gel chromatography (gradient elution 0-10% methanol in DCM for 20 min) to yield a crude acetic acid 2-(6-cyclopropyl-1-oxo-3,4-dihydro-1H-isoquinolin-2yl)-6-[1-methyl-5-(1-methyl-1H-pyrazol-3-ylamino)-6-oxo-1,6-dihydro-pyridazin-3-yl]-benzyl ester (0.200g, 0.371 mmol). MS (ESI) 540.1 $(M+H)^+$

**Example 62:** 6-Cyclopropyl-2-{2-hydroxymethyl-3-[1-methyl-5-(1-methyl-1H-pyrazol-3-ylamino-6-oxo-1,6-dihydro-pyridazine-3-yl)-phenyl}-3,4-dihydro-2H-isoquinolin-1-one

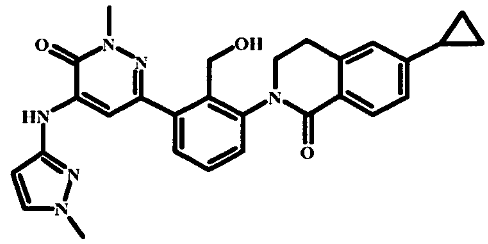

Acetic acid 2-(6-cyclopropyl-1-oxo-3,4-dihydro-1H-isoquinolin-2yl)-6-[1-methyl-5-(1-methyl-1H-pyrazol-3-ylamino)-6-oxo-1,6-dihydro-pyridazin-3-yl]-benzyl ester (0.200g, 0.371 mmol) was dissolved in 2 ml THF, 1 ml water and 1 ml methanol. 1 M aqueous lithium hydroxide solution (1.1 ml, 1.11 mmol) was added and stirred for several hours at RT. It was extracted with DCM /ammonium chloride solution and the organic phase was washed with brine, dried over sodium sulfate, filtered and concentrated. The residue was purified by silica gel chromatography (gradient elution 0-10% methanol in DCM for 20 min) to yield 6-Cyclopropyl-2-{2-hydroxymethyl-3-[1-methyl-5-(1-methyl-1H-pyrazol-3-ylamino-6-oxo-1,6-dihydro-pyridazine-3-yl)-phenyl}-3,4-dihydro-2H-isoquinolin-1-one (0.087g, 0.175 mmol). MS (ESI) 597.2 (M+H) +

**Example 63:** Acetic Acid 2-(6-cyclopropyl-1-oxo-3,4-dihydro-1H-isoquinolin-2yl)-6-{1-methyl-5-[5-(morpholine-4-carbonyl)-pyridin-2-ylamino]-6-oxo-1,6-dihydro-pyridazin-3-yl}-benzyl ester

6-Chloro-2-methyl-4-[5-(morpholine-4-carbonyl)-pyridin-2-ylamino]-2H-pyridazin-3-one (0.070g, 0.2 mmol), acetic acid 2-(6cyclopropyl-1-oxo-3,4-dihydro-1H-isoquinolin-2yl)-6-(4,4,5,-tetramethyl-[1,3,2]dioxaborolan-2yl)-benzyl ester (0.102g, 0.221 mmol) and cesium carbonate (0.228g, 0.7 mmol) were treated with a degassed solution of 1 ml dioxane/0.1 ml water. After 5 min stirring [1,1'-bis(diphenylphosphino)ferrocene]dichloropalladium(II) complex (0.016g, 0.02 mmol) was added and the mixture heated to 135°C for 30 min in the microwave. The reaction mixture was filtered over cellulose; washed with 5 ml of dioxane and concentrated in vacuo. The residue was purified by silica gel chromatography (gradient elution 0-10% methanol in DCM for 20 min) to yield a crude acetic Acid 2-(6-cyclopropyl-1-oxo-3,4-dihydro-1H-isoquinolin-2yl)-6- {1-methyl-5-[5-(morpholine-4-carbonyl)-pyridin-2-ylamino]-6-oxo-1,6-dihydro-pyridazin-3-yl}-benzyl ester (0.121g, 0.187 mmol). MS (ESI) 671.1 $(M+Na)^+$

**Example 64**: 6-Cyclopropyl-2-(2-hydroxymethyl-3-{1-methyl-5-[5-(morpholine-4-carbonyl)-pyridin-2ylamino]-6-1,6-dihydro-pyridazin-3-yl}-phenyl)-3,4-dihydro-2H-isoquinolin-1-one

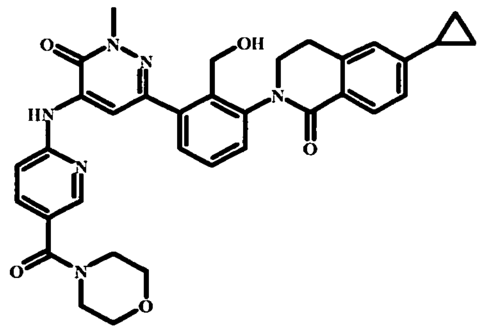

Acetic Acid 2-(6-cyclopropyl-1-oxo-3,4-dihydro-1H-isoquinolin-2yl)-6-{1-methyl-5-[5-(morpholine-4-carbonyl)-pyridin-2-ylamino]-6-oxo-1,6-dihydro-pyridazin-3-yl}-benzyl ester (0.121 g, 0.187 mmol) was dissolved in 2 ml THF, 1 ml water and 1 ml methanol. 1 M aqueous lithium hydroxide solution (0.560 ml, 0.561 mmol) was added and stirred for several hours at RT. It was extracted with DCM /ammonium chloride solution and the organic phase was washed with brine, dried over sodium sulfate, filtered, and concentrated. The residue was purified by silica gel chromatography (gradient elution 0-10% methanol in DCM for 20 min) to yield 6-Cyclopropyl-2-(2-hydroxymethyl-3-{1-methyl-5-[5-(morpholine-4-carbonyl)-pyridin-2ylamino]-6-1,6-dihydro-pyridazin-3-yl}-phenyl)-3,4-dihydro-2H-isoquinolin-1-one (0.070 g, 0.115 mmol). MS (ESI) 607.2 $(M+H)^+$

**Example 65:** 4-Isopropenyl-2-methyl-benzoic acid methyl ester

4-Bromo-2-methyl-benzoic acid methyl ester (4g, 17.46 mmol), isopropenylboronic acid pinacol ester (3.228g, 19.21 mmol) and cesium carbonate (19.913g, 61.11 mmol) were treated with a degassed solution of 15 ml dioxane/5 ml water. After 5 min stirring [1,1'-bis(diphenylphosphino)-ferrocene]dichloropalladium(II) complex (0.718 g, 0.873 mmol) was added and heated to 120°C for 40 min in the microwave. The reaction mixture was filtered over cellulose; washed with 20 ml dioxane and concentrated in vacuo. The residue was purified by 120 g silica gel chromatography (gradient elution 0-50% ethyl acetate in hexane during 50 min) to yield 4-Isopropenyl-2-methyl-benzoic acid methyl ester (2.94 g, 15.45 mmol). MS (ESI) 191.3 $(M+H)^+$

**Example 66:** 2-Methyl-4-(1-methyl-cyclopropyl)-benzoic acid methyl ester

Formation of Diazomethane: N-Nitroso-N-methylurea (9.1g, 61.8 mmol) was added under stirring in portions to a two phase mixture of 50 ml potassium hydroxide solution (23.9g in 50 ml water) and 50 ml diethyl ether at 0°C. The color of the organic phase changed from colorless to yellow. The two phase mixture was vigorously stirred for 40 min at 0°C. The organic layer that contains diazomethane was separated. Cyclopropanation by adding diazomethane solution to methyl styrene: 4-isopropenyl-2-methyl-benzoic acid methyl ester (2.94g, 15.45 mmol) was dissolved in 15 ml diethyl ether and cooled to 0°C. Palladium (II) acetate (0.173g, 0.773 mmol) was added. The yellow organic phase (containing diazomethane) was added dropwise. In total 20 ml of the organic phase (approximately 4 eq. of diazomethane) was added until the reaction was done. You observe releasing nitrogen by adding diazomethane to the methyl styrene intermediate. The reaction mixture was filtered over cellulose; washed with diethyl ether; concentrated; The residue (brown liquid) was purified by 40 g silica gel chromatography (gradient elution 0-100% ethyl acetate in hexane for 15 min) 2.9 g of a crude light yellow liquid was obtained. NMR shows 8% 2-methylbenzoic acid methyl ester. The crude residue was purified again by 110 g flash chromatography (gradient elution 0-20% EtOAc in Hex for 30 min) to give 2-Methyl-4-(1-methyl-cyclopropyl)-benzoic acid methyl ester (2.75g, 13.46 mmol) MS (ESI) 268.9 (M+ $Na^+$ ACN)

**Example 67:** 2-Methyl-4-(1-methyl-cyclopropyl)-benzoic acid

2-Methyl-4-(1-methyl-cyclopropyl)-benzoic acid methyl ester (2.75g, 13.46 mmol) was treated with methanol and 5 M aqueous sodium hydroxide solution (20.46 ml, 102.32 mmol). This solution was heated to 80°C for 4 hours. The reaction mixture was concentrated until methanol was evaporated. A white solid was obtained. The solid was dissolved in 50 ml water under heating then cooled with an ice bath; acidified with 10 ml conc. hydrochloric acid. A white precipitate was formed; filtered; washed with water; dried under high vacuum over night to yield 2-Methyl-4-(1-methyl-cyclopropyl)-benzoic acid (2.18g, 11.46 mmol) MS (ESI) 189.1 $(M-H)^-$

**Example 68:** 2-Methyl-4-(1-methyl-cyclopropyl)-benzoyl chloride

2-Methyl-4-(1-methyl-cyclopropyl)-benzoic acid (2.139g, 11.243 mmol) and phosphoruspenta-chloride (2.575g, 12.37 mmol) were charged into a 50 ml flask under stirring. These both solids dissolved at 100°C. The reaction mixture was stirred 2 hours at 120°C with an reflux condenser in a N2 atmosphere. After that the resulting phosphorus oxylchloride was distilled off at 140°C from the reaction mixture. The whole reaction mixture was cooled to RT and the reaction mixture still remained as a solution. The desired product was distilled by Kugelrohr distillation (150°C / 4 mbar) to give 2-Methyl-4-(1-methyl-cyclopropyl)-benzoyl chloride (1.92g, 9.2 mmol)

**Example 69:** N-[3-Bromo-2-(tert-butyl-dimethyl-silanyloxymethyl)-phenyl]-2-methyl-4-(1-methyl-cyclopropyl)-benzamide

3-Bromo-2-(tert-butyl-dimethyl-silanyloxymethyl)-phenylamine (2.91g, 9.2mmol), 2-Methyl-4-(1-methyl-cyclopropyl)-benzoyl chloride (1.92g, 9.2 mmol), N,N-diisopropylethylamine (2.41 ml, 13.8 mmol) and 4-dimethylaminopyridine (0.112 g, 0.92 mmol) were dissolved in 20 ml anhydrous THF. The reaction mixture was refluxed over night; filtered off the precipitate; concentrated and extracted with ethyl acetate; washed with 2 M phosphate buffer pH 5.5, then with water and brine; dried over sodium sulfate; filtered; concentrated. 4.69g of an oil was obtained. The crude was purified by 80g silica gel chromatography (gradient elution 0-20% ethyl acetate in hexane for 25 min, then 20-100 % ethyl acetate in hexane for 30 min) to give N-[3-Bromo-2-(tert-butyl-dimethyl-silanyloxymethyl)-phenyl]-2-methyl-4-(1-methyl-cyclopropyl)-benzamide (3.51 g, 7.185 mmol) MS (ESI) 510 $(M+Na^+)$

**Example 70:** 2-[3-Bromo-2-(tert-butyl-dimethyl-silanyloxymethyl)-phenyl]-3-hydroxy-7-(1-methyl-cyclopropyl)-3,4-dihydro-2H-isoquinolin-1-one

2,2,6,6-tetramethylpiperidine (2.28g, 16.17 mmol) was dissolved in 13 ml anhydrous THF under stirring; cooled by means of an ethylen glycol/ice bath mixture to -15°C. Buthyllithium, 2.5 M in hexanes (6.16 ml, 15.4 mmol) was added dropwise and the temperature was kept around -15°C and stirred additionally 30 min at -15°C. A solution of N-[3-Bromo-2-(tert-butyl-dimethyl-silanyloxymethyl)-phenyl]-2-methyl-4-(1-methyl-cyclopropyl)-benzamide in 20 ml anhydrous THF was added dropwise over a period of 10 minutes to the reaction mixture at -15°C. The reaction mixture was stirred for 2 hours. After that 3.55 ml of dimethylformamide was added in one portion. The reaction mixture was allowed to warm up to RT. It was stirred for 2 hours at RT, then cooled to 0°C, quenched with 25 ml of 1 M potassium hydrogen sulfate solution; extracted with ethyl acetate/water; organic phase was washed with brine; dried over sodium sulfate; filtered and concentrated. 2.71 g of a brown oil was obtained. Crystallization with DCM and hexane gave 2-[3-Bromo-2-(tert-butyl-dimethyl-silanyloxymethyl)-phenyl]-3-hydroxy-7-(1-methyl-cyclopropyl)-3,4-dihydro-2H-isoquinolin-1-one (1.134g, 2.2 mmol) MS (ESI) 516.0 $(M-H)^-$

**Example 71:** 2-[3-Bromo-2-(tert-butyl-dimethyl-silanyloxymethyl)-phenyl]-7-(1-methyl-cyclopropyl)-2H-isoquinolin-1-one

2-[3-Bromo-2-(tert-butyl-dimethyl-silanyloxymethyl)-phenyl]-3-hydroxy-7-(1-methyl-cyclopropyl)-3,4-dihydro-2H-isoquinolin-1-one (1.134g, 2.2 mmol) was dissolved in 13 ml DCM at RT; triethylamine (1.31 ml, 9.44 mmol) followed by addition of methanesulfonyl chloride (0.478g, 4.171 mmol) were added. It was stirred for 1.5 hours at RT but it's already done in 10 minutes according to LCMS. The reaction mixture was extracted with DCM/water; organic phase was washed with brine; dried over sodium sulfate; filtered; concentrated to give 2-[3-Bromo-2-(tert-butyl-dimethyl-silanyloxymethyl)-phenyl]-7-(1-methyl-cyclopropyl)-2H-isoquinolin-1-one (1.094g, 2.2 mmol) MS (ESI) 520.0 $(M+ Na^+)$

**Example 72:** 2-(2-tert-Butyl-dimethyl-silanyloxymethyl)-3- {1-methyl-5-[5-(morpholine-4-carbonyl)-pyridin-2-ylamino]-6-oxo-1,6-dihydro-pyridin-3-yl}-phenyl)-6-(1-methyl-cyclopropyl)-2H-isoquinolin-1-one

2-[3-Bromo-2-(tert-butyl-dimethyl-silanyloxymethyl)-phenyl]-7-(1-methyl-cyclopropyl)-2H-isoquinolin-1-one (0.102g, 0.205 mmol) and 1-Methyl-3-[5-(morpholine-4-carbonyl)-pyridin-2-ylamino]-5-(4,4,5,5-tetramethyl-[1,3,2]dioxaborolan-2-yl)-1H-pyridin-2-one (0.1g, 0.227 mmol) were dissolved in 2.5 ml dioxane under heating; 0.5 ml of water followed by cesium carbonate (0.259g, 0.795 mmol) were added. After that [1,1'-bis(diphenylphosphino)ferrocene]dichloro-palladium(II) complex (0.019g, 0.023 mmol) was added and heated to 135°C for 30 min by microwave. The reaction mixture was filtered over cellulose; washed with dioxane; concentrated; residue was purifed by 24 g silica gel chromatography (gradient elution DCM for 5 min, then 0-10% methanol in DCM during in 25 min, then DCM 9:1 methanol for 10 min) to yield 2-(2-tert-Butyl-dimethyl-silanyloxymethyl)-3-{1-methyl-5-[5-(morpholine-4-carbonyl)-pyridin-2-ylamino]-6-oxo-1,6-dihydro-pyridin-3-yl}-phenyl)-6-(1-methyl-cyclopropyl)-2H-isoquinolin-1-one (0.095g, 0.13 mmol) MS (ESI) 732.2 $(M+H)^+$

**Example 73:** 2-(2-Hydroxymethyl-3-{1-methyl-5-[5-(morpholine-4-carbonyl)-pyridin-2-ylamino]-6-oxo-1,6-dihydro-pyridin-3-yl}-phenyl)-6-(1-methyl-cyclopropyl)-2*H*-isoquinolin-1-one

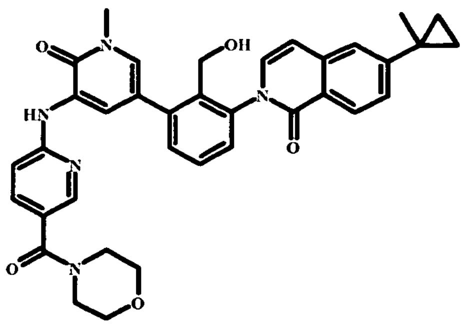

2-(2-tert-Butyl-dimethyl-silanyloxymethyl)-3-{1-methyl-5-[5-(morpholine-4-carbonyl)-pyridin-2-ylamino]-6-oxo-1,6-dihydro-pyridin-3-yl}-phenyl)-6-(1-methyl-cyclopropyl)-2H-isoquinolin-1-one (0.095g, 0.13 mmol) was dissolved in 3

ml dioxane. 3 M of aqueous hydochloric acid solution (0.22 ml, 0.39 mmol) was added at RT. It was stirred for 30 min, then extracted with ethyl acetate/sodium bicarbonate solution; organic phase was washed with brine; dried over sodium sulfate; concentrated. The crude was purified by 12 g silica gel chromatography (gradient elution 0-10% methanol in DCM during 30 min) to yield 2-(2-Hydroxymethyl-3-{1-methyl-5-[5-(morpholine-4-carbonyl)-pyridin-2-ylamino]-6-oxo-1,6-dihydro-pyridin-3-yl} -phenyl)-6-(1-methyl-cyclopropyl)-2*H*-isoquinolin-1-one (0.045g, 0.0728 mmol) MS (ESI) 618.3 $(M+H)^+$

**Example 74:** 6-Bromo-3,4-dihydro-2H-isoquinolin-1-one

Methanesulfonic acid (100 mL) was added to a solution of 5-bromoindanone (25 g, 46 mmol) in $CH_2Cl_2$ (200 mL) at 0 °C. Sodium azide 10.5g, 162 mmol) was added slowly in portions to this mixture. After the sodium azide addition was complete, the mixture was stirred for an additional 30 min, and an aqueous mixture of NaOH (20 wt%) was added until the mixture was slightly basic. The mixture was extracted with methylene chloride, and the combined organic layers were evaporated under reduced pressure. Purification of the mixture by flash column chromatography on silica gel (0% to 50% EtOAc/Hexanes then 0% to 7% MeOH/CH2Cl2) provided 11.5 g of 6-Bromo-3,4-dihydro-2H-isoquinolin-1-one. MS (ESI) 226.1 $(M + H)^+$.

**Example 75**: 6-Cyclopropyl-3,4-dihydro-2H-isoquinolin-1-one

To a round bottomed flask charged with 6-Bromo-3,4-dihydro-2H-isoquinolin-1-one (16.9 g, 74.7 mmol), cyclopropylboronic acid (9.45 g, 1.5 equiv), tricyclohexylphosphine (1.04 mg, 0.025 equiv), and $K_3PO_4$ hexahydrate (50 g, 2 equiv) in toluene (210 mL) and H2O (15 mL) was added $Pd(OAc)_2$ (100 mg, 0.05 equiv).The combined mixture was heated for 4 h at 100 °C. The reaction mixture was cooled, filtered and washed with toluene. The organic phase was partitioned and washed with water and brine, dried over $Na_2SO_4$, filtered and concentrated to an oil. Addition of hexanes produced 6-Cyclopropyl-3,4-dihydro-2H-isoquinolin-1-one as a tan solid (13.6 g). MS (ESI) 187.1 $(M + H)^+$.

**Example 76**: 2-Bromo-6-(6-cyclopropyl-1-oxo-3,4-dihydro-1H-isoquinolin-2-yl)-benzaldehyde

A round bottom flask was charged with 6-Cyclopropyl-3,4-dihydro-2H-isoquinolin-1-one (13.4 g, 5 mmol), 2,6-Dibromo-benzaldehyde (47.5 g, 72.0 mmol), $Pd_2(dba)_3.CHCl_3$ (660 mg, 0.72 mmol), xanthphos (832 mg, 1.44 mmol), and cesium carbonate (46.8 g, 144 mmol). The vial was flushed with argon, 140 mL of dioxane was added, and the reaction mixture waas heated at 110 °C for 4 h. The reaction mixture was cooled to rt and 30 mL of water and 60 mL of ethyl acetate were added before filtering over Solkaflok. The organic phase was separated and washed with brine followed by drying over $Na_2SO_4$. After filtration, the solvent was removed and the brown mass obtained was triturated with methylene chloride and diethyl ether to afford 6.5 grams of 2-Bromo-6-(6-cyclopropyl-1-oxo-3,4-dihydro-1H-isoquinolin-2-yl)-benzaldehyde. A second crop of 7.5 grams of material was collected by addition of more diethyl ether. MS (ESI) 370.0 $(M + H)^+$.

**Example 77**: 2-(3-bromo-2-hydroxymethyl-phenyl)-6-cyclopropyl-3,4-dihydro-2H-isoquinolin-1-one

To a solution of 2-Bromo-6-(6-cyclopropyl-1-oxo-3,4-dihydro-1H-isoquinolin-2-yl)-benzaldehyde (5.0 g, 13.5 mmol) in 60 mL of toluene and 10 mL of THF at -10 °C was added sodium borohydride (740 mg, 20 mmol) in portions. After 30 minutes the reaction mixture was quenched with water and partitioned into diethyl ether. The organic phase was washed with brine, dried over sodium sulfate and filtered. After concentrating under reduced pressure, purification by silica gel chromatography afforded 3.7 g of 2-(3-bromo-2-hydroxymethyl-phenyl)-6-cyclopropyl-3,4-dihydro-2H-isoquinolin-1-one as a colorless solid. MS (ESI) 372.0 $(M + H)^+$.

**Example 78:** 6-Cyclopropyl-2-(2-hydroxymethyl-3-{1-methyl-6-[5-(morpholine-4-carbonyl)-pyridin-2-ylamino]-2-oxo-1,2-dihydro-pyridin-4-yl}-phenyl)-3,4-dihydro-2H-isoquinolin-1-one

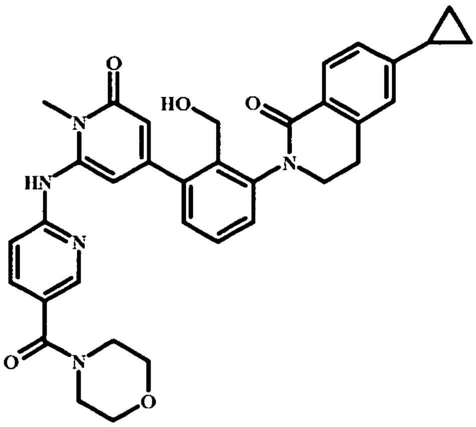

To a flask charged with 2-(3-bromo-2-hydroxymethyl-phenyl)-6-cyclopropyl-3,4-dihydro-2H-isoquinolin-1-one (3.70 g, 9.9 mmol), 1-Methyl-6-[4-(morpholine-4-carbonyl)-phenylamino]-4-(4,4,5,5-tetramethyl-[1,3,2]dioxaborolan-2-yl)-1H-pyridin-2-one (4.38 g, 9.9 mmol), $Pd_2(dba)_3 \cdot CHCl_3$ (229 mg, 0.25 mmol), 2-Dicyclohexylphosphino-2',4',6'-Tri-I-Propyl-1,1'-Biphenyl (238 mg, 0.50 mmol), and $K_3PO_4$ hexahydrate (7.5g, 20 mmol) was added 40 mL of 4:1 dioxane: water and the mixture heated to reflux for 4 h, cooled and filtered over Solka-Floc®, rinsing with ethyl acetate. Partitioned and washed the organic phase with water and brine. Dried over sodium sulfate, filtered and concentrated to afford a dark oil. Purification by silica gel chromatography (methylene chloride/methanol) afforded 3.2 g of 6-Cyclopropyl-2-(2-hydroxymethyl-3- {1-methyl-6-[4-(morpholine-4-carbonyl)-phenylamino]-2-oxo-1,2-dihydro-pyridin-4-yl}-phenyl)-3,4-dihydro-2H-isoquinolin-1-one as a colorless solid. MS (ESI) 606.1 $(M + H)^+$.

**Example 80:** 1-(4-tert-Butyl-phenyl)-3-chloro-propan-1-one

To aluminum chloride (29.33g, 220mmol) in DCM (300mL) at 0 °C with stirring was added dropwise a solution of t-butyl benzene (31 mL, 200 mmol) and 3-chloropropionyl chloride (19 mL, 200mmol) in DCM. After the addition was complete, the reaction mixture was stirred from 0 °C to RT overnight. Then next morning, TLC indicated that all of the t-butyl benzene was consumed, and the reaction mixture was cooled to 0 °C. With stirring, water (about 120 mL) in a dropwise fashion until the effervescence ceased. Finally, the layers were separated, and the organic layer was washed with water (3X150mL) and then brine (1X150mL). The DCM layer was dried over magnesium sulfate, filtered, concentrated and pumped to dryness to afford the title compound as a light tan powder (45.6g).

**Example 81**: 5-tert-Butyl-indan-1-one

1-(4-tert-Butyl-phenyl)-3-chloro-propan-1-one (45.6g, 447 mmol) was taken up in concentrated sulfuric acid (200mL) and the resulting mixture was heated to 100 °C with stirring for 2.5 hours. TLC indicated that all of the starting material had been consumed. After cooling to RT, the reaction mixture was very carefully poured onto about 1Kg of crushed ice. Then some diethyl ether was added and the mixture was stirred carefully until it had cooled to about RT. Ethyl acetate (1200 mL) was added and after partitioning, the layers were separated. The acidic layer was then further extracted with ethyl acetate (2X200mL). The combined ethyl acetate layers were washed with saturated sodium bicarbonate (5X300mL). Finally the ethyl acetate layer was dried over magnesium sulfate, filtered, concentrated and pumped to dryness to afford the title compound as a colorless oil (15.764g).

**Example 82:** 6-tert-Butyl-3,4-dihydro-2H-isoquinolin-1-one

To 5-tert-Butyl-indan-1-one (15.7g, 83.4mmol) in DCM (150 mL) was added methanesulfonic acid (100mL) and the resulting mixture was cooled to 0 °C. Then sodium azide (10.83g, 2 eq) was added carefully portion-wise over 15 minutes. The resulting mixture was stirred at 0°C for about 2.5 hours. TLC analysis confirmed that all of the 5-tert-Butyl-indan-1-one had been consumed. With stirring at 0°C was added very carefully a solution of aqueous sodium hydroxide (20%) until pH = 14. Then added DCM (1000 mL) and water (500 mL) which results in a large emulsion. The layers were separated and the aqueous layer was further extracted with dichloromethane (2X200mL). Finally the combined DCM layers were washed with brine (9X200mL), dried over magnesium sulfate and filtered through a bed of celite. After concentrating and pumping to dryness there was 13.5g of crude product as a tan solid. Purification on a 400 g Analogix Column eluting with a gradient of 10% to 60% ethyl acetate in hexane provided the correct isomer as a white powder (7.22g) ($(M+H)^+$ =204) and the undesired isomer (1.555g) as a white powder.

**Example 83**: Acetic acid 2-bromo-6-(6-tert-butyl-1-oxo-3,4-dihydro-1H-isoquinolin-2-yl)-benzyl ester

6-tert-Butyl-3,4-dihydro-2H-isoquinolin-1-one (4g, 19.67 mmol), Acetic acid 2,6-dibromo-benzyl ester (12.1g, 2eq), potassium phosphate tribasic (8.35g, 2 eq) and copper iodide (787mg, 0.2 eq) were taken up in dioxane (40mL). Finally added N,N'-Dimethyl-cyclohexane-1,2-diamine (1.24 mL, 0.4eq) and the resulting mixture was heated to reflux for 24 hours, after which time more copper iodide (394mg, 0.1eq) and N,N'-Dimethyl-cyclohexane-1,2-diamine (0.62 mL, 0.2 eq) were added. Stirred an additional 64 hours and then added more copper iodide (400 mg, 0.1 eq). Continued to stir at reflux for a total of 168 hours. Cooled to RT and then added ethylacetate (300mL) and water (100mL,), partitioned and separated the layers. Washed with further water (2X100mL) and then finally washed with brine (X100mL). The ethyl acetate layer was dried over magnesium sulfate, filtered and concentrated to give 4.45 g of crude product. Purification on a 240 g Analogix column afforded the title compound as a white foamy solid (516mg) ($(M+H)^+ = 431$) and recovered 6-tert-Butyl-3,4-dihydro-2H-isoquinolin-1-one (2.188g).

**Example 84:** Acetic acid 2-(6-tert-butyl-1-oxo-3,4-dihydro-1H-isoquinolin-2-yl)-6-(1-methyl-5-[5-(morpholine-4-carbonyl)-pyridin-2-ylamino]-6-oxo-1,6-dihydro-pyridin-3-yl}-benzyl ester

1-Methyl-3-[5-(morpholine-4-carbonyl)-pyridin-2-ylamino]-5-(4,4,5,5-tetramethyl-[1,3,2]dioxa-borolan-2-yl)-1H-pyridin-2-one (208 mg, 1 eq), Acetic acid 2-bromo-6-(6-tert-butyl-1-oxo-3,4-dihydro-1H-isoquinolin-2-yl)-benzyl ester (203 mg, 0.472 mmol), XPHOS (14mg, 0.06eq), potassium phosphate tribasic (200mg, 2 eq), n-butanol (2.8mL) and water (0.93mL) were charged to a 50 mL round bottom flask, and then nitrogen gas was bubbled through the mixture for 10 minutes, before adding $Pd(dba)_2$ (8 mg, 0.03 eq). The resulting mixture was heated to 100°C for 40 minutes, and by TLC analysis there was no starting material remaining. The reaction mixture was cooled to RT and then added ethyl acetate (150 mL) and water (40 mL). Partitioned and separated the layers and washed further with water (2X40mL) and then brine (1X40mL). Finally, the ethyl acetate layer was dried over magnesium sulfate, filtered and concentrated and pumped to dryness to afford the title compound which was used in the next step without any further purification ($(M+H)^+ = 664$).

**Example 85:** 6-tert-Butyl-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one

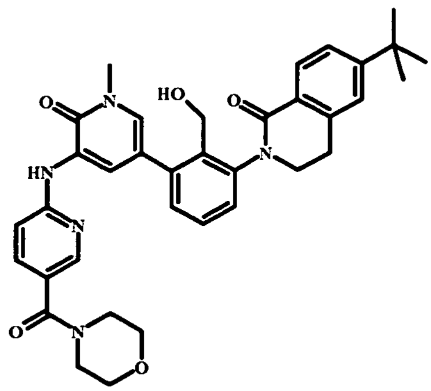

Acetic acid 2-(6-tert-butyl-1-oxo-3,4-dihydro-1H-isoquinolin-2-yl)-6-{1-methyl-5-[5-(morpholine-4-carbonyl)-pyridin-2-ylamino]-6-oxo-1,6-dihydro-pyridin-3-yl}-benzyl ester (0.472 mmol) was taken up in THF (7mL) and methanol (3mL) and water (5mL) and then added lithium hydroxide monohydrate (40 mg, 2 eq). The resulting mixture was stirred at RT overnight. The next morning the reaction was complete by TLC and most of the THF and methanol was removed under reduced pressure at 55°C. Then ethyl acetate (75mL) and water (30 mL) were added and the layers were partitioned and then separated. Next, the ethyl acetate layer was washed with water (2X30mL), brine (1X30mL) and then dried over magnesium sulfate, filtered and concentrated to give 286 mg of crude product. Preparative Thin Layer Chromatography purification eluting on two 20X40 cm 1000 μM plates in 6% methanol in dicholomethane afforded the title compound (99mg) as a white powder ($(M+H)^+ = 622$).

**Example 86:** [2-(4-Methoxy-benzyl)-1-oxo-1,2,3,4-tetrahydro-isoquinolin-6-yl]-acetic acid tert-butyl ester (I)

6-Bromo-2-(4-methoxy-benzyl)-3,4-dihydro-2H-isoquinolin-1-one(1.9 g, 5.5 mmole), Q-phos (0.0632 g, 0.11mmole) and Pd(dba)$_2$ (0.0781g, 0.11 mmole) in 10 ml THF were added to 2-tert-butoxy-2-oxoethylzinc chloride 15 ml (0.55 M) under argon. The reaction mixture was stirred at RT for 16 hours. Next a third of the initial amount of Q-phos, Pd(dba)$_2$ and zinc enolate were added and the mixture heated for 1 hour at 70°C to bring the reaction to completion. The desired product (2 g ; 95.6% yield) was isolated by flash chromatography using silica gel column chromatography with 10% - 40% ethylacetate in hexane as eluent.

**Example 87:** 2-(4-Methoxy-benzyl)-1-oxo-1,2,3,4-tetrahydro-isoquinolin-6-yl]-acetic acid (II)

The tert-butyl ester (I) (1 g, 5.7 mmole) was dissolved in 40 ml methanol and to this solution was added LiOH monohydrate (0.72 g , 17.3 mmole) in 6 ml water. The mixture was stirred at RT for 16 hours, then concentrated in vacuo, acidified with HCl 2N and extracted with ethylacetate. The organic layer was washed with brine, dried over sodium sulfate and concentrated in vacuo. The residue (1.8 g; 97% yield) was used in the next step without further purification.

**Example 88:** 2-[2-(4-Methoxy-benzyl)-1-oxo-1,2,3,4-tetrahydro-isoquinolin-6-yl]-acetamide (III)

To the carboxylic acid (II) (2.3 g,7 mmole) in 22 ml chloroform was added EEDQ (2.07 g, 8.4 mmole) and ammonium bicarbonate (1.66 g, 21 mmole). After stirring the mixture at RT for 16 hours, the amide was precipitated by addition of water (20 ml). The solid was filtered, washed with water and dried in vacuo. The residue was triturated with 50% ethylacetate in hexane, filtered and dried in vacuo to afford 1.4 g amide (III), 63% yield.

**Example 89:** [2-(4-Methoxy-benzyl)-1-oxo-1,2,3,4-tetrahydro-isoquinolin-6-yl]-acetonitrile (IV)

The amide (III) (1.3 g, 4 mmole) was suspended in 5 ml THF and 10 ml DMF. To this mixture was added cyanuric chloride (0.370 g, 2 mmole) and after stirring at RT for 0.5 hour, the reaction mixture was partitioned between ethylacetate and brine; the organic layer was washed with 5% sodium bicarbonate, followed by brine and then dried over sodium sulfate. Purification by flash chromatograpy on silica gel column using 75% ethylacetate in hexane as eluent afforded 1.2 g ( 98% yield) of nitrile (IV).

**Example 90:** 1-[2-(4-Methoxy-benzyl)-1-oxo-1,2,3,4-tetrahydro-isoquinolin-6-yl]-cyclopropanecarbonitrile (V)

To a suspension of sodium hydrate (0.228 g, 60%, 5.72 mmole) in 15 ml DMF was added nitrile (IV) (1.2 g, 3.9 mmole) and after stirring for 15 minutes at RT 1,2-dibromo-ethane (1.1 g, 5.8 mmole) in 1.5 ml DMF was added. The resulting mixture was stirred 0.5 hour at RT and then more sodium hydrate (0.114 g, 2.86 mmole) was added and the reaction mixture heated for about 10 minutes at 30-35°C. After cooling the mixture was partitioned between ethylacetate and brine, the organic layer was dried over sodium acetate and concentrated in vacuo. Purification by silica gel column chromatography with 30% - 50% ethylacetate in hexane afforded compound (V) 1 g (77% yield).

**Example 91:** 1-[2-(4-Methoxy-benzyl)-1-oxo-1,2,3,4-tetrahydro-isoquinolin-6-yl]-cyclopropanecarbaldehyde (VI)

To a solution of nitrile (V) (0.722 g, 2.17 mmole) in 3 ml DCM and 9 ml toluene, cooled at -50°C was added dropwise DIBAH (4.8 ml, 4.77 mmole). After stirring 1 hour at -50°C, the reaction was quenched with 5 ml HCl 1N, left to warm to RT and stirred 0.5 hour. Next the mixture was extracted with ethylacetate; the organic layer was washed with HCl 0.5N, sodium carbonate 5% solution, brine, next dried over sodium sulfate and concentrated in vacuo. The residue was purified by silica gel column chromatography using 30%-60% ethylacetate in hexane to provide aldehyde (VI) 0.075 g (10.3% yield).

**Example 92:** 6-(1-Difluoromethyl-cyclopropyl)-2-(4-methoxy-benzyl)-3,4-dihydro-2H-isoquinolin-1-one (VII)

To a solution of DAST (0.042 g, 0.26 mmole) in 1.5 ml dichloromethylene was added aldehyde (VI) (0.075 g, 0.22 mmole) in 0.5 ml dichloromethylene. This mixture was stirred at RT for 16 hours. After cooling in an ice bath, water 5 ml was added to the reaction mixture followed by ethylacetate. The organic layer was washed with 5% sodium bicarbonate solution and brine, then dried over sodium sulfate and concentrated in vacuo. The residue was purified silica gel prep TLC affording compound (VII) 0.068 g, 87% yield.

**Example 93:** 6-(1-Difluoromethyl-cyclopropyl)-3,4-dihidro-2H-isoqinolin-1-one (VIII)

Compound (VII) (0.068 g, 0.19 mmole) was dissolved in TFA 1 ml and heated to 70°C for 1.5 hours. To the reaction mixture cooled to RT was added ethylacetate and the solution was washed with brine followed by sodium bicarbonate 5% solution, and again with brine. The organic layer was dried over sodium sulfate and concentrated in vacuo. The residue was purified by silica gel prep TLC with 5% methanol in dichloromethylene affording compound (VIII) 0.030 g, 66% yield.

**Example 94:** 2-promo-6-[6-(1-difluoromethyl-cyclopropyl)-1-oxo-3,4-dihydro-1H-isoquinolin-benzaldehyde (IX)

To a mixture of compound (VIII) (0.030 g, 0.12 mmole ), 2,6-dibromo-benzaldehyde (0.064 g, 0.25 mmole), cesium carbonate (0.054 g, 0.16 mmole) and Xantphos (0.002 G, 0.004 mmole) in a microwave tube under argon was added $Pd(dba)_2$ (0.0014 g, 0.0024 mmole). The tube was sealed and the reaction mixture was heated at 100° C for 16 hours. After cooling the mixture was partitioned between ethylacetate and brine, the organic layer dried over sodium sulfate and concentrated in vacuo. The residue was purified by silica gel prep TLC with 40% ethylacetate in hexane as eluent affording 0.024 g, 48% yield.

**Example 95:** 2-[6-(1-Difluoromethyl-cyclopropyl)-1-oxo-3,4-dihydro-1H-isoquinolin-2-yl]-6-{1-methyl-5-[5-(morpholine-4-carbonyl)-pyridin-2-ylamino]-6-oxo-1,6-dihydro-pyridin-3-yl}-benzaldehyde (X)

1-Methyl-3-[5-(morpholine-4-carbonyl)-pyridin-2-ylamino]-5-(4,45,5-tetramethyl-[1,3,2]dioxa-borolan-2-yl)-1H-pyridin-2-one (0.025 g, 0.057 mmole) and benzaldehyde IX (0.024 g, 0.057 mmole) were dissolved in 0.5 ml n-butanol. To this solution under argon was added $K_3PO_4$ (0.024 g, 0.114 mmole), water 0.150 ml, Xphos (0.0027 g, 0.0057 mmole) and $Pd(dba)_2$(0.0016 g, 0.0028 mmole). The mixture was heated 1 hour at 100°C and after cooling partitioned between ethylacetate and brine. The organic layer was dried over sodium sulfate, concentrated in vacuo and the residue purified by silica gel prep TLC to afford 0.025 g (67% yield) of X.

**Example 96:** 6-(1-Difluoromethyl-cyclopropyl)-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (XI)

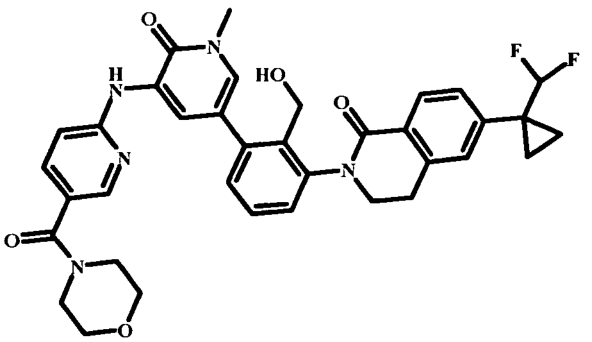

2-[6-(1-Difluoromethyl-cyclopropyl)-1-oxo-3,4-dihydro-1H-isoquinolin-2-yl]-6-{1-methyl-5-[5-(morpholine-4-carbonyl)-pyridin-2-ylamino]-6-oxo-1,6-dihydro-pyridin-3-yl}-benzaldehyde (0.025 g, 0.038 mmole) was dissolved into THF (2 ml). To this solution was added $NaBH_4$ (0.006 g, 0.015 mmole) and the mixture stirred at RT 0.5 hour after which it was quenched with ice water (4 ml) and extracted with ethylacetate. The organic layer was washed with brine, dried over sodium sulfate and concentrated in vacuo. The residue was purified by preparative TLC to afford 6-(1-Difluoromethyl-cyclopropyl)-2-(2-hydroxymethyl-3-{1-methyl-5-[5-(morpholine-4-carbonyl)-pyridin-2-ylamino]-6-oxo-1,6-dihydro-pyridin-3-yl}-phenyl)-3,4-dihydro-2H-isoquinolin-1-one (XI), 0.020 g (80% yield).

**Example 97: Bruton's tyrosine kinase (Btk) inhibition Assay**

The assay is a capture of radioactive $^{33}P$ phosphorylated product through filtration. The interactions of Btk, biotinylated $SH_2$ peptide substrate (Src homology), and ATP lead to phosphorylation of the peptide substrate. Biotinylated product is bound streptavidin sepharose beads. All bound, radio labeled products are detected by scintillation counter.
Plates assayed are 96-well polypropylene (Greiner) and 96-well 1.2 μm hydrophilic PVDF filter plates (Millipore).

Concentrations reported here are final assay concentrations: 10- 100 μM compounds in DMSO (Burdick and Jackson), 5-10 nM Btk enzyme (His-tagged, full-length), 30 μM peptide substrate (Biotin-Aca-AAAEEIYGEI-$NH_2$), 100 μM ATP (Sigma), 8 mM imidazole (Sigma, pH 7.2), 8 mM glycerol-2-phosphate (Sigma), 200 μM EGTA (Roche Diagnostics), 1 mM $MnCl_2$ (Sigma), 20 mM $MgCl_2$ (Sigma), 0.1 mg/ ml BSA (Sigma), 2 mM DTT (Sigma), 1 μCi $^{33}P$ ATP (Amersham), 20% streptavidin sepharose beads (Amersham), 50 mM EDTA (Gibco), 2 M NaCl (Gibco), 2 M NaCl w/ 1% phosphoric acid (Gibco), microscint-20 (Perkin Elmer).

$IC_{50}$ determinations are calculated from 10 data points per compound utilizing data produced from a standard 96-well plate assay template. One control compound and seven unknown inhibitors were tested on each plate and each plate was run twice. Typically, compounds were diluted in half-log starting at 100 μM and ending at 3 nM.The control compound was stauro-sporine. Background was counted in the absence of peptide substrate. Total activity was determined in the presence of peptide substrate. The following protocol was used to determine Btk inhibition.

1) Sample preparation: The test compounds were diluted at half-log increments in assay buffer (imidazole, glycerol-2-phosphate, EGTA, $MnCl_2$, $MgCl_2$, BSA).
2) Bead preparation

a.) rinse beads by centrifuging at 500 g
b.) reconstitute the beads with PBS and EDTA to produce a 20% bead slurry

3) Pre-incubate reaction mix without substrate (assay buffer, DTT, ATP, $^{33}P$ ATP) and mix with substrate (assay buffer, DTT, ATP, $^{33}P$ ATP, peptide substrate) 30°C for 15 min.
4) To start assay, pre-incubate 10 μL Btk in enzyme buffer (imidazole, glycerol-2-phosphate, BSA) and 10μL of test compounds for 10 min at RT.
5) Add 30 μL reaction mixture without or with substrate to Btk and compounds.
6) Incubate 50 μL total assay mix for 30 min at 30°C.
7) Transfer 40 μL of assay to 150 μL bead slurry in filter plate to stop reaction.
8) Wash filter plate after 30 min, with following steps

a. 3 x 250 μL NaCl
b. 3 x 250 μL NaCl containing 1% phosphoric acid
c. 1 x 250 μL $H_2O$

9) Dry plate for 1 h at 65°C or overnight at RT
10) Add 50 μL microscint-20 and count $^{33}P$ cpm on scintillation counter. Calculate percent activity from raw data in cpm

$$\text{percent activity} = (\text{sample} - \text{bkg}) / (\text{total activity} - \text{bkg}) \times 100$$

Calculate $IC_{50}$ from percent activity, using one-site dose response sigmoidal model

$y = A + ((B - A) / (1 + ((x / C)^D)))$
x = cmpd conc, y = % activity, A = min, B = max, C = $IC_{50}$, D = 1 (hill slope)

Representative results are in Table II below:

TABLE II.

| Compound | Btk inhibition $IC_{50}$ (μM) |
|---|---|
| I-1 | 0.42 |
| 1-2 | 0.04 |
| II-2 | 0.37 |
| III-1 | 1.08 |
| III-2 | 2.76 |

**Example 98: Inhibition of B-cell Activation - B cell FLIPR assay in Ramos cells**

Inhibition of B-cell activation by compounds of the present invention is demonstrated by determining the effect of the test compounds on anti-IgM stimulated B cell responses.
The B cell FLIPR assay is a cell based functional method of determining the effect of potential inhibitors of the intracellular calcium increase from stimulation by an anti-IgM antibody. Ramos cells (human Burkitt's lymphoma cell line. ATCC-No. CRL-1596) were cultivated in Growth Media (described below). One day prior to assay, Ramos cells were resuspended in fresh growth media (same as above) and set at a concentration of 0.5 x $10^6$/mL in tissue culture flasks. On day of assay, cells are counted and set at a concentration of 1 x $10^6$/mL1 in growth media supplemented with 1μM FLUO-3AM (TefLabs Cat-No. 0116, prepared in anhydrous DMSO and 10% Pluronic acid) in a tissue culture flask, and incubated at 37°C (4% $CO_2$) for one h. To remove extracellular dye, cells were collected by centrifugation (5min, 1000 rpm), resuspended in FLIPR buffer (described below) at 1 x $10^6$ cells/mL and then dispensed into 96-well poly-D-lysine coated black/clear plates (BD Cat-No. 356692) at 1 x $10^5$ cells per well. Test compounds were added at various concentrations ranging from 100 μM to 0.03 μM (7 concentrations, details below), and allowed to incubate with cells for 30 min at RT. Ramos cell $Ca^{2+}$ signaling was stimulated by the addition of 10 μg/mL anti-IgM (Southern Biotech, Cat-No. 2020-01) and measured on a FLIPR (Molecular Devices, captures images of 96 well plates using a CCD camera with an argon laser at 480nM excitation).

Media/Buffers:

Growth Medium: RPMI 1640 medium with L-glutamine (Invitrogen, Cat-No. 61870-010), 10% Fetal Bovine Serum (FBS, Summit Biotechnology Cat-No. FP-100-05); 1 mM Sodium Pyruvate (Invitrogen Cat. No. 11360-070).
FLIPR buffer: HBSS (Invitrogen, Cat-No. 141175-079), 2mM $CaCl_2$ (Sigma Cat-No. C-4901), HEPES (Invitrogen, Cat-No. 15630-080), 2.5mM Probenecid (Sigma, Cat-No. P-8761), 0.1% BSA (Sigma, Cat-No.A-7906), 11mM Glucose (Sigma, Cat-No.G-7528)

Compound dilution details: In order to achieve the highest final assay concentration of 100 μM, 24 μL of 10 mM compound stock solution (made in DMSO) is added directly to 576 μL of FLIPR buffer. The test compounds are diluted in FLIPR Buffer (using Biomek 2000 robotic pipettor) resulting in the following dilution scheme: vehicle, 1.00 x $10^{-4}$ M, 1.00 x $10^{-5}$, 3.16 x $10^{-6}$, 1.00 x $10^{-6}$, 3.16 x $10^{-7}$, 1.00 x $10^{-7}$, 3.16 x $10^{-8}$.

Intracellular increases in calcium were reported using a max - min statistic (subtracting the resting baseline from the peak caused by addition of the stimulatory antibody using a Molecular Devices FLIPR control and statistic exporting software. The $IC_{50}$ was determined using a nonlinear curve fit (GraphPad Prism software).

**Example 99: Pharmaceutical compositions**

Composition for Oral Administration (A)

| Ingredient | % wt./wt. |
|---|---|
| Active ingredient | 20.0% |
| Lactose | 79.5% |
| Magnesium stearate | 0.5% |

The ingredients are mixed and dispensed into capsules containing about 100 mg each; one capsule would approximate a total daily dosage.

Composition for Oral Administration (B)

| Ingredient | % wt./wt. |
|---|---|
| Active ingredient | 20.0% |
| Magnesium stearate | 0.5% |
| Crosscarmellose sodium | 2.0% |

(continued)

| Ingredient | % wt./wt. |
|---|---|
| Lactose | 76.5% |
| PVP (polyvinylpyrrolidine) | 1.0% |

The ingredients are combined and granulated using a solvent such as methanol. The formulation is then dried and formed into tablets (containing about 20 mg of active compound) with an appropriate tablet machine.

Composition for Oral Administration (C)

| Ingredient | % wt./wt. |
|---|---|
| Active compound | 1.0 g |
| Fumaric acid | 0.5 g |
| Sodium chloride | 2.0 g |
| Methyl paraben | 0.15 g |
| Propyl paraben | 0.05 g |
| Granulated sugar | 25.5 g |
| Sorbitol (70% solution) | 12.85 g |
| Veegum K (Vanderbilt Co.) | 1.0 g |
| Flavoring | 0.035 ml |
| Colorings | 0.5 mg |
| Distilled water | q.s. to 100 ml |

The ingredients are mixed to form a suspension for oral administration.

Parenteral Formulation (D)

| Ingredient | % wt./wt. |
|---|---|
| Active ingredient | 0.25 g |
| Sodium Chloride | qs to make isotonic |
| Water for injection to | 100 ml |

The active ingredient is dissolved in a portion of the water for injection. A sufficient quantity of sodium chloride is then added with stirring to make the solution isotonic. The solution is made up to weight with the remainder of the water for injection, filtered through a 0.2 micron membrane filter and packaged under sterile conditions.

Suppository Formulation (E)

| Ingredient | % wt./wt. |
|---|---|
| Active ingredient | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

Topical Formulation (F)

| Ingredients | grams |
|---|---|
| Active compound | 0.2-2 |
| Span 60 | 2 |
| Tween 60 | 2 |
| Mineral oil | 5 |
| Petrolatum | 10 |
| Methyl paraben | 0.15 |
| Propyl paraben | 0.05 |
| BHA (butylated hydroxy anisole) | 0.01 |
| Water | q.s. 100 |

**Example 100: Mouse Collagen-induced arthritis (mCIA)**

On day 0 mice are injected at the base of the tail or several spots on the back with an emulsion of Type II Collagen (i.d.) in Complete Freund's adjuvant (CFA). Following collagen immunization, animals will develop arthritis at around 21 to 35 days. The onset of arthritis is synchronized (boosted) by systemic administration of collagen in Incomplete Freund's adjuvant (IFA; i.d.) at day 21. Animals are examined daily after day 20 for any onset of mild arthritis (score of 1 or 2; see score description below) which is the signal to boost. Following boost, mice are scored and dosed with candidate therapeutic agents for the prescribed time (typically 2—3 weeks) and dosing frequency, daily (QD) or twice-daily (BID).

**Example 101: Rat Collagen-induced arthritis (rCIA)**

On day 0, rats are injected with an emulsion of Bovine Type II Collagen in Incomplete Freund's adjuvant (IFA) is injected intradermally (i.d.) on several locations on the back. A booster injection of collagen emulsion is given around day 7, (i.d.) at the base of the tail or alternative sites on the back. Arthritis is generally observed 12-14 days after the initial collagen injection. Animals may be evaluated for the development of arthritis as described below (Evaluation of arthritis) from day 14 onwards. Animals are dosed with candidate therapeutic agents in a preventive fashion starting at the time of secondary challenge and for the prescribed time (typically 2-3 weeks) and dosing frequency, daily (QD) or twice-daily (BID).

**Example 102: <u>Evaluation of Arthritis</u>**

In both models, developing inflammation of the paws and limb joints is quantified using a scoring system that involves the assessment of the 4 paws following the criteria described below:

Scoring:

1= swelling and/or redness of paw or one digit.
2= swelling in two or more joints.
3= gross swelling of the paw with more than two joints involved.
4= severe arthritis of the entire paw and digits.

Evaluations are made on day 0 for baseline measurement and starting again at the first signs or swelling for up to three times per week until the end of the experiment. The arthritic index for each mouse is obtained by adding the four scores of the individual paws, giving a maximum score of 16 per animal.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims.

## Claims

1. A compound of Formula I, II or III

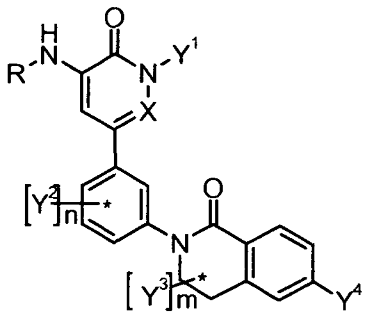
(I)

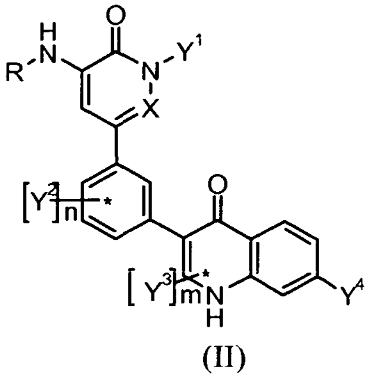
(II)

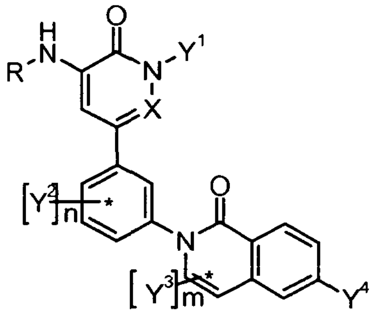
(III)

wherein:

R is H, $-R^1$, $-R^1-R^2-R^3$, $-R^1-R^3$, or $-R^2-R^3$;
$R^1$ is aryl, heteroaryl, cycloalkyl, or heterocycloalkyl, and is optionally substituted with one or more $C_{1-6}$ alkyl, hydroxy, hydroxy $C_{1-6}$ alkyl, $C_{1-6}$ alkoxy, halo, nitro, amino, amido, cyano, oxo, or halo-$C_{1-6}$ alkyl;
$R^2$ is -C(=O), -C(=O)O, -C(=O)$NR^2$, -NHC(=O)O, , -C(=NH)$NR^2$, or $-S(=O)_2$; wherein $R^2$ is H or $C_{1-6}$ alkyl;
$R^3$ is H or $R^4$; wherein $R^4$ is $C_{1-6}$ alkyl, amino, aryl, arylalkyl, alkylaryl, heteroaryl, alkyl heteroaryl, heteroaryl alkyl, cycloalkyl, alkyl cycloalkyl, cycloalkyl alkyl, heterocycloalkyl, alkyl heterocycloalkyl, or heterocycloalkyl alkyl, and is optionally substituted with one or more $C_{1-6}$ alkyl, hydroxy, $C_{1-6}$ alkoxy, hydroxy $C_{1-6}$ alkyl, hydroxy $C_{1-6}$ alkoxy, $C_{1-6}$ alkyl sulfonyl, $C_{1-6}$ alkyl sulfonamido, carbamate, carboxy, ester, amido, acyl, halo, nitro, amino, cyano, oxo, or halo-$C_{1-6}$ alkyl;
X is CH or N;
$Y^1$ is H, $C_{1-6}$ alkyl, or $C_{1-6}$ haloalkyl;
each $Y^2$ is independently halogen, oxime, or $C_{1-6}$ alkyl, wherein $C_{1-6}$ alkyl is optionally substituted with one or more substituents selected from the group consisting of hydroxy, $C_{1-6}$ alkoxy, $C_{1-6}$ haloalkoxy, $C_{1-6}$ haloalkyl, carboxy, amino, and halogen;
n is 0, 1, 2, or 3.
$Y^3$ is H, halogen, or $C_{1-6}$ alkyl, wherein $C_{1-6}$ alkyl is optionally substituted with one or more substituents selected from the group consisting of hydroxy, $C_{1-6}$ alkoxy, amino, and halogen;
m is 0 or 1;
$Y^4$ is $Y^{4a}$, $Y^{4b}$, $Y^{4c}$, or $Y^{4d}$ ; wherein $Y^{4a}$ is H or halogen; $Y^{4b}$ is $C_{1-6}$ alkyl, optionally substituted with one or more substituents selected from the group consisting of $C_{1-6}$ haloalkyl, halogen, hydroxy, amino, cyano, and $C_{1-6}$ alkoxy; $Y^{4c}$ is $C_{1-6}$ cycloalkyl, optionally substituted with one or more substituents selected from the group consisting of $C_{1-6}$ alkyl, $C_{1-6}$ haloalkyl, halogen, hydroxy, amino, cyano, and $C_{1-6}$ alkoxy; and $Y^{4a}$ is amino, optionally susbstituted with one or more $C_{1-6}$ alkyl, alkoxyl $C_{1-6}$ alkyl, or hydroxy $C_{1-6}$ alkyl;

or a pharmaceutically acceptable salt thereof

2. A compound according to claim 1 of formula I.

3. A compound according to claim 1 of formula II.

4. A compound according to claim 1 of formula III.

5. A compound according to claim 2, 3 or 4 wherein $Y^1$ is methyl.

6. A compound according to claim 2, 3 or 4 wherein X is CH.

7. A compound according to claim 2, 3 or 4 wherein n is 1 and m is 0.

**8.** A compound according to claim 2, 3 or 4 wherein $Y^3$ is H.

**9.** A compound according to claim 2, 3 or 4 wherein $Y^2$ is methyl, hydroxymethyl, hydroxyethyl or halogen.

**10.** A compound according to claim 2, 3 or 4 wherein $Y^4$ is

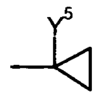

wherein $Y^5$ is halogen, $C_{1-6}$ alkyl or $C_{1-6}$ haloalkyl;

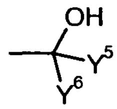

wherein and $Y^6$ are independently H, $C_{1-6}$ alkyl, or $C_{1-6}$ haloalkyl,

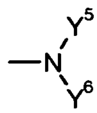

wherein $Y^5$ and $Y^6$ are independently H or $C_{1-6}$ alkyl or

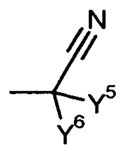

wherein $Y^5$ and $Y^6$ are independently H, $C_{1-6}$ alkyl, or $C_{1-6}$ haloalkyl.

**11.** A compound according to claim 2, 3 or 4 wherein R is $-R^1-R^2-R^3$; wherein $R^1$ is phenyl or pyridyl; $R^2$ is -C(=O); $R^3$ is $R^4$; and $R^4$ is morpholine or piperazine, optionally substituted with one or more $C_{1-6}$ alkyl.

**12.** A compound of any one of claims 1-11 for use in treating an inflammatory and/or autoimmune condition, e.g. arthritis, or of inhibiting B-cell proliferation.

**13.** A pharmaceutical composition comprising the Btk inhibitor compound of any one of claims 1-11, admixed with at least one pharmaceutically acceptable carrier, excipient or diluent.

**14.** The use of a compound of formula I according to any one of claims 1-11 for the preparation of a medicament for the treatment of an inflammatory and/or autoimmune condition.

**Patentansprüche**

**1.** Verbindung der Formel I, II oder III

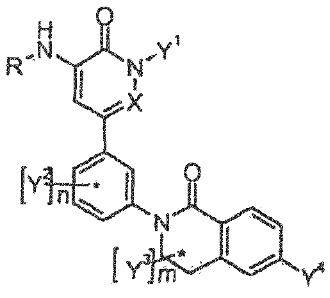

(I)

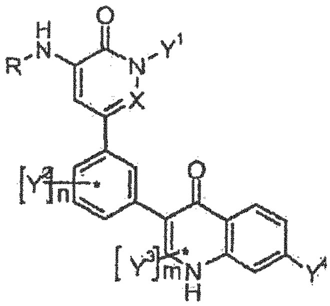

(II)

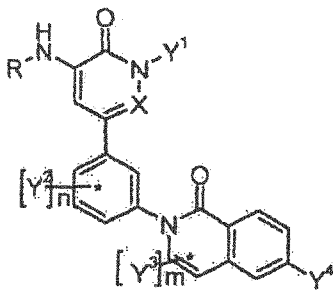

(III)

wobei:

R H, $-R^1$, $-R^1-R^2-R^3$, $-R^1-R^3$ oder $-R^2-R^3$ ist;
$R^1$ Aryl, Heteroaryl, Cycloalkyl Heterocycloalkyl ist und gegebenenfalls mit einem oder mehreren $C_{1-6}$-Alkyl, Hydroxy, Hydroxy-$C_{1-6}$-alkyl, $C_{1-6}$-Alkoxy, Halogen, Nitro, Amino, Amido, Cyano, Oxo oder Halogen-$C_{1-6}$-alkyl substituiert ist;
$R^2$ -C(O), -C(=O)O, -C(=O)NR$^{2'}$, -NHC(=O)O, -C(=NH)NR$^2$ oder -S(=O)$_2$ ist; wobei $R^2$ gleich H oder $C_{1-6}$-Alkyl ist;
$R^3$ In oder $R^4$ ist; wobei $R^4$ $C_{1-6}$-Alkyl, Amino, Aryl, Arylalkyl, Alkylaryl, Heteroaryl, Alkylheteroaryl, Heteroarylalkyl, Cycloalkyl, Alkylcycloalkyl, Cycloalkylalkyl, Heterocycloalkyl, Alkylheteroeyeloalkyl oder Heterocydoalkylalkyl ist und gegebenenfalls mit einem oder mehreren $C_{1-6}$-Alkyl, Hydroxy, $C_{1-6}$-Alkoxy, Hydroxy-$C_{1-6}$-alkyl, Hydroxy-$C_{1-6}$-alkoxy, $C_{1-6}$-Alkylsulfonyl, $C_{1-6}$-Alkylsulfonamido, Carbamat, Carboxy, Ester, Amido, Acyl, Halogen, Nitro, Amino, Cyano, Oxo oder Halogen-$C_{1-6}$-alkyl substituiert ist;
X CH oder N ist;
$Y^1$ H, $C_{1-6}$-Alkyl oder $C_{1-6}$-Halogenalkyl ist;
jedes $Y^2$ unabhängig Halogen, Oxim oder $C_{1-6}$-Alkyl ist, wobei $C_{1-6}$-Alkyl gegebenenfalls mit einem oder mehreren Substituenten, ausgewählt aus der Gruppe bestehend aus Hydroxy, $C_{1-6}$-Alkoxy, $C_{1-6}$-Halogenalkoxy, $C_{1-6}$-Halogenalkyl, Carboxy, Amino und Halogen, substituiert ist;
n 0, 1, 2 oder 3 ist;
$Y^3$ H, Halogen oder $C_{1-6}$-Alkyl ist, wobei $C_{1-6}$-Alkyl gegebenenfalls mit einem oder mehreren Substituenten, ausgewählt aus der Gruppe aus Hydroxy, $C_{1-6}$-Alkoxy, Amino und Halogen, substituiert ist;
m 0 oder 1 ist;
$Y^4$ $Y^{4a}$, $Y^{4b}$, $Y^{4c}$ oder $Y^{4d}$ ist; wobei $Y^{4a}$ gleich H oder Halogen ist; $Y^{4b}$ $C_{1-6}$-Alkyl, gegebenenfalls substituiert mit einem oder mehreren Substituenten, ausgewählt aus der Gruppe bestehend aus $C_{1-6}$-Halogenalkyl, Halogen, Hydroxy, Amino, Cyano und $C_{1-6}$-Alkoxy, ist; $Y^{4c}$, $C_{1-6}$-Cycloalkyl, gegebenenfalls substituiert mit einem oder Substituenten, ausgewählt aus der Gruppe bestehend aus $C_{1-6}$-Alkyl, $C_{1-6}$-Halogenalkyl, Halogen, Hydroxy, Amino, Cyano und $C_{1-6}$-Alkoxy, ist; und $Y^{4d}$ Amino, gegebenenfalls mit eine oder mehreren $C_{1-6}$-Alkyl, Alkoxy-$C_{1-6}$-alkyl oder Hydroxy-$C_{1-6}$-alkyl, ist;

oder ein pharmazeutisch verträgliches Salz davon.

**2.** Eine Verbindung nach Anspruch 1 der Formel I.

**3.** Eine Verbindung nach Anspruch 1 der Formel II.

**4.** Eine Verbindung nach Anspruch 1 der Formel III.

**5.** Eine Verbindung nach Anspruch 2, 3 oder 4, wobei $Y^1$ Methyl ist.

**6.** Eine Verbindung nach Anspruch 2, 3 oder 4, wobei X gleich CH ist.

**7.** Eine Verbindung nach Anspruch 2, 3 aden 4, wobei n gleich 1 ist und m gleich 0 ist.

**8.** Eine Verbindung nach Anspruch 2, 3 oder 4, wobei $Y^3$ gleich H ist.

**9.** Eine Verbindung Anspruch 2, 3 oder 4, wobei $Y^2$ ethyl, Hydroxymethyl, Hydroxyethyl oder Halogen ist.

**10.** Eine Verbindung nach Anspruch 2, 3 oder 4, wobei $Y^4$,

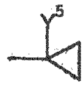

wobei $Y^5$ Halogen, $C_{1-6}$-Alkyl oder $C_{1-6}$-Halogenalkyl ist;

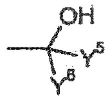

,

wobei $Y^5$ und $Y^6$ unabhängig H, $C_{1-6}$-Alkyl oder $C_{1-6}$-Halogenalkyl sind;

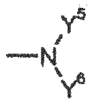

,

wobei $Y^5$ und $Y^6$ unabhängig H oder $C_{1-6}$-Alkyl sind, oder

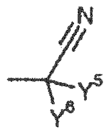

,

wobei $Y^5$ und $Y^6$ unabhängig H, $C_{1-6}$-Alkyl $C_{1-6}$-Halogenalkyl ist.

**11.** Eine Verbindung nach Anspruch 2, 3 oder 4, wobei R gleich $-R^1-R^2-R^3$ ist; wobei $R^1$ Phenyl oder Pyridyl ist; $R^2$ gleich -C(=O) ist; $R^3$ gleich $R^4$ ist; und $R^4$ Morpholin oder Piperazin ist, gegebenenfalls substituiert mit einem oder mehreren $C_{1-6}$-Alkyl.

**12.** Verbindung nach eine der Ansprüche 1 - 11 zur Verwendung bei der Behandlung einer entzündlichen und/oder Autoimmunerkrankung, z.B. Arthritis, oder der Hemmung der B-Zellenproliferation.

**13.** Ein Arzneimittel, umfassend die Btk-Inhibitorverbindung nach der Ansprüche 1-11 in Mischung mit mindestens eine pharmazeutisch verträglichen Träger, Exzipienten oder Verdünnungsmittel.

**14.** Die Verwendung einer Verbindung der Formel I nach einem der Ansprüche 1-11 zur Herstellung eines Medikaments zur Behandlung einer entzündlichen und/oder Autoimmunerkrankung.

**Revendications**

**1.** Composé de formule I, II ou III

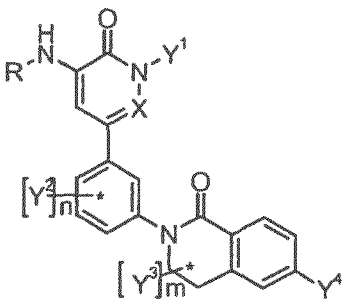

(I)

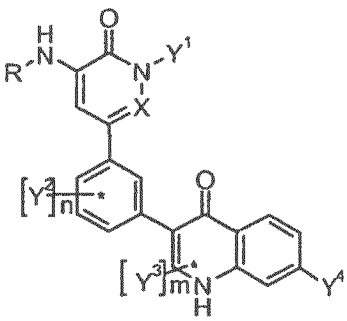

(II)

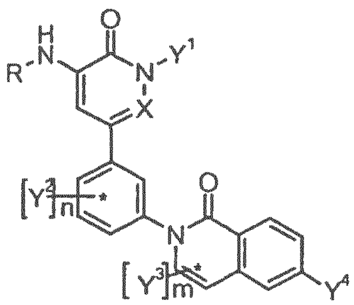

(III)

dans laquelle :

R représente H, un groupe $-R^1$, $-R^1-R^2-R^3$, $-R^1-R^3$ ou $-R^2-R^3$ ;
$R^1$ représente un groupe aryle, hétéroaryle, cycloalkyle ou hétérocycloalkyle, et est facultativement substitué avec un ou plusieurs substituants alkyle en $C_1$ à $C_6$, hydroxy, hydroxyalkyle en $C_1$ à $C_6$, alkoxy en $C_1$ à $C_6$, halogène, nitro, amino, amido, cyano, oxo ou halogénoalkyle en $C_1$ à $C_6$ ;
$R^2$ représente un groupe -C(=O), -C(=O)O, $-C(=O)NR^{2'}$, -NHC(=O)O, $-C(=NH)NR^{2'}$, ou $-S(=O)_2$ ; dans lequel $R^{2'}$ représente H ou un groupe alkyle en $C_1$ à $C_6$ ;
$R^3$ représente H ou un groupe $R^4$ ; $R^4$ représentant un groupe alkyle en $C_1$ à $C_6$, amino, aryle, arylalkyle, alkylaryle, hétéroaryle, alkylhétéroaryle, hétéroaryl-alkyle, cycloalkyle, alkylcycloalkyle, cycloalkylalkyle, hétérocycloalkyle, alkylhétérocycloalkyle ou hétéro-cycloalkylalkyle et étant facultativement substitué avec un ou plusieurs substituants alkyle en $C_1$ à $C_6$, hydroxy, alkoxy en $C_1$ à $C_6$, hydroxyalkyle en $C_1$ à $C_6$, hydroxyalkoxy en $C_1$ à $C_6$, alkylsulfonyle en $C_1$ à $C_6$, alkylsulfonamido en $C_1$ à $C_6$, carbamate, carboxy, ester, amido, acyle, halogéno, nitro, amino, cyano, oxo, ou halogénoalkyle en $C_1$ à $C_6$ ;
X représente un groupe CH ou N ;
$Y^1$ représente H, un groupe alkyle en $C_1$ à $C_6$ ou halogénoalkyle en $C_1$ à $C_6$;
chaque groupe $Y^2$ représente indépendamment un groupe halogéno, oxime ou alkyle en $C_1$ à $C_6$, ledit groupe alkyle en $C_1$ à $C_6$ étant facultativement substitué avec un ou plusieurs substituants choisis dans le groupe consistant en des substituants hydroxy, alkoxy en $C_1$ à $C_6$, halogéno-alkoxy en $C_1$ à $C_6$, halogénoalkyle en Ci à $C_6$, carboxy, amino et halogène ;
n égal à 0, 1, 2 ou 3 ;
$Y^3$ représente H, un groupe halogène ou alkyl en $C_1$ à $C_6$, ledit groupe alkyle en $C_1$ à $C_6$ étant facultativement substitué avec un ou plusieurs substituants choisis dans le groupe consistant en substituant hydroxy, alkoxy en $C_1$ à $C_6$, amino et halogène ;
m est égal à 0 ou 1 ;
$Y^4$ représente un groupe $Y^{4a}$, $Y^{4b}$, $Y^{4c}$ ou $Y^{4d}$ ; ledit groupe $Y^{4a}$ représentant H ou un groupe halogène ; ledit groupe $Y^{4b}$ représentant un groupe alkyl en $C_1$ à $C_6$, facultativement substitué un ou plusieurs substituant choisis dans le groupe consistant en des substituant halogénoalkyle en $C_1$ à $C_6$, halogène, hydroxy, amino, cyano et alkoxy en $C_1$ à $C_6$ ; ledit groupe $Y^{4c}$ représentant un groupe cycloalkyle en $C_1$ à $C_6$, facultativement substitué un ou plusieurs substituants choisis dans le groupe consistant en des substituants alkyle en $C_1$ à $C_6$, halogénoalkyle en $C_1$ à $C_6$, halogéno, hydroxy, amine, cyano et alkoxy en $C_1$ à $C_6$ ; et ledit groupe $Y^{4d}$ représentant un groupe amino, facultativement substitué avec un ou plusieurs substituants alkyle en $C_1$ à $C_6$, alkoxy (alkyle en Ci à $C_6$) ou hydroxyalkyle en $C_1$ à $C_6$ ;

ou un de sels pharmaceutiquement acceptables.

2. Composé suivant la revendication 1, de formule I.

3. Composé suivant la revendication 1, de formule II.

4. Composé suivant la revendication 1, de formule III.

5. Composé suivant la revendication 2, 3 ou 4, dans lequel $Y^1$ représente un groupe méthyle.

6. Composé suivant la revendication 2, 3 ou 4, dans lequel X représente un groupe CH.

7. Composé suivant la revendication 2, 3 ou 4, dans lequel n est égal à 1 et m est égal à 0.

8. Composé suivant la revendication 2, 3 ou 4, dans lequel $Y^3$ représente H.

9. Composé suivant la revendication 2, 3 ou 4, dans lequel $Y^2$ représente un groupe méthyle, hydroxyméthyle, hydroxyéthyle ou halogéno.

10. Composé suivant la revendication 2, 3 ou 4, dans lequel $Y^4$ représente un groupe

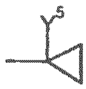

dans lequel $Y^5$ représente un groupe halogéno, alkyle en $C_1$ à $C_6$ ou halogénoalkyle en $C_1$ à $C_6$ ;

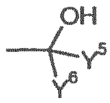

dans lequel $Y^5$ et $Y^6$ représentent indépendamment H, un groupe alkyl en $C_1$ à $C_6$ ou halogéno-alkyl en $C_1$ à $C_6$ ;

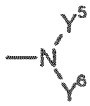

dans lequel $Y^5$ et $Y^6$ représentent indépendamment H ou groupe alkyle en Ci à $C_6$ ; ou

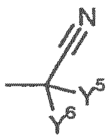

dans lequel $Y^5$ et $Y^6$ représentent indépendamment H, un groupe alkyle en $C_1$ à $C_6$ ou halogénoalkyle en $C_1$ à $C_6$.

11. Composé suivant la revendication 2, 3 ou 4, dans lequel R représente un groupe $-R^1-R^2-R^3$ dans lequel $R^1$ représente un groupe phényle ou pyridyle ; $R^2$ représente un groupe -C(=O) ; $R^3$ représente un groupe $R^4$; et $R^4$ représente un groupe morpholine ou pipérazine, facultativement substitué avec un ou plusieurs substituants alkyle en $C_1$ à $C_6$.

12. Composé suivant l'une quelconque des revendications 1 à 11, pour une utilisation dans le traitement d'une affection inflammatoire et/ou auto-immune, par exemple l'arthrite, ou dans l'inhibition de la prolifération de lymphocytes B.

13. Composition pharmaceutique comprenant le composé inhibiteur de Btk de l'une quelconque des revendications 1 à 11, en mélange avec au moins un support, excipient ou diluant pharmaceutiquement acceptable.

14. Utilisation d'un composé de formule I suivant l'une quelconque des revendications 1 à 11 pour la préparation d'un pour le traitement d'une affection inflammatoire et/ou auto-immune.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HUNTER.** *Cell,* 1987, vol. 50, 823-829
- **RASTETTER et al.** *Annu Rev Med,* 2004, vol. 55, 477
- **KHAN et al.** *Immunity,* 1995, vol. 3, 283
- **ELLMEIER et al.** *J. Exp. Med.,* 2000, vol. 192, 1611
- **ROSEN et al.** *New Eng. J. Med.,* 1995, vol. 333, 431
- **LINDVALL et al.** *Immunol. Rev.,* 2005, vol. 203, 200
- **JANSSON ; HOLMDAHL.** *Clin. Exp. Immunol.,* 1993, vol. 94, 459
- **PAN et al.** *Chem. Med Chem.,* 2007, vol. 2, 58-61
- **IWAKI et al.** *J. Biol. Chem.,* 2005, vol. 280, 40261
- **HORWOOD et al.** *J Exp Med,* 2003, vol. 197, 1603
- **ISLAM ; SMITH.** *Immunol. Rev.,* 2000, vol. 178, 49
- **FELDHAHN et al.** *J. Exp. Med.,* 2005, vol. 201, 1837
- **GOODMAN ; GILMAN'S.** The Pharmacological Basis of Therapeutics. McGraw Hill Companies Inc, 2001
- **RIGAUDY ; KLESNEY.** Nomenclature in Organic Chemistry. Pergamon Press, 1979
- **VASSILEV et al.** *J. Biol. Chem.,* 1998, vol. 274, 1646-1656
- **FELDHAHN et al.** *J. Exp. Med.,* 2005, vol. 201 (11), 1837-1852
- Remington: The Science and Practice of Pharmacy. Mack Publishing Company, 1995